# EUROPEAN PATENT APPLICATION

(11) **EP 4 349 832 A1**
(43) Date of publication of application: **10.04.2024**
(21) Application number: 22815080.1
(22) Date of filing: 23.05.2022
(51) Int. Cl.: C07D 403/14, C07D 271/107, A61K 45/00, A61P 35/00, A61P 37/02

(54) **CHEMICAL COUPLING LINKER AND USE THEREOF**

(30) Priority: 02.06.2021 CN 202110615166; 21.10.2021 CN 202111225483
(71) Applicant: Sichuan Kelun-Biotech Biopharmaceutical Co., Ltd., Chengdu, Sichuan 611138 (CN)
(72) Inventor: TIAN, Qiang, CHENGDU, Sichuan 611138 (CN); WANG, Xiaobei, CHENGDU, Sichuan 611138 (CN); LI, Deliang, CHENGDU, Sichuan 611138 (CN); ZHANG, Yitao, CHENGDU, Sichuan 611138 (CN); YE, Jian, CHENGDU, Sichuan 611138 (CN); HU, Ruibin, CHENGDU, Sichuan 611138 (CN); SONG, Hongmei, CHENGDU, Sichuan 611138 (CN); GE, Junyou, CHENGDU, Sichuan 611138 (CN); WANG, Jingyi, CHENGDU, Sichuan 611138 (CN)
(74) Representative: Cabinet Nony
(86) International application number: PCT/CN2022/094540
(87) International publication number: WO 2022/253033

(57) **Abstract**

The present invention relates to a chemical coupling linker and use thereof, and a bioactive conjugate prepared by the chemical coupling linker. The present invention also relates to use of the bioactive conjugate in the preparation of a drug for the prevention or treatment of tumor diseases.

## Description

The present application is based on the application with the CN application number 202110615166.4 and application date of June 2, 2021, and the application with the CN application number 202111225483.1 and application date of October 21, 2021, and claims their priorities. The disclosure of these CN applications is hereby incorporated into the present application in its entirety.

### Technical Field

The present invention relates to a novel linker used for chemical coupling, an antibody-drug conjugate and corresponding composition prepared therefrom, as well as preparation method and application thereof in the treatment of tumor-related diseases.

### Background Art

In recent years, antibody-drug conjugates have become one of the hot directions of precise tumor treatment, which bringing hope for tumor treatment. Antibody-drug conjugate (ADC) is composed of a monoclonal antibody drug targeting a specific antigen and a small-molecule cytotoxic drug conjugated through a linker, which has both the powerful killing effect of traditional small-molecule chemotherapy and the tumor-targeting of antibody drug. As of April 2021, with the approval of Zynlonta, 12 ADC drugs have been approved for marketing worldwide, of which 7 are used to treat hematological tumors and 5 are used to treat solid tumors.

An antibody-drug conjugate is composed of an antibody, a linker, a payload, and a conjugation mode. The conjugation mode refers to the connection mode between the antibody and the drug-linker. Conjugation modes are mainly divided into non-site-specific conjugation and site-specific conjugation. In the early days, the non-site-specific conjugation mode was used, mainly by lysine conjugation and cysteine conjugation. The drug was directly conjugated with an amino acid residue on the antibody by chemical method, which did not involve the transformation or modification of the antibody, both the number of cytotoxins conjugated and the conjugation site were uncertain, and the uniformity was poor. At present, the commonly used site-specific conjugation mode is specific conjugation through genetic engineering sites or special connectors to achieve more uniform conjugation, which can realize the connection of cytotoxins at specific site. The antibody-drug conjugates produced by site-specific conjugation can reduce the fluctuation of efficacy, pharmacokinetics, and CMC quality control caused by differences in conjugation sites and conjugation number.

Currently, the common site-specific conjugation methods include THIOMAB technology, unnatural amino acid conjugation technology, glutamine enzymatic conjugation technology, Sortase transpeptidase conjugation technology, and ThioBridge technology. Among them, the modification of antibody using antibody engineering or enzymatic conjugation may have a certain impact on the structural stability of the antibody, and at the same time have certain requirements for CMC. In addition, the partial ThioBridge technology using chemical conjugation also have certain defects, for example, DBM (dibromomaleimides) linkers may undergo substitution with other sulfhydryl-containing biological groups, and thus are unstable in plasma, resulting in the decrease of drug efficacy and increased toxic and side effects (Chem. - Eur. J., 2019, 25, 43-59). Therefore, it is still of great significance to develop novel linker structures for developing antibody-drug conjugates with good efficacy and safety.

### Contents of the present invention

One object of the present application is to provide a novel linker for chemical conjugation. This type of linker has high reactivity, mild conjugation conditions, easy to operate, and can realize site-specific conjugation, and the obtained bioactive conjugate has good uniformity and plasma stability, as well as clear drug efficacy in vivo and in vitro.

### Compound

In a first aspect, the present application provides a compound or a pharmaceutically acceptable salt, ester, stereoisomer, tautomer, polymorph, solvate, N-oxide or isotopically labeled compound thereof, wherein the compound has the structure of Formula I: 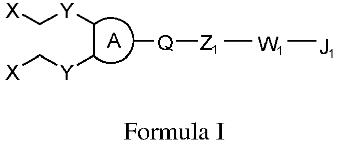 wherein:
X is a leaving group such as Cl, Br, I, OMs, OTs, OTf or 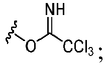
Y is absent or a carbonyl;
ring A is selected from the group consisting of substituted or unsubstituted C₆₋₁₀ aromatic ring, 5- to 12-membered heteroaromatic ring or 5- to 12-membered heterocyclic ring;
Q is absent or -C(=O)-NH-;
Z₁ is absent or selected from the group consisting of -CH₂- or C₂₋₆ alkynylene;
W₁ is absent or one or more selected from the group consisting of C₁₋₁₀ alkylene, - (CH₂CH₂O)ₚ-, and -(OCH₂CH₂)ₚ-;
J₁ is selected from the group consisting of -COOH, -NH₂, 3- to 10-membered nitrogen-containing heterocyclic group, sulfonylurea group or hydroxyl;
p is an integer of 1 to 10.

In some embodiments, X is one or more selected from the group consisting of Cl, Br, I, OMs, OTs and OTf.

In some embodiments, Y is absent.

In some embodiments, Z₁ is absent or C₂₋₆ alkynylene.

In some embodiments, W₁ is absent or one or more selected from the group consisting of C₁₋₁₀ alkylene and -(CH₂CH₂O)ₚ-.

In some embodiments, W₁ is C₁₋₁₀ alkylene; preferably C₁₋₆ alkylene, more preferably C₁₋₃ alkylene.

In some embodiments, J₁ is -COOH.

In some embodiments, ring A is selected from the group consisting of substituted or unsubstituted 5- to 12-membered nitrogen-containing heteroaromatic ring or 5- to 12-membered nitrogen-containing heterocyclic ring; preferably, ring A is selected from the group consisting of 5- to 12-membered nitrogen-containing heteroaromatic ring or 5- to 12-membered nitrogen-containing heterocyclic ring that are unsubstituted or substituted by oxo or -COOH.

In some embodiments, p is 1, 2, 3, 4, 5, 6, 7, 8, 9, or 10.

In some embodiments, X is selected from the group consisting of Cl, Br, I, OMs, OTs, OTf and 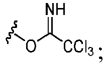
Y is absent;
ring A is selected from the group consisting of 5- to 12-membered nitrogen-containing heteroaromatic ring or 5- to 12-membered nitrogen-containing heterocyclic ring that are unsubstituted or substituted by oxo or -COOH;
Q is absent or -C(=O)-NH-;
Z₁ is absent or -CH₂-;
W₁ is absent or one or more selected from the group consisting of C₁₋₁₀ alkylene and - (CH₂CH₂O)ₚ-;
J₁ is -COOH;
p is an integer of 1 to 10.

In some embodiments, Formula I is selected from the group consisting of the following structures:

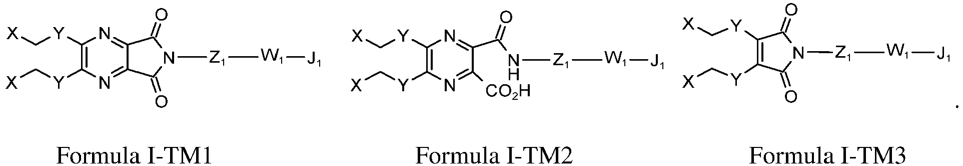

In some embodiments, the compound of Formula I has the following structure: 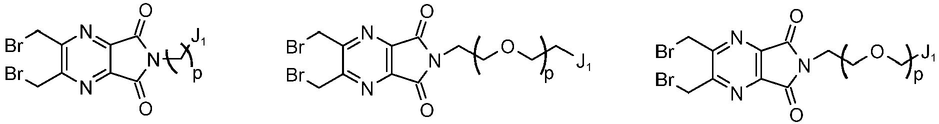 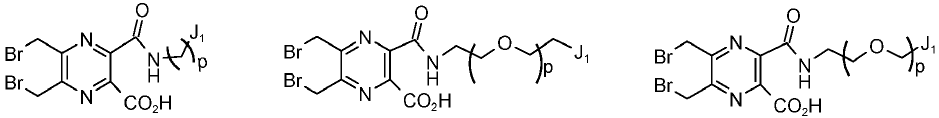 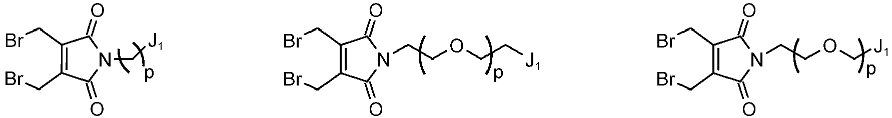 wherein p is an integer selected from 1 to 10, and J₁ is -COOH or -NH₂.

In some embodiments, the compound of Formula I has the following structure:

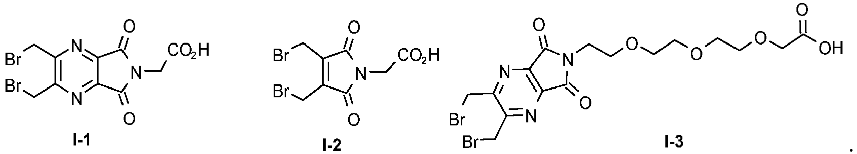

In a second aspect, the present application provides a compound or a pharmaceutically acceptable salt, ester, stereoisomer, tautomer, polymorph, solvate, *N*-oxide or isotopically labeled compound thereof, wherein the compound has the structure of Formula II: 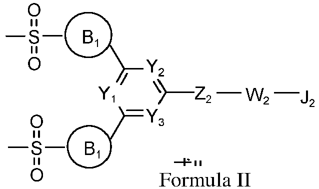
wherein, B₁ in each occurrence is independently selected from the group consisting of single bond or 5- to 12-membered nitrogen-containing heteroaromatic ring;
Y₁, Y₂ and Y₃ in each occurrence are each independently selected from the group consisting of CH and N;
Z₂ is absent or selected from the group consisting of -NH-, -CH₂-, carbonyl, -C(=O)NH-, - NHC(=O)- or C₂₋₆ alkynylene; preferably, Z₂ is absent or selected from -NH-, -CH₂-, carbonyl or C₂₋₆ alkynylene;
W₂ is absent or one or more selected from the group consisting of C₁₋₁₀ alkylene, - (CH₂CH₂O)ₚ- or -(OCH₂CH₂)ₚ-;
J₂ is selected from the group consisting of -COOH, -NH₂, 3- to 10-membered nitrogen-containing heterocyclic group, sulfonylurea group or hydroxyl;
p is an integer of 1 to 10.

In some embodiments, B₁ in each occurrence is independently selected from the group consisting of single bond or 5- to 6-membered nitrogen-containing heteroaromatic ring; preferably, B₁ in each occurrence is independently selected from the group consisting of single bond or 5- to 6-membered nitrogen-containing heteroaromatic ring; further preferably, B₁ in each occurrence is independently selected from the group consisting of single bond or pyrimidine ring.

In some embodiments, Y₁, Y₂ and Y₃ are all N; or Y₁ is CH, both of Y₂ and Y₃ are N; or Y₁ is N, both of Y₂ and Y₃ are CH; or Y₁, Y₂ and Y₃ are all CH.

In some embodiments, Z₂ is absent or C₂₋₆ alkynylene.

In some embodiments, W₂ is absent or C₁₋₁₀ alkylene.

In some embodiments, J₂ is selected from the group consisting of -COOH or -NH₂.

In some embodiments, B₁ in each occurrence is independently selected from the group consisting of single bond or pyrimidine ring;
Y₁, Y₂ and Y₃ are all CH;
Z₂ is absent or -C(=O)NH-;
W₂ is absent or one or more selected from the group consisting of C₁₋₁₀ alkylene, - (CH₂CH₂O)ₚ;
J₂ is -COOH;
p is an integer of 1 to 10.

In some embodiments, the compound of Formula II has the following structure: 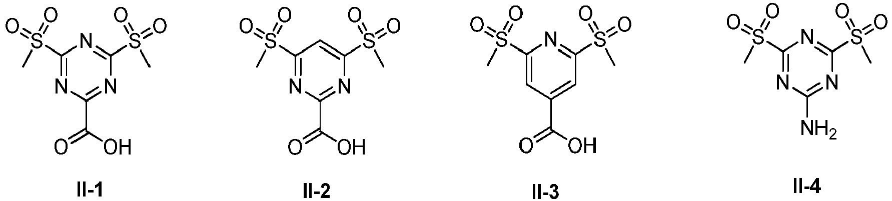 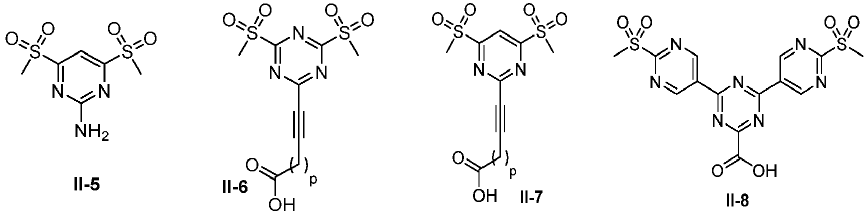 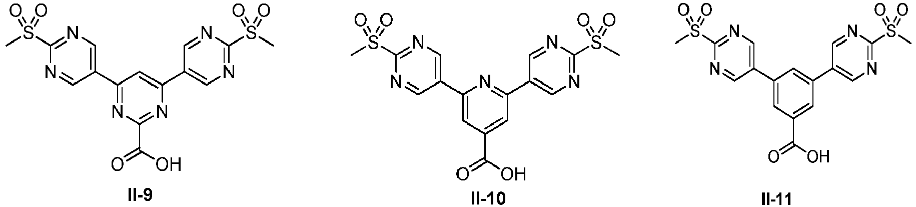 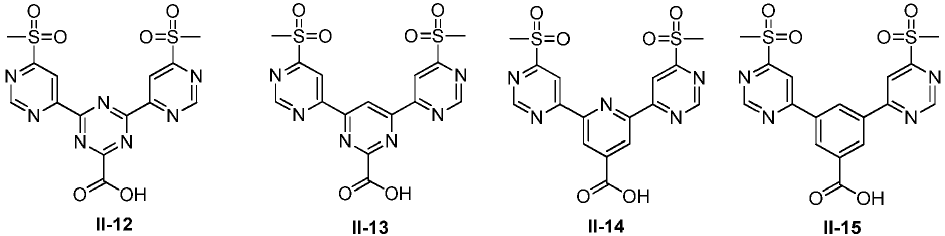 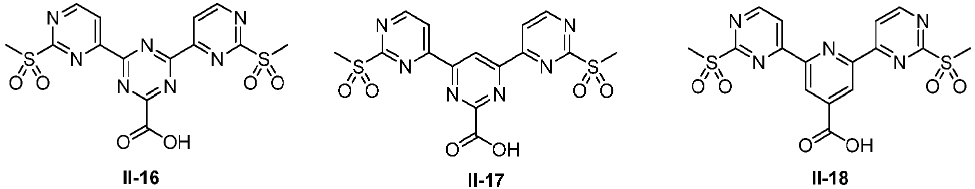 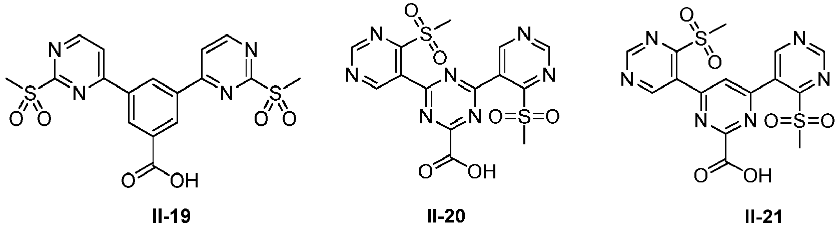 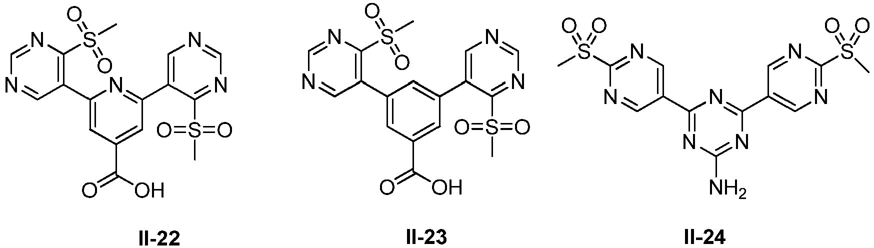 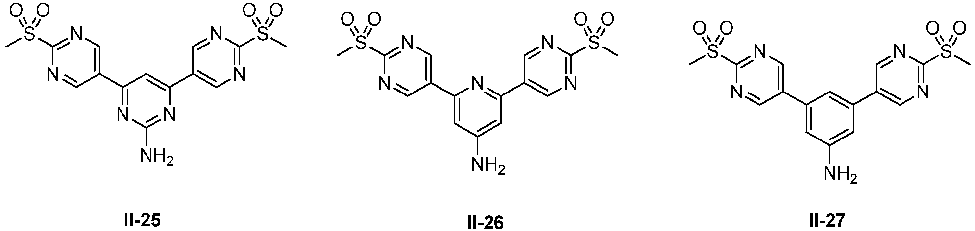 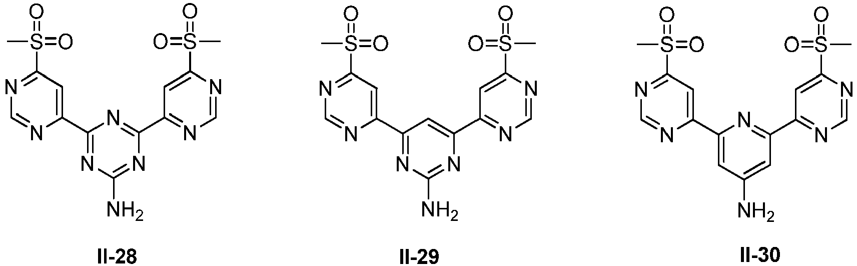 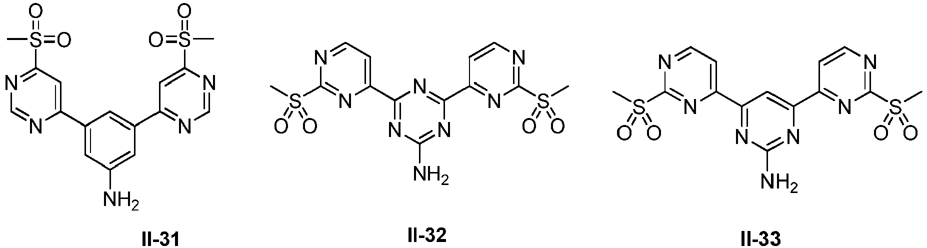 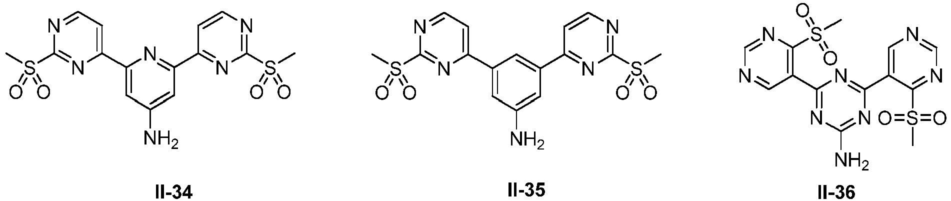 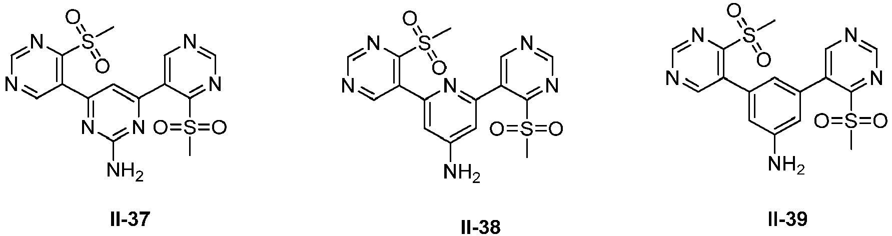 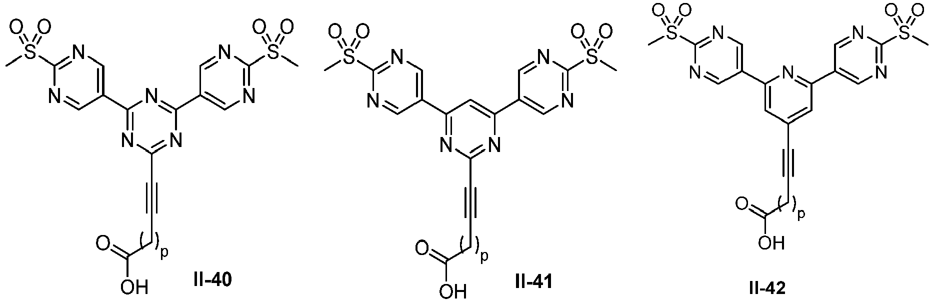 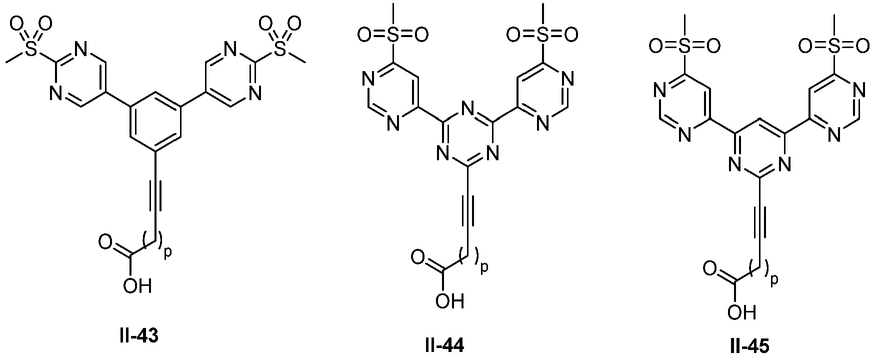 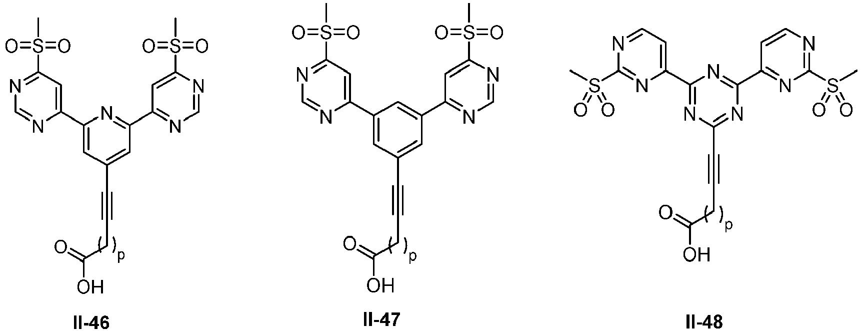 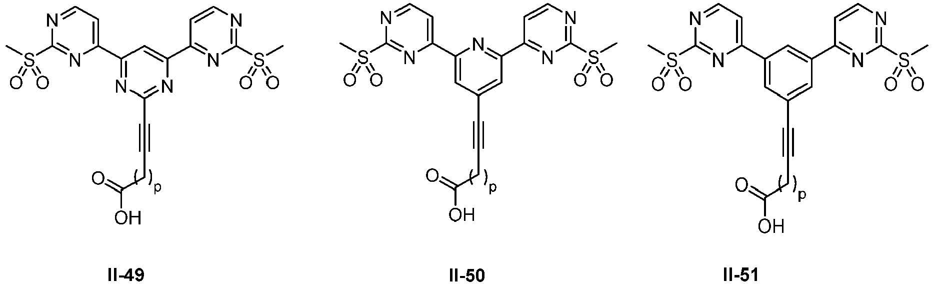 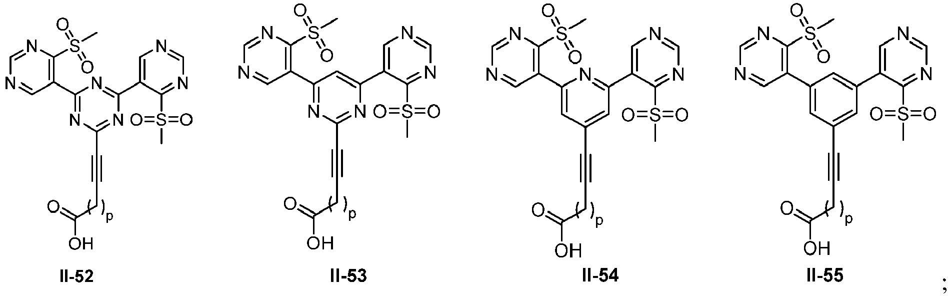 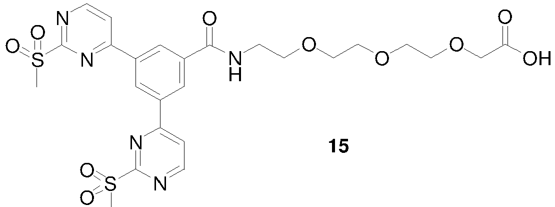 p is an integer of 1 to 10.

In some embodiments, p is an integer of 1 to 8; more preferably, p is an integer of 1 to 5.

In some embodiments, the compound of Formula II has the following structure:

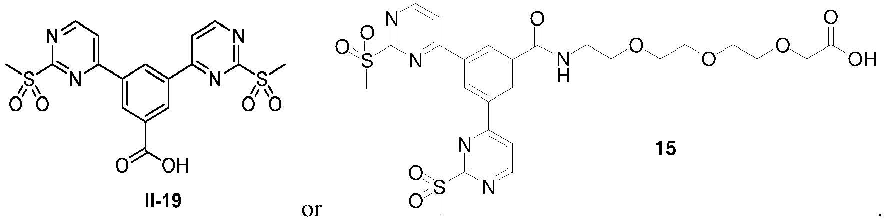

In some embodiments, the compound of Formula II has the following structure:

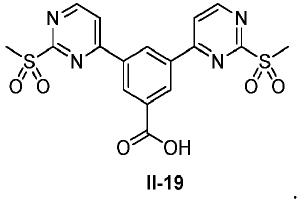

In a third aspect, the present application provides a compound or a pharmaceutically acceptable salt, ester, stereoisomer, tautomer, polymorph, solvate, *N*-oxide or isotopically labeled compound thereof, wherein the compound has the structure of Formula III: 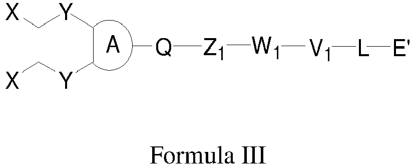 wherein:
V₁ is a group formed from J₁ of the compound of Formula I in the first aspect of the present application when it is connected to L; preferably, V₁ is selected from the group consisting of -CO-, -N(R₁)-, -O-, 3- to 10-membered nitrogen-containing heterocyclic ring group or sulfonylurea group, wherein R₁ is H, C₁₋₆ alkyl or C₂₋₆ alkoxyalkyl; further preferably, V₁ is selected from the group consisting of -C(O)- and -N(R₁)-, wherein R₁ is H, C₁₋₆ alkyl or C₂₋₆ alkoxyalkyl;
L is a linker connecting V₁ and E';
E' is selected from the group consisting of H, -NHCH₂-Lg, -COOH, 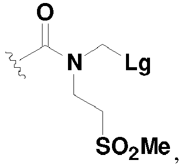 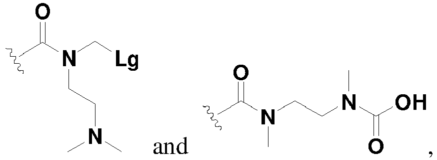 wherein, Lg represents a leaving group, such as Cl, Br, I, OMs, OTs, OTf or 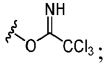
X, Y, A, Q, Z₁, and W₁ are as defined in any items of the foregoing first aspecta.

In some embodiments, L is one or more selected from the group consisting of the following groups: C₁₋₆ alkylene, -N(R₆)-, carbonyl, -O-, Val, Cit, Phe, Lys, D-Val, Leu, Gly, Phe, Ala, Asn, Val-Cit, Val-Ala, Val-Lys, Val-Lys(Ac), Phe-Lys, Phe-Lys(Ac), D-Val-Leu-Lys, Gly -Gly-Arg, Ala-Ala-Asn, Gly-Gly-Gly, Gly-Gly-Phe-Gly, Gly-Gly-Gly-Gly-Gly, 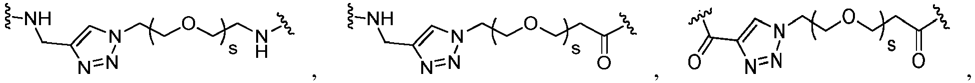 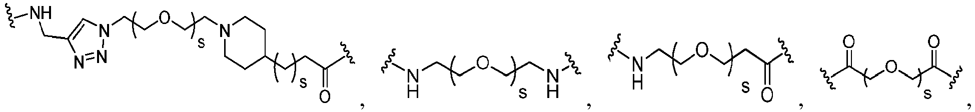 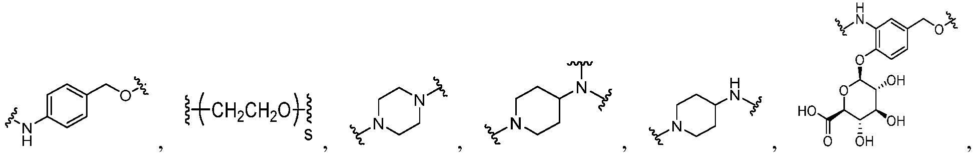 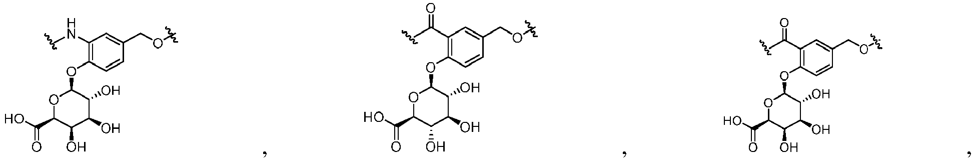 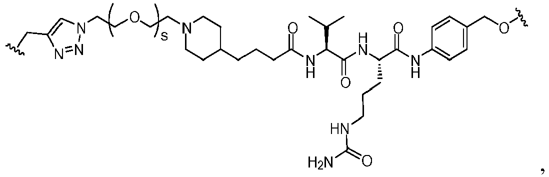 wherein R₆ is H, C₁₋₆ alkyl or C₂₋₆ alkoxyalkyl, and s is an integer of 1 to 10.

In some embodiments, L is one or more selected from the group consisting of the following groups: Val, Cit, Gly, Phe, Ala, Val-Cit, Val-Ala, Gly-Gly-Phe-Gly,

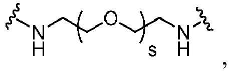

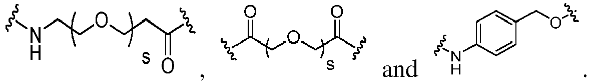

In some embodiments, L is selected from 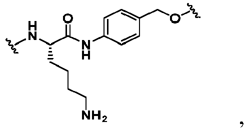 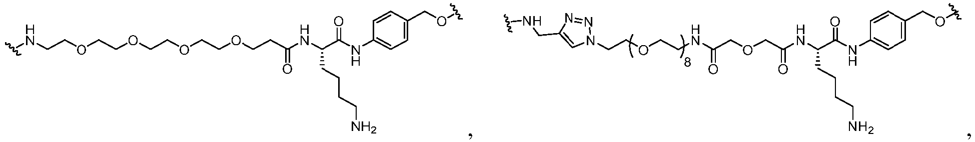 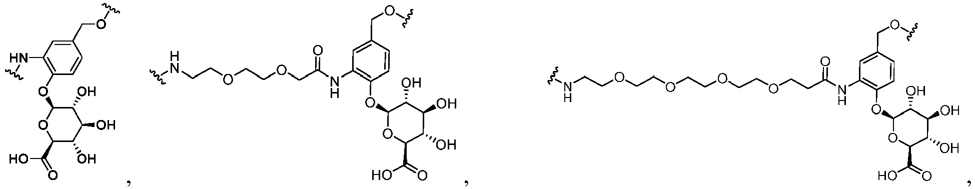 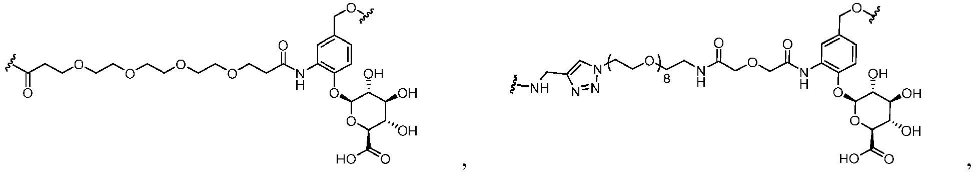 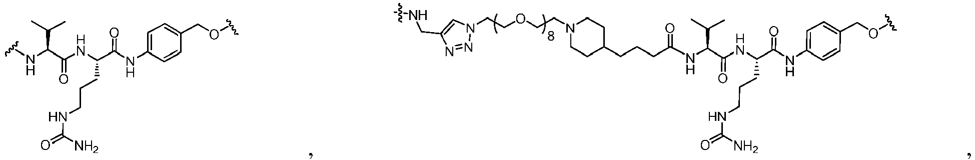 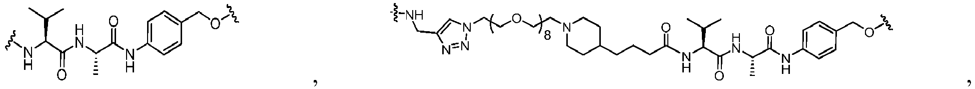 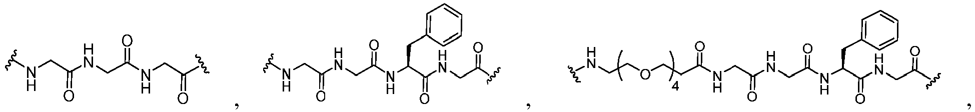 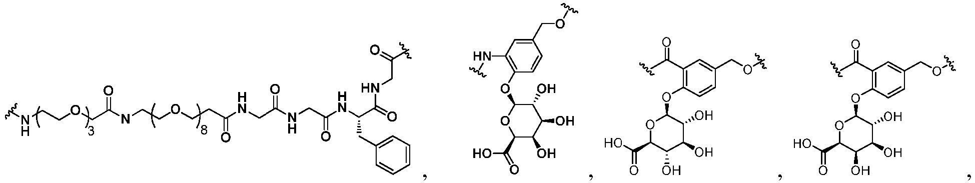 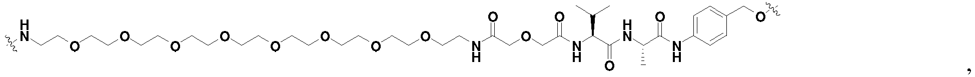 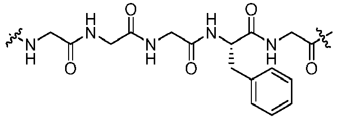 and

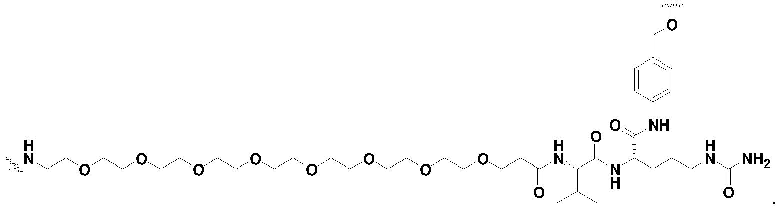

In some embodiments, L is selected from 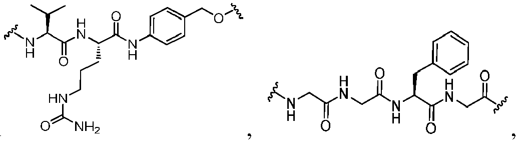 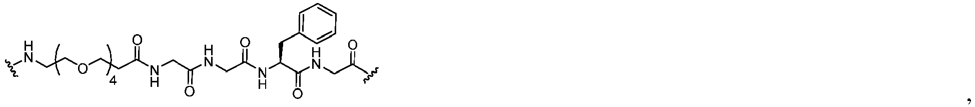 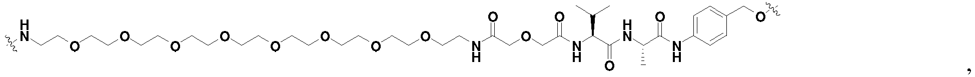 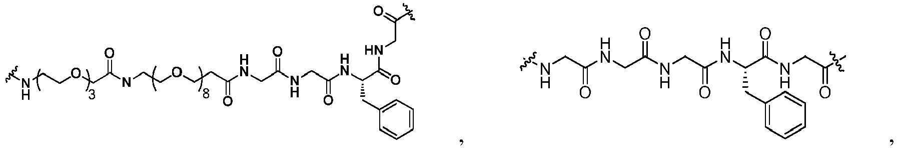 and

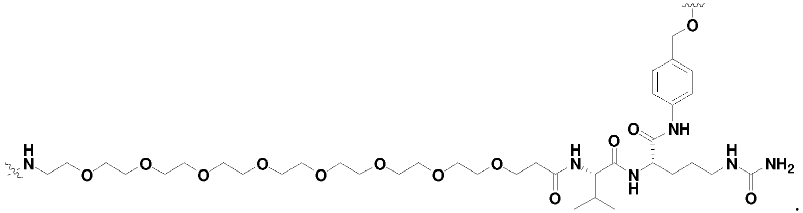

In a fourth aspect, the present application provides a compound or a pharmaceutically acceptable salt, ester, stereoisomer, tautomer, polymorph, solvate, N-oxide or isotopically labeled compound thereof, wherein the compound has the structure of Formula IV: 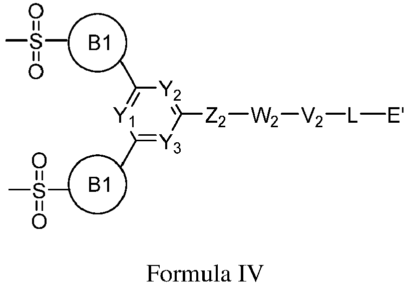 wherein,
V₂ is a group formed from J₂ of the compound of Formula II in the second aspect of the present application when it is connected to L; preferably, V₂ is selected from the group consisting of -CO-, -N(R₂)-, -O-, 3- to 10-membered nitrogen-containing heterocyclic ring group or sulfonylurea group, wherein R₂ is H, C₁₋₆ alkyl or C₂₋₆ alkoxyalkyl; further preferably, V₂ is selected from the group consisting of -C(O)- and -N(R₂)-, wherein R₂ is H, C₁₋₆ alkyl or C₂₋₆ alkoxyalkyl;
B₁, Y₁, Y₂, Y₃, Z₂ and W₂ are as defined in any items of the foregoing second aspect;
L and E' are as defined in any items of the foregoing third aspect.

In a fifth aspect, the present application provides a compound or a pharmaceutically acceptable salt, ester, stereoisomer, tautomer, polymorph, solvate, *N*-oxide or isotopically labeled compound, wherein the compound has the structure of Formula V: 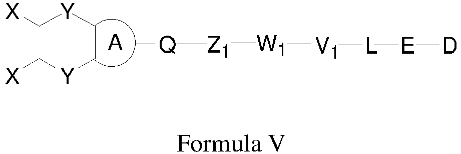 wherein:
E is selected from the group consisting of single bond, -NH-CH₂-, or the following structures: 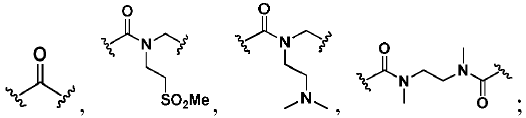
D is a fragment of a bioactive molecule (e.g., a cytotoxic drug);
X, Y, A, Q, Z₁, W₁ are as defined in any items of the foregoing first aspect;
V₁ and L are as defined in any items of the third aspect.

In some embodiments, the bioactive molecule is selected from the group consisting of an antitubulin agent, a DNA intercalator, a DNA topoisomerase inhibitor and a RNA polymerase inhibitor.

In some embodiments, the bioactive molecule is selected from the group consisting of: tubulin inhibitors such as auristatin compounds or maytansine compounds; DNA intercalators such as pyrrolobenzodiazepine (PBD); DNA topoisomerase inhibitors such as topoisomerase I inhibitors (e.g., camptothecin, hydroxycamptothecin, 9-aminocamptothecin, SN-38, irinotecan, topotecan, belotecan, or rubitecan) or topoisomerase II inhibitors (e.g., adriamycin, doxorubicin, PNU-159682, duocarmycin, daunorubicin, mitoxantrone, podophyllotoxin, or etoposide); RNA polymerase inhibitors such as α-amanitin, etc.; or pharmaceutically acceptable salts, esters or analogs thereof.

In some embodiments, the bioactive molecule is selected from: a topoisomerase I inhibitor (e.g., camptothecin, hydroxycamptothecin, 9-aminocamptothecin, SN-38, irinotecan, topotecan, belotecan, rubitecan), MMAE and MMAE derivatives.

In some embodiments, the bioactive molecule is selected from: MMAE and MMAE derivatives.

In some embodiments, D is selected from:

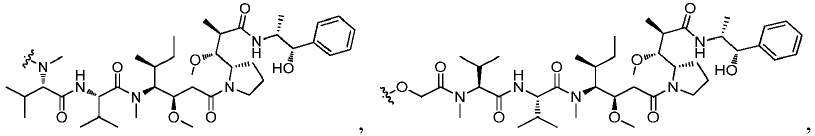

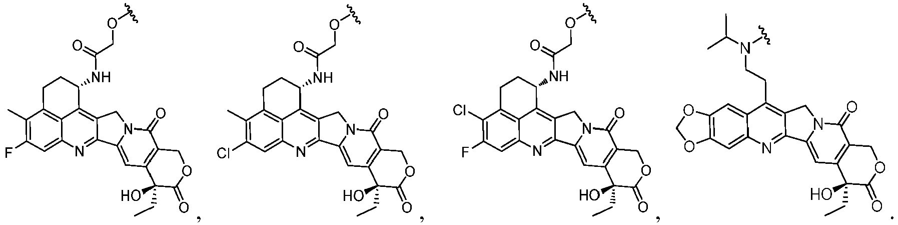

In some embodiments, the compound of Formula V is selected from:

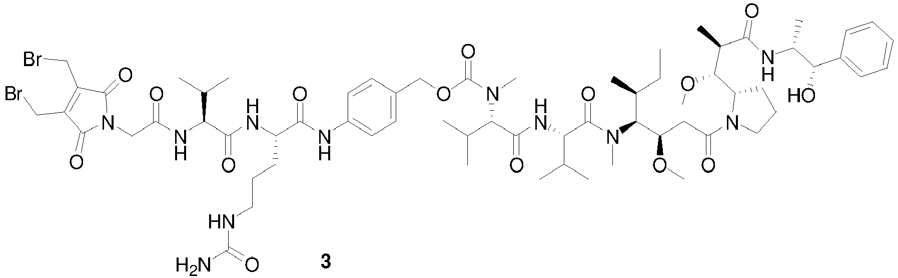

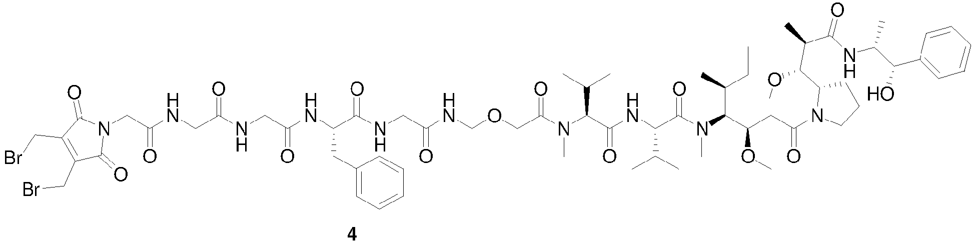

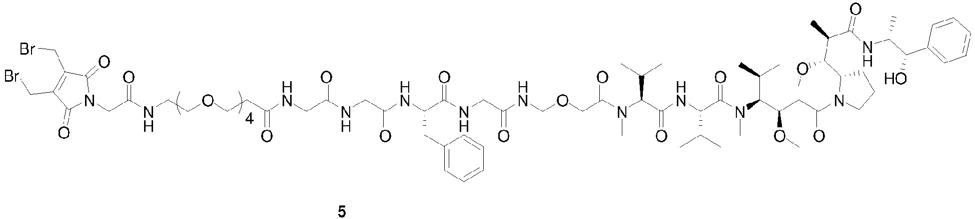

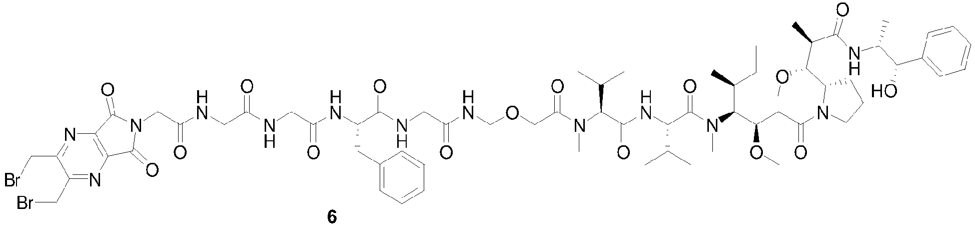

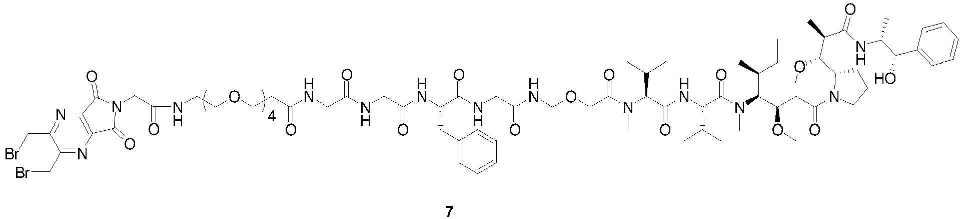

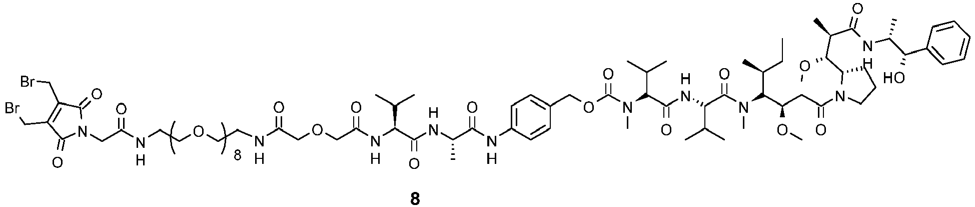

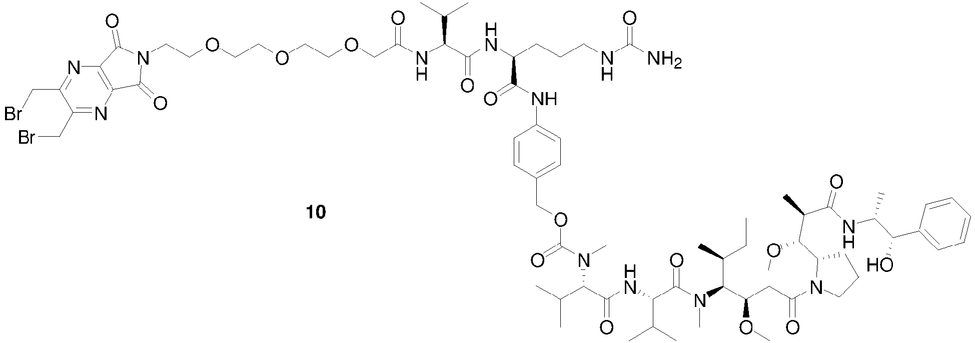

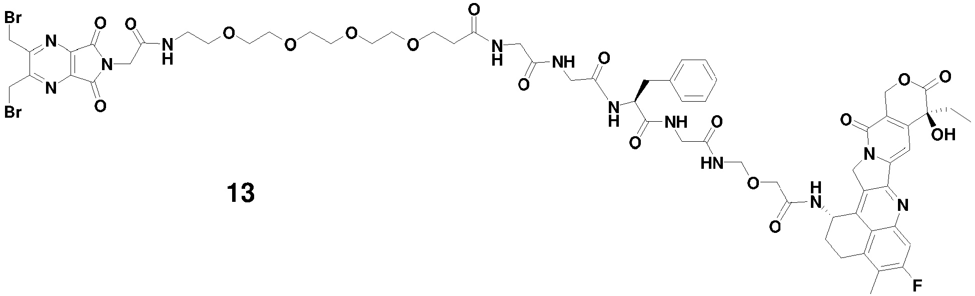

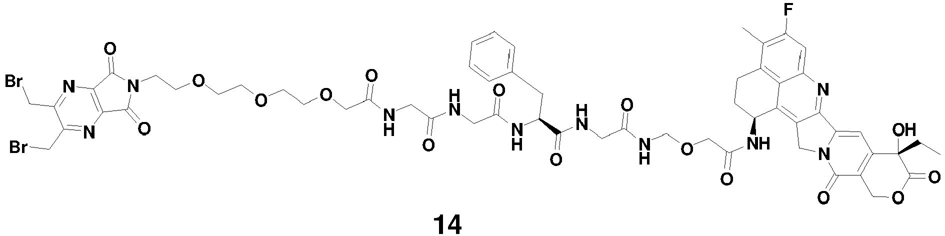

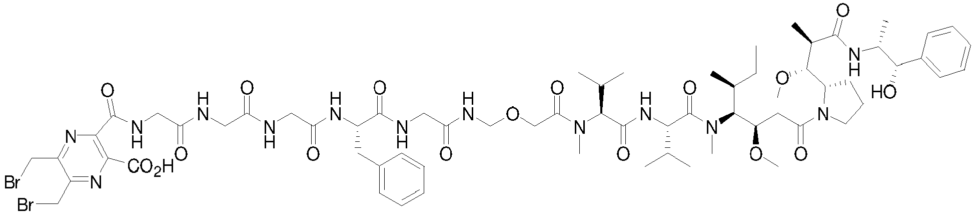

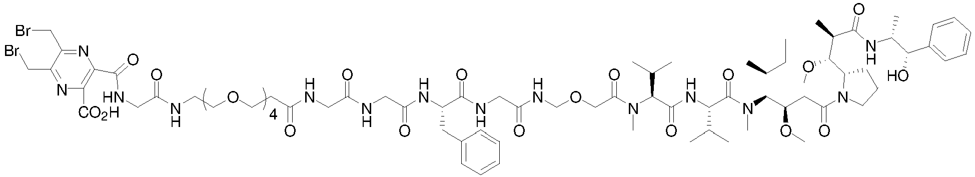

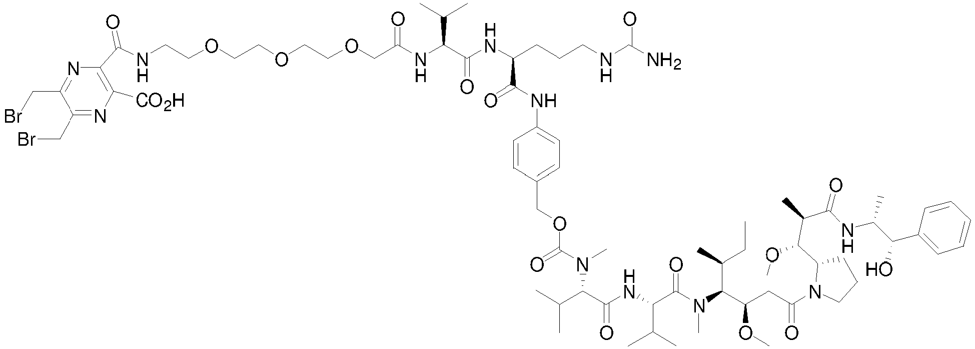

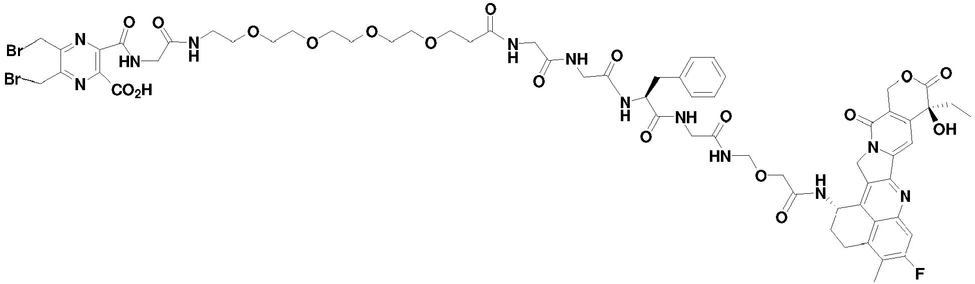

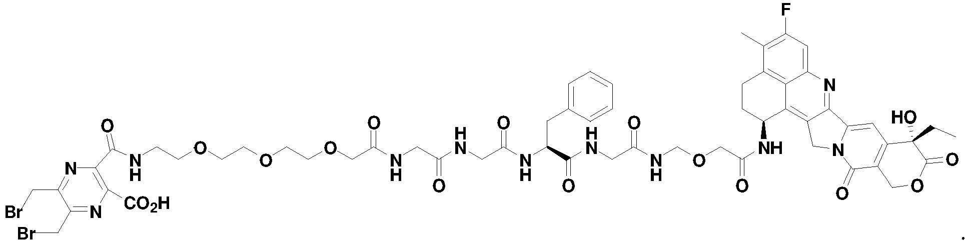

In a sixth aspect, the present application provides a compound or a pharmaceutically acceptable salt, ester, stereoisomer, tautomer, polymorph, solvate, *N*-oxide or isotopically labeled compound thereof, wherein the compound has the structure of Formula VI: 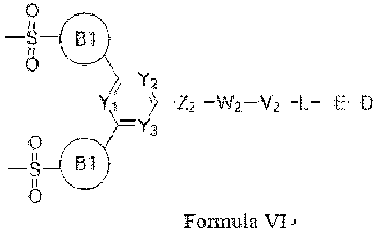
B₁, Y₁, Y₂, Y₃, Z₂ and W₂ are as defined in any items of the foregoing second aspect;
L is as defined in any items of the foregoing third aspect;
V₂ is as defined in any items of the foregoing fourth aspect;
E and D are as defined in any items of the foregoing fifth aspect.

In some embodiments, the compound of Formula VI is

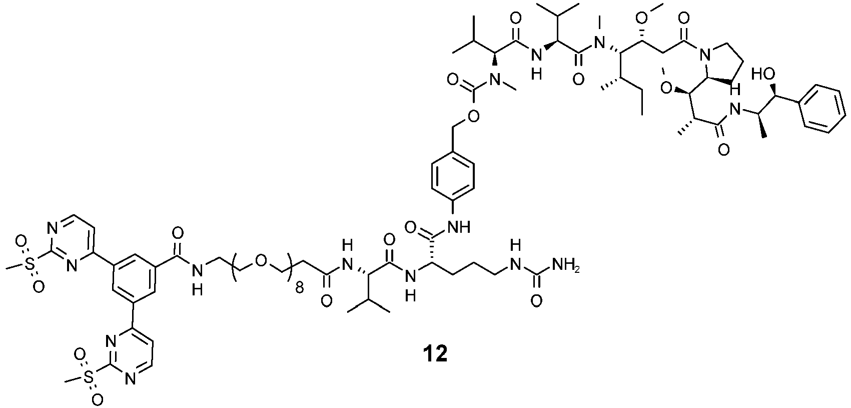

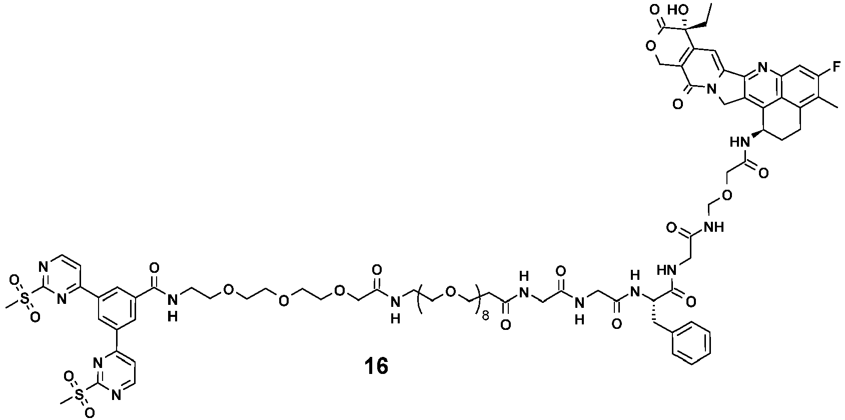

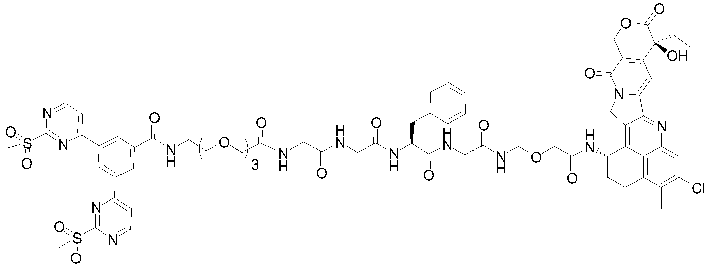

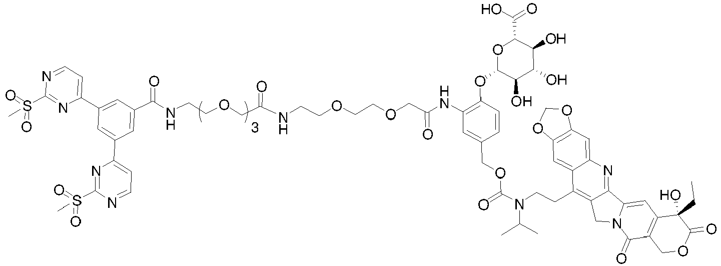

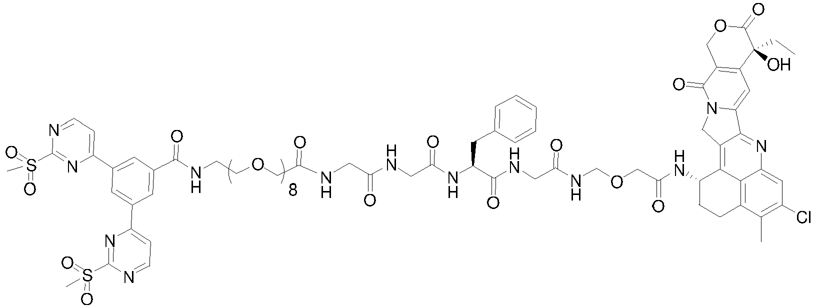

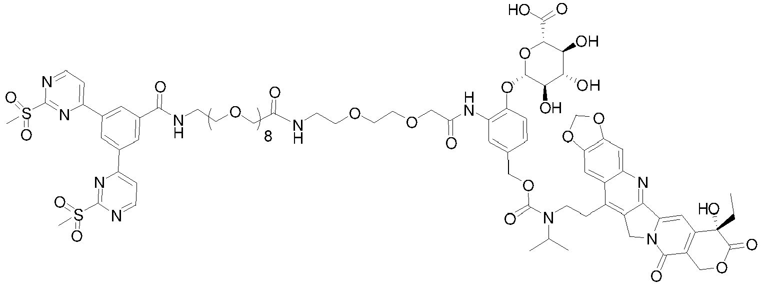

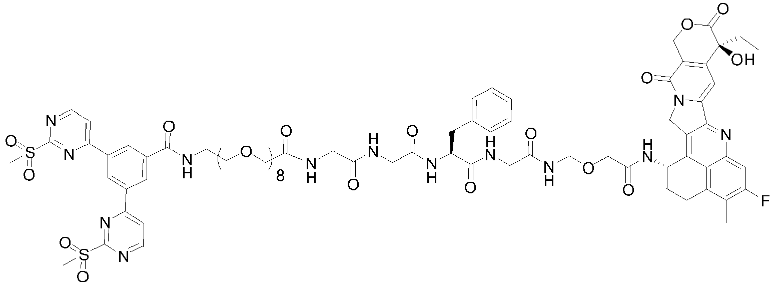

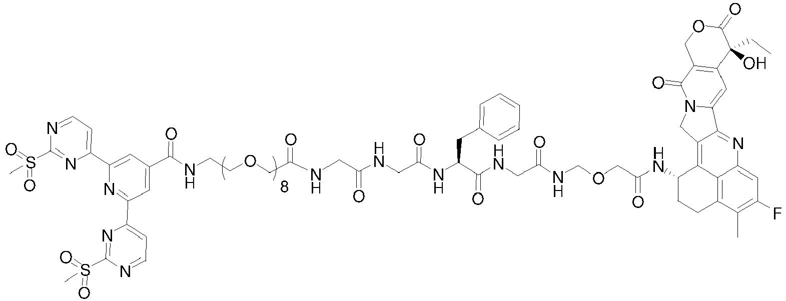

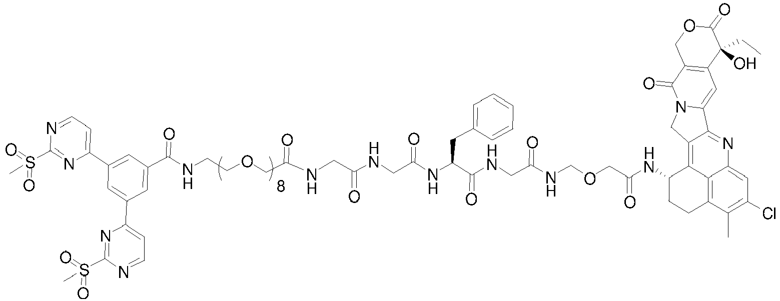

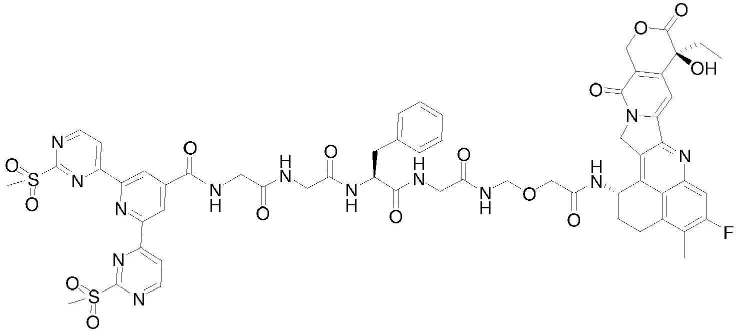

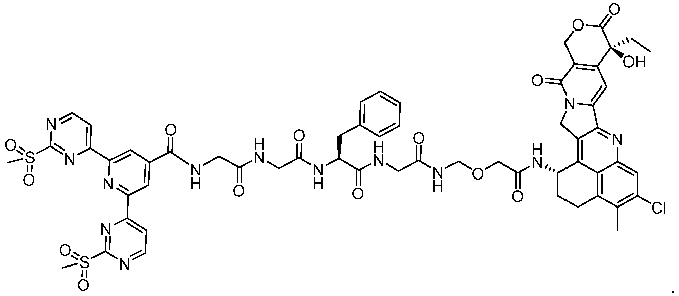

In the seventh aspect, the present application provides a bioactive conjugate, which has a structure as shown in Formula VII: 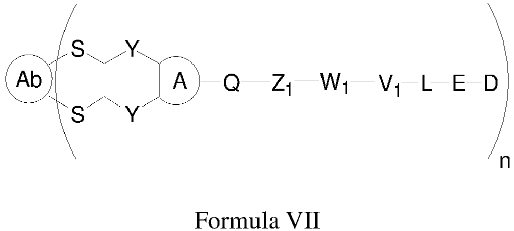
wherein, Ab is a targeting moiety (e.g., small molecule ligand, protein (e.g., antibody), polypeptide, non-protein reagent (e.g., saccharide, RNA or DNA)); n is an integer or decimal of 1 to 10;
V₁ is selected from the group consisting of -C(O)- and -N(R₁)-, wherein R₁ is H, C₁₋₆ alkyl or C₂₋₆ alkoxyalkyl;
L is a linker connecting V₁ and E;
E is a structural fragment connecting L and D;
D is a fragment of a bioactive molecule (e.g., a cytotoxic drug);
when the targeting moiety is an antibody, 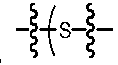 in the conjugate represents a specific way that a sulfhydryl group in the antibody connects to the rest of the conjugate;
all the other groups are as defined in any items of the above aspects.

Another aspect of the present invention provides a bioactive conjugate, which has a structure as shown in Formula VIII: 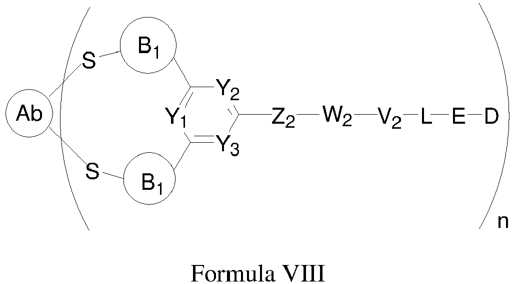
wherein, Ab is a targeting moiety (e.g., small molecule ligand, protein (e.g., antibody), polypeptide, non-protein reagent (e.g., saccharide, RNA or DNA)); n is an integer or decimal of 1 to 10;
V₂ is selected from the group consisting of -C(O)- and -N(R₂)-, wherein R₂ is H, C₁₋₆ alkyl or C₂₋₆ alkoxyalkyl;
L is a linker connecting V₂ and E;
E is a structural fragment connecting L and D;
D is a fragment of a bioactive molecule (e.g., a cytotoxic drug);
when the targeting moiety is an antibody, 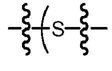 in the conjugate represents a specific way that a sulfhydryl group in the antibody connects to the rest of the conjugate;
all the other groups are as defined in any items of the above aspects.

In some embodiments, Ab is selected from the group consisting of epidermal growth factor, Trop-2, CD37, HeR2, CD70, EGFRvIII, Mesothelin, Folate receptor1, Mucin 1, CD138, CD20, CD19, CD30, SLTRK6, Nectin 4, Tissue factor, Mucin16, Endothelin receptor, STEAP1, SLC39A6, Guanylylcyclase C, PSMA, CCD79b, CD22, Sodium phosphate cotransporter 2B, GPNMB, Trophoblast glycoprotein, AGS-16, EGFR, CD33, CD66e, CD74, CD56, PD-L1, TACSTD2, DR5, E16, STEAP1, 0772P, MPF, Napi3b, Sema 5b, PSCA hlg, ETBR, MSG783, STEAP2, TrpM4, CRIPTO, CD21, CD79b, FcRH2, NCA, MDP, IL20Rα, Brevican, EphB2R, ASLG659, PSCA, GEDA, BAFF-R, CD22, CD79a, CXCR5, HLA-DOB, P2X5, CD72, LY64, FcRH1, IRTA2, TENB2, Integrin α5β6, α4β7, FGF2, FGFR2, Her3, CD70, CA6, DLL3, DLL4, P-cadherin, EpCAM, pCAD, CD223, LYPD3, LY6E, EFNA4, ROR1, SLITRK6, 5T4, ENPP3, SLC39A6, Claudin18.2, BMPR1B, E16, STEAP1, Tyro7, 0772P, MPF, Napi3b, Sema 5b, PSCA hlg, ETBR, MSG783, STEAP2, TrpM4, CRIPTO, CD21, CD79b, FcRH2, NCA, MDP, IL20Rα, Brevican, EphB2R, ASLG659, PSCA, GEDA, CD22, CD79a, CXCR5, HLA-DOB, P2X5, CD72, LY64, FcRH1, IRTA2, c-Met, ApoE, CD11c, CD40, CD45(PTPRC), CD49D(ITGA4), CD80, CSF1R, CTSD, GZMB, Ly86, MS4A7, PIK3AP1, PIK3CD, CCR5, IFNG, IL10RA1, IL-6, ACTA2, COL7A1, LOX, LRRC15, MCPT8, MMP10, NOG, SERPINE1, STAT1, TGFBR1, CTSS, PGF, VEGFA, C1QA, C1QB, ANGPTL4, EGLN, ANGPTL4, EGLN3, BNIP3, AIF1, CCL5, CXCL10, CXCL11, IFI6, PLOD2, KISS1R, STC2, DDIT4, PFKFB3, PGK1, PDK1, AKR1C1, AKR1C2, CADM1, CDH11, COL6A3, CTGF, HMOX1, KRT33A, LUM, WNT5A, IGFBP3, MMP14, CDCP1, PDGFRA, TCF4, TGF, TGFB₁, TGFB2, CDl lb, ADGRE1, EMR2, TNFRSF21, UPK1B, TNFSF9, MMP16, MFI2, IGF-1R, RNF43, NaPi2b, BCMA, B7H3, and TENB2;
preferably, Ab is selected from the group consisting of the anti-Her2 antibody trastuzumab or the anti-Trop2 antibody sacituzumab or the anti-ROR1 antibody 19F6_Hu35V1.

In some embodiments, L is one or more selected from the group consisting of the following groups: C₁₋₆ alkylene, -N(R₆)-, carbonyl, -O-, Val, Cit, Phe, Lys, D-Val, Leu, Gly, Phe, Ala, Asn, Val-Cit, Val-Ala, Val-Lys, Val-Lys(Ac), Phe-Lys, Phe-Lys(Ac), D-Val-Leu-Lys, Gly -Gly-Arg, Ala-Ala-Asn, Gly-Gly-Gly, Gly-Gly-Phe-Gly, Gly-Gly-Gly-Gly-Gly, 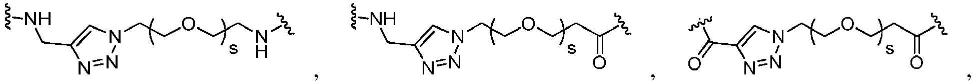 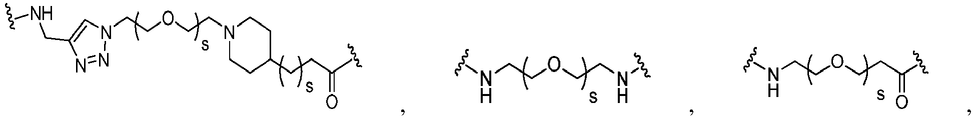 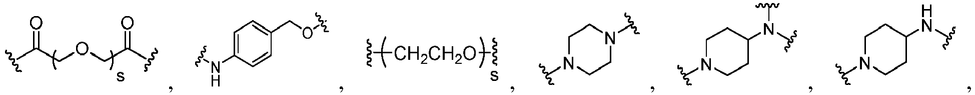 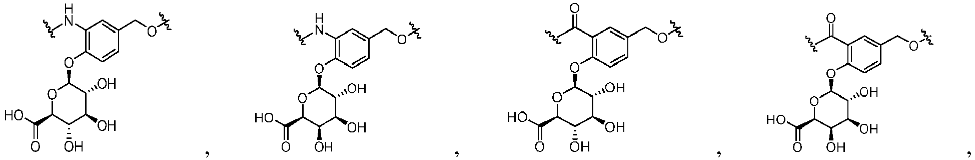 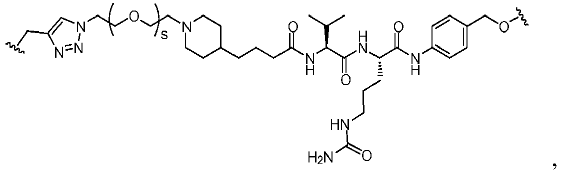 wherein R₆ is H, C₁₋₆ alkyl or C₂₋₆ alkoxyalkyl, s is an integer of 1 to 10;
preferably, L is selected from the group consisting of the following structures:

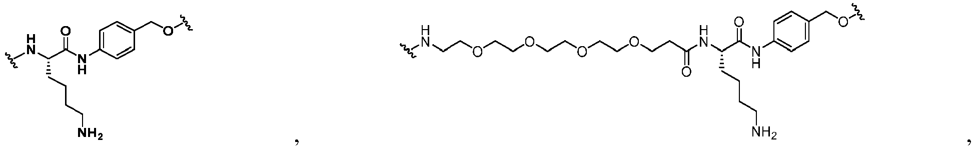

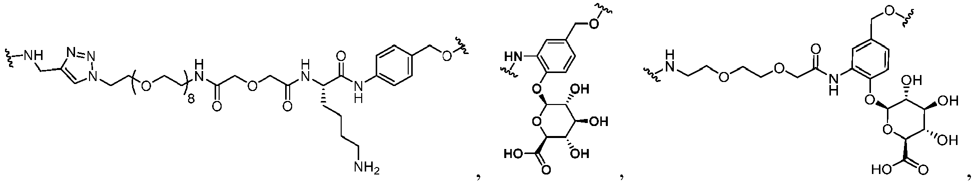

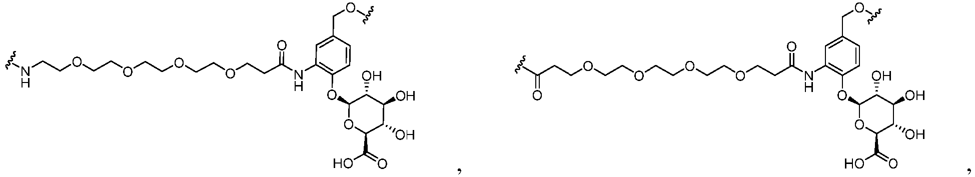

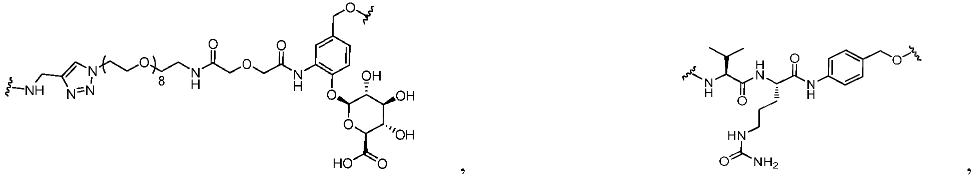

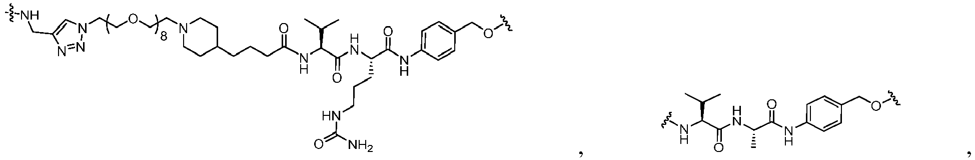

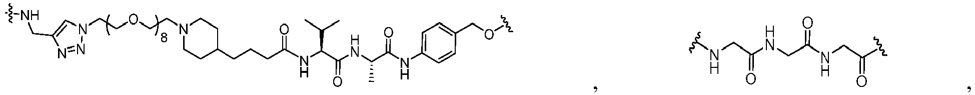

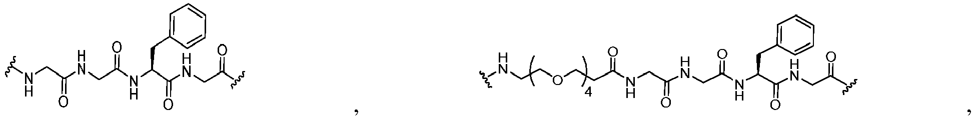

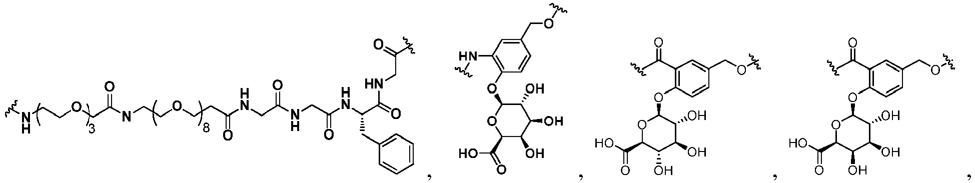

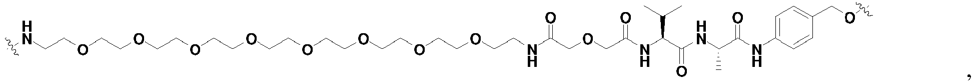

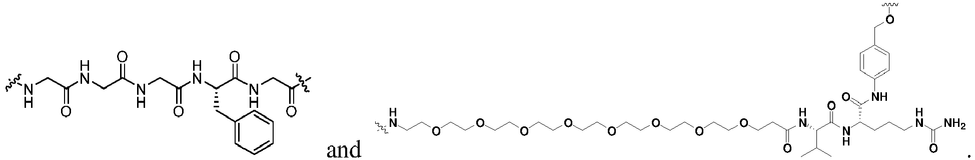

In some embodiments, E is single bond, -NH-CH₂-, and the following structures:

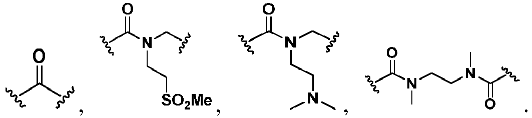

In some embodiments, E is -NH-CH₂-.

In some embodiments, the bioactive molecule is selected from the group consisting of an antitubulin agent, a DNA intercalator, a DNA topoisomerase inhibitor and a RNA polymerase inhibitor.

In some embodiments, the bioactive molecule is selected from the group consisting of: tubulin inhibitors such as auristatin compounds or maytansine compounds; DNA intercalators such as pyrrolobenzodiazepine (PBD); DNA topoisomerase inhibitors such as topoisomerase I inhibitors (e.g., camptothecin, hydroxycamptothecin, 9-aminocamptothecin, SN-38, irinotecan, topotecan, belotecan, or rubitecan) or topoisomerase II inhibitors (e.g., adriamycin, doxorubicin, PNU-159682, duocarmycin, daunorubicin, mitoxantrone, podophyllotoxin, or etoposide); RNA polymerase inhibitors such as α-amanitin, etc.; or pharmaceutically acceptable salts, esters or analogs thereof.

In some embodiments, the bioactive molecule is selected from: a topoisomerase I inhibitor (e.g., camptothecin, hydroxycamptothecin, 9-aminocamptothecin, SN-38, irinotecan, topotecan, belotecan, rubitecan), MMAE and MMAE derivatives.

In some embodiments, the bioactive molecule is selected from: MMAE and MMAE derivatives.

In some embodiments, D is selected from:

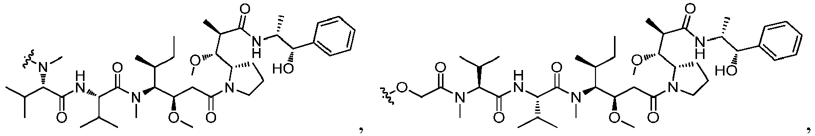

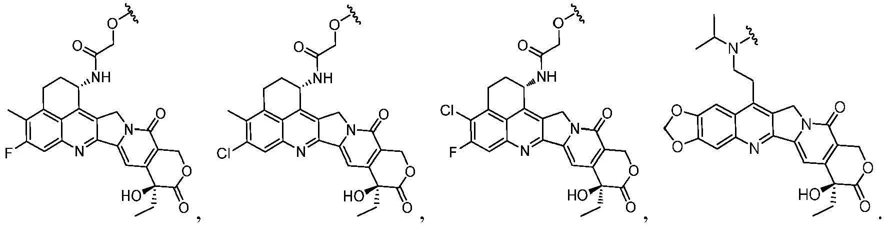

In some embodiments, n is 1 to 8; more preferably n is 3 to 5.

In some embodiments, the bioactive conjugate has the following structure, wherein Ab is selected from the group consisting of the anti-Her2 antibody trastuzumab or the anti-Trop2 antibody sacituzumab or the anti-ROR1 antibody 19F6_Hu35V1, n₁ is 1 to 8; more preferably 3 to 5 :

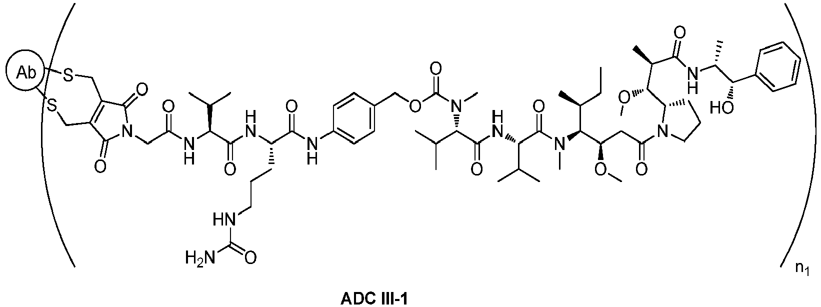

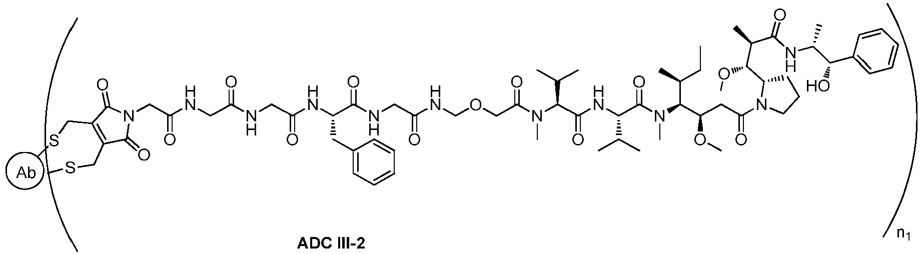

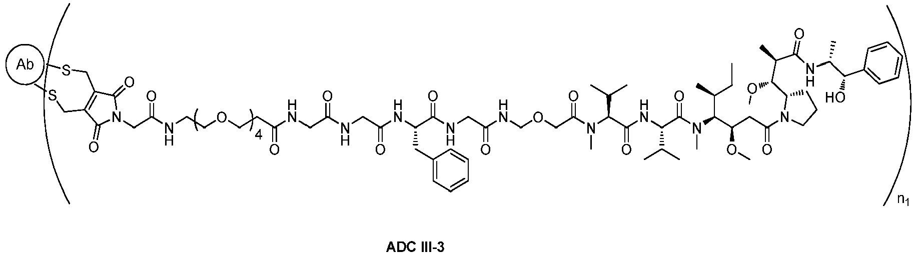

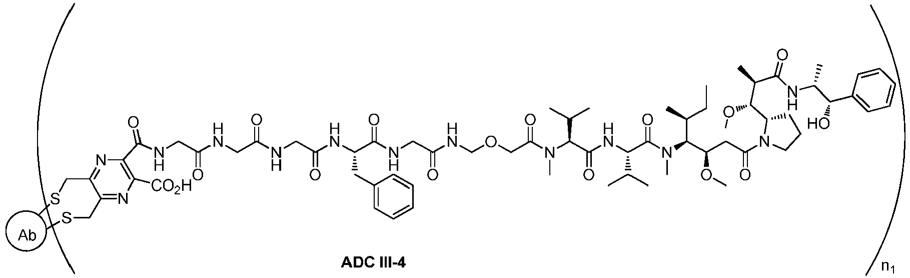

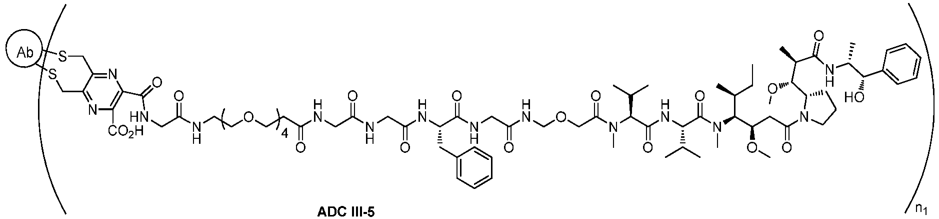

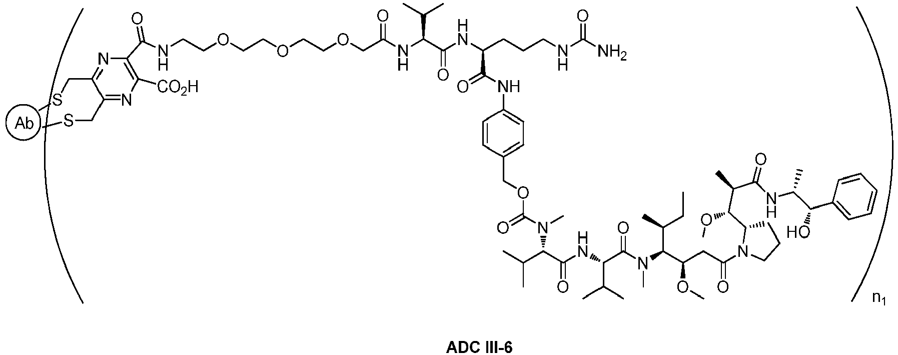

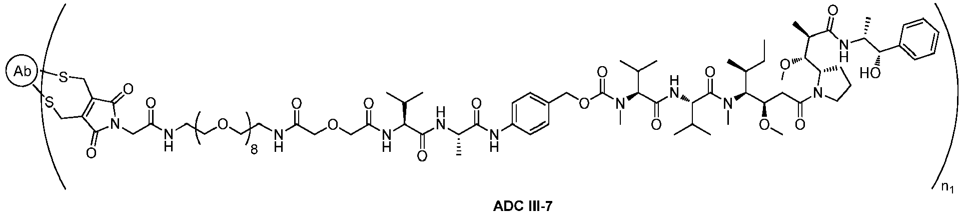

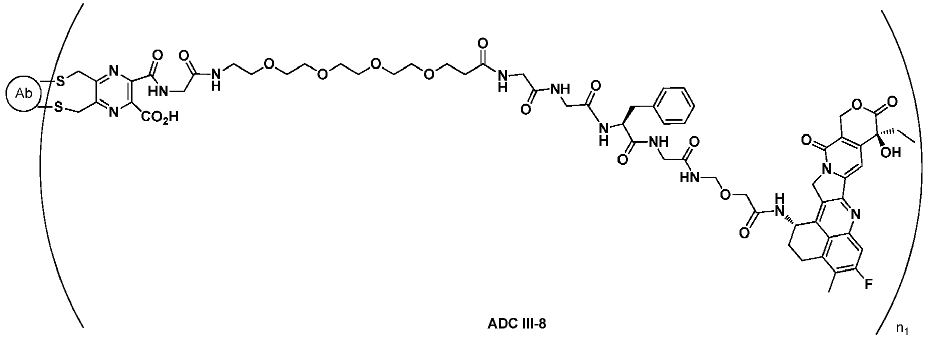

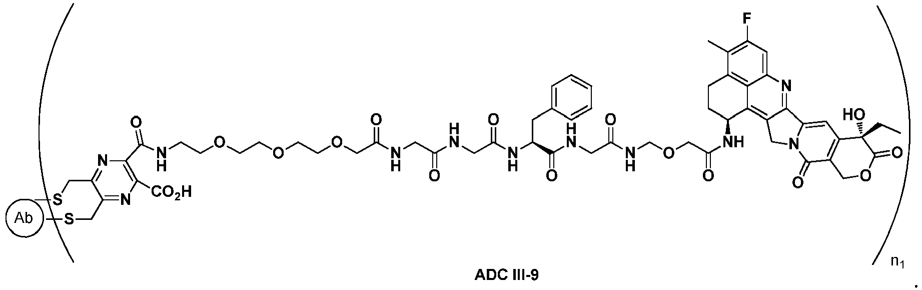

In some embodiments, the bioactive conjugate has the follow structure, wherein Ab is selected from the group consisting of the anti-Her2 antibody trastuzumab or the anti-Trop2 antibody sacituzumab or the anti-ROR1 antibody 19F6_Hu35V1, n₁ is 1 to 8; more preferably 3 to 5:

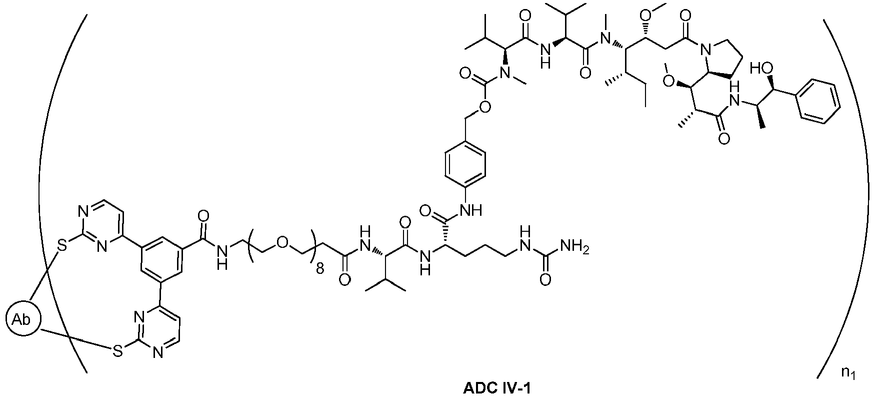

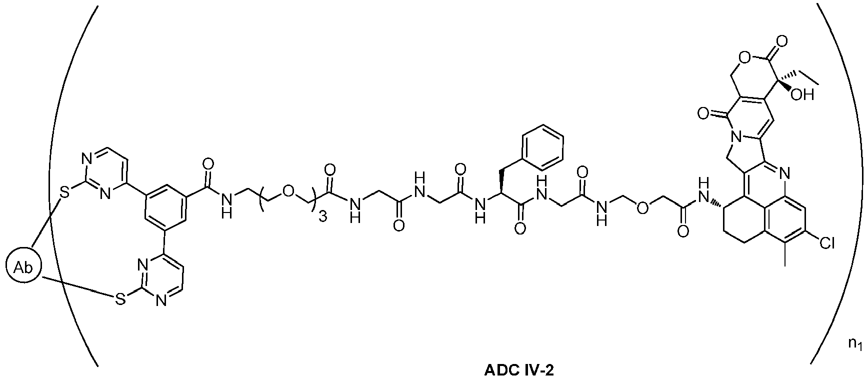

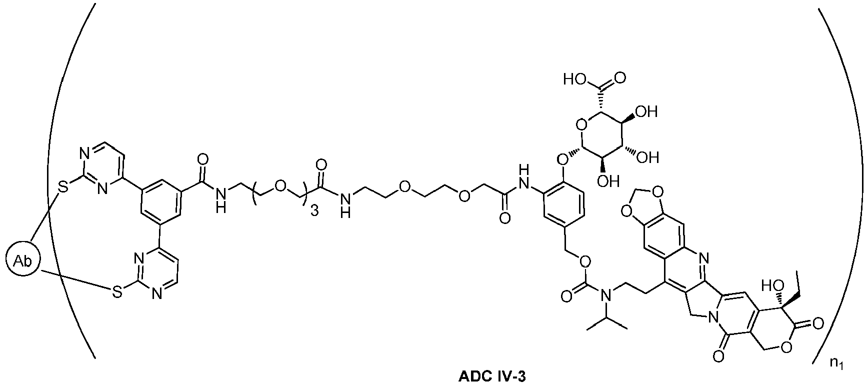

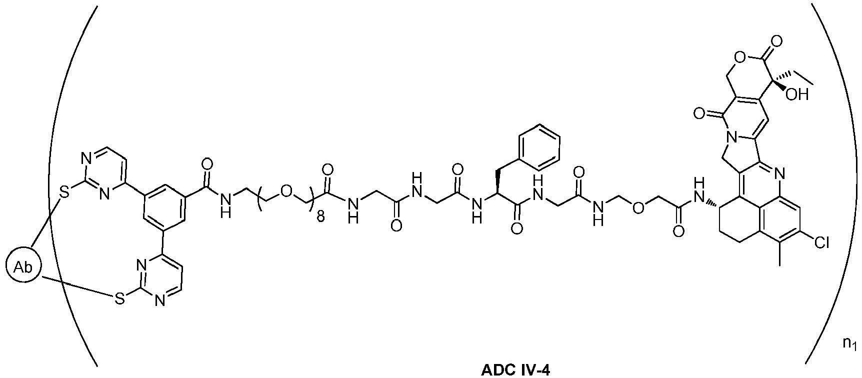

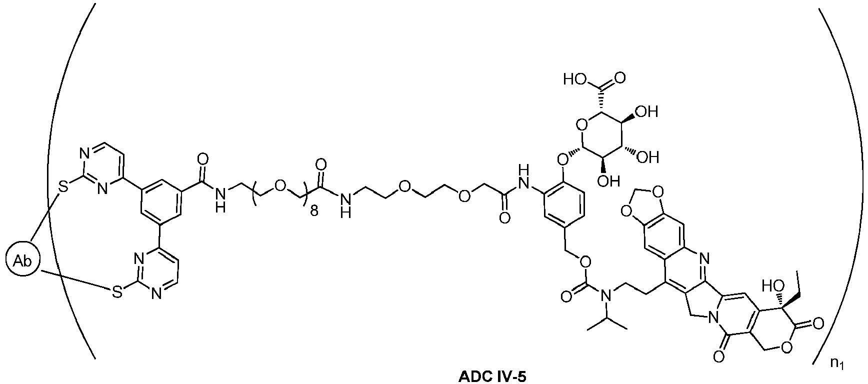

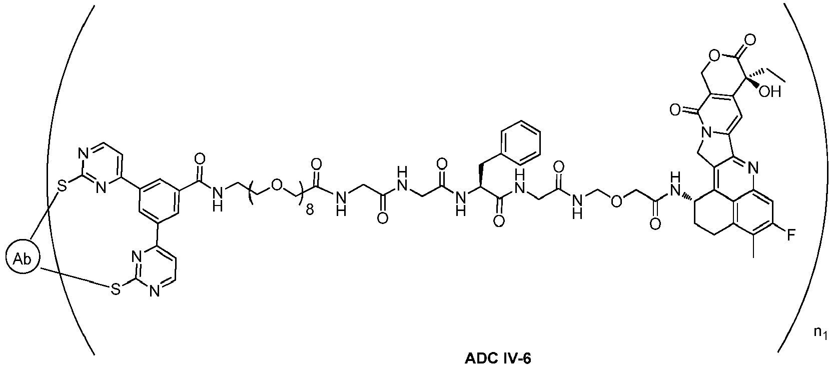

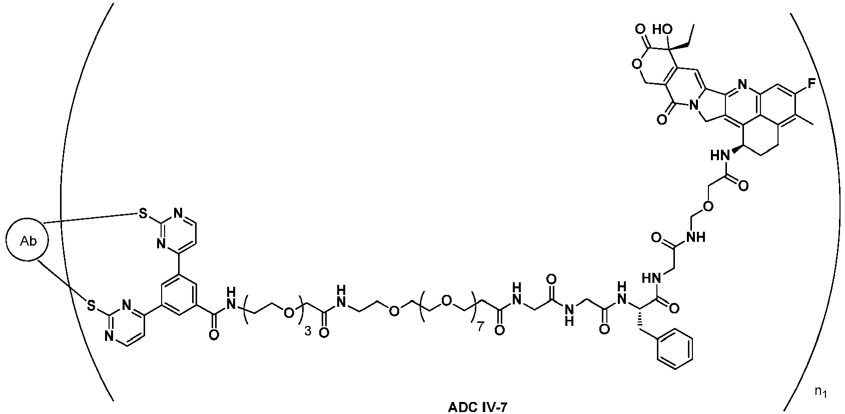

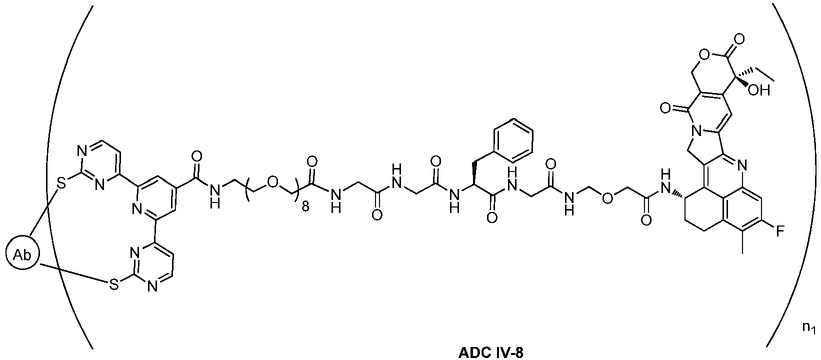

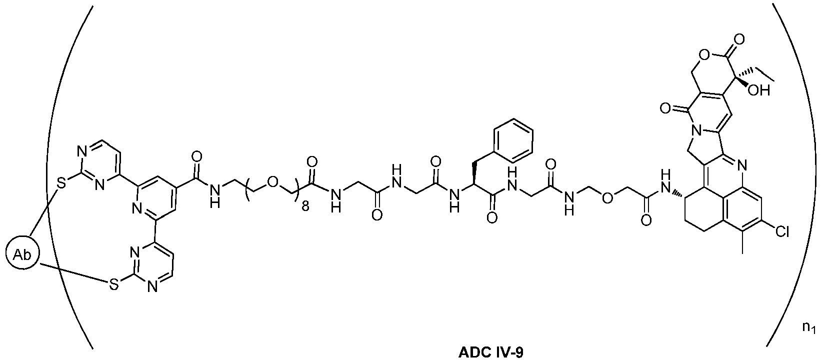

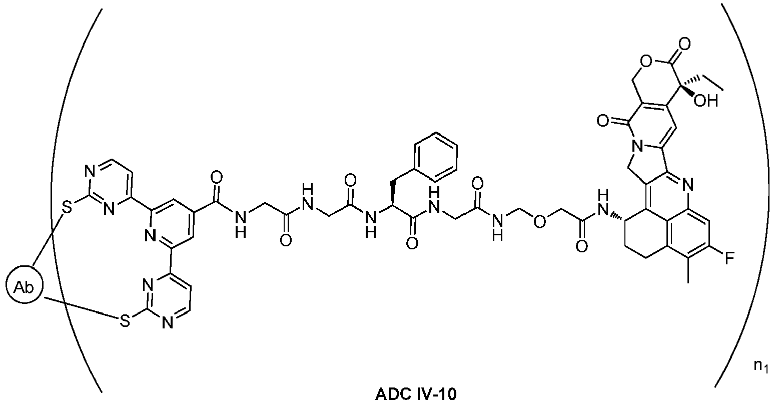

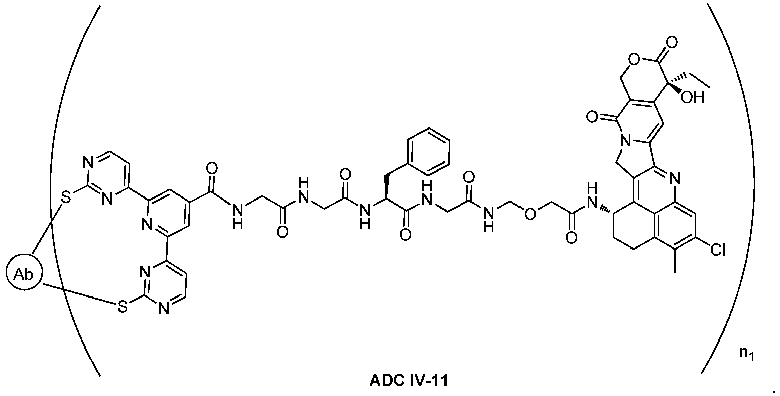

### Definition

Unless defined otherwise hereinafter, all technical and scientific terms used herein have the same meaning as commonly understood by one of ordinary skill in the art. References to techniques used herein are intended to refer to techniques commonly understood in the art, including those changes in technology or substitutions of equivalent technologies obvious to the skilled person. Though the following terms are believed to be well understood by those skilled in the art, the following definitions are set forth to better explain the present invention.

As used herein, the term "alkylene" refers to a saturated divalent hydrocarbon radical, preferably a saturated divalent hydrocarbon radical having 1, 2, 3, 4, 5 or 6 carbon atoms, such as methylene, ethylene, propylene or butylene.

As used herein, the term "alkyl" is defined as a linear or branched saturated aliphatic hydrocarbon radical. In some embodiments, the alkyl has 1 to 12, for example, 1 to 6 carbon atoms. For example, as used herein, the term "C₁₋₆ alkyl" refers to a linear or branched aliphatic hydrocarbon radical having 1 to 6 carbon atoms (e.g., methyl, ethyl, n-propyl, isopropyl, n-butyl, isobutyl, sec-butyl, tert-butyl, n-pentyl, isopentyl, neopentyl or n-hexyl), which may be optionally substituted by one or more (e.g., 1 to 3) suitable substituents (e.g., halogen) (the group is called "haloalkyl" in this case) (e.g., CH₂F, CHF₂, CF₃, CCl₃, C₂F₅, C₂Cl₅, CH₂CF₃, CH₂Cl or - CH₂CH₂CF₃, etc.). The term "C₁₋₄ alkyl" refers to a linear or branched aliphatic hydrocarbon radical having 1 to 4 carbon atoms (i.e. methyl, ethyl, n-propyl, isopropyl, n-butyl, isobutyl, sec-butyl or tert-butyl).

As used herein, the term "alkoxy" is defined as -O-alkyl, in which the alkyl is as defined above. For example, as used herein, the term "C₁₋₆ alkoxy" refers to -O-C₁₋₆ alkyl.

As used herein, the term "alkoxyalkyl" is defined as an alkyl substituted with an alkoxy, in which the alkyl is as defined above. For example, as used herein, the term "C₂₋₆ alkoxyalkyl" refers to an alkoxy-substituted alkyl having 2 to 6 carbon atoms.

As used herein, the term "alkynylene" refers to a divalent hydrocarbon radical comprising at least one carbon-carbon triple bond, preferably having 1, 2, 3, 4, 5 or 6 carbon atoms, for example ethynylene, propynylene or butynylene.

As used herein, the term "heterocyclyl", "heterocyclic ring" refers to a saturated or partially unsaturated (i.e., there are one or more double bonds and/or triple bonds in the ring) cyclic group in which at least one ring atom is a heteroatom selected from the group consisting of N, O, and S and the remaining ring atoms are C. For example, "5- to 12-membered heterocyclic ring (group)" is a saturated or partially unsaturated heterocyclic group having 4 to 11 (e.g., 2, 3, 4, 5, 6, 7, 8 or 9) ring-forming carbon atoms and one or more (e.g., 1, 2, 3 or 4) ring-forming heteroatoms independently selected from the group consisting of N, O and S. "5- to 12-membered nitrogen-containing heterocyclic ring (group)" is a heterocyclic ring (group) in which at least one ring-forming atom is N. Examples of the heterocyclic group include, but are not limited to: oxiranyl, aziridinyl, azetidinyl, oxetanyl, tetrahydrofuranyl, dioxolinyl, pyrrolidinyl, pyrrolidonyl, imidazolidinyl, pyrazolidinyl, pyrrolinyl, tetrahydropyranyl, piperidinyl, morpholinyl, dithianyl, thiomorpholinyl, piperazinyl or trithianyl. The heterocyclic group can be optionally substituted by one or more (for example, 1, 2, 3 or 4) suitable substituents, and can be optionally fused with one or more aromatic rings, heteroaryl rings to form a fused ring structure.

As used herein, the term "aromatic ring" or "aryl" refers to a monocyclic or polycyclic aromatic ring system, which has, for example, 5, 6, 8, 9, 10, 11, 12, 13 or 14 ring-forming carbon atoms, especially 1 or 2 or 3 or 4 or 5 or 6 or 9 or 10 carbon atoms.

As used herein, the term "heteroaromatic ring" or "heteroaryl" refers to a monocyclic or polycyclic aromatic ring system, which has, for example, 5, 6, 8, 9, 10, 11, 12, 13 or 14 ring atoms, in particular 1, 2, 3, 4, 5, 6, 9 or 10 carbon atoms, and at least one heteroatom that may be the same or different (the heteroatom is, for example, oxygen, nitrogen or sulfur), and, which additionally in each case may be benzo-fused.

As used herein, the term "halogen" includes F, Cl, Br or I.

As used herein, the term "sulfonylurea group" refers to -SO₂-NH-(C=O)-NH₂ or -NH-(C=O)-NH-SO₂H.

The term "substitution" refers to that one or more (e.g., one, two, three or four) hydrogens on the designated atom are substituted by an indicated group, provided that the substitution number does not exceed the normal valence of the designated atom in the present case and such substitution results in a stable compound. Combinations of substituents and/or variables are permissible only if such combinations result in stable compounds.

If a substituent is described as being "optionally substituted", the substituent can be (1) unsubstituted or (2) substituted. If a carbon of a substituent is described as being optionally substituted with one or more of the listed substituents, one or more hydrogens on the carbon (to the extent of any hydrogen present) may be individually and/or together substituted by the independently selected optional substituent. If a nitrogen of a substituent is described as being optionally substituted with one or more of the listed substituents, one or more hydrogens on the nitrogen (to the extent of any hydrogen present) may each be substituted by the independently selected optional substituent.

If substituents are described as being "independently selected from" a group, each substituent is selected independently between each other. Accordingly, each substituent may be the same as or different from another (other) substituent.

As used herein, the term "one or more" means 1 or more than 1, such as 2, 3, 4, 5 or 10, under reasonable conditions.

As used herein, unless otherwise indicated, the linking site of a substituent may be from any suitable position of the substituent.

When a substituent has a bond that is shown to pass through a bond connecting two atoms in a ring, such substituent may be bonded to any ring-forming atom in the ring to be substituted.

The present invention also comprises all pharmaceutically acceptable isotopically labeled compounds which are identical to the compounds of the present invention except that one or more atoms have been substituted with the corresponding atoms that have same atomic number but different atomic mass or mass number from the atomic mass or mass number prevailing in nature. Examples of isotopes suitable for inclusion in compounds of the present invention include, but are not limited to, isotopes of hydrogen (e.g., deuterium (²H), tritium (³H)); isotopes of carbon (e.g., ¹¹C, ¹³C, and ¹⁴C); isotopes of chlorine (e.g., ³⁶Cl); isotopes of fluorine (e.g., ¹⁸F); isotopes of iodine (e.g., ¹²³I and ¹²⁵I); isotopes of nitrogen (e.g., ¹³N and ¹⁵N); isotopes of oxygen (e.g., ¹⁵O, ¹⁷O and ¹⁸O); isotopes of phosphorus (e.g., ³²P); and isotopes of sulfur (e.g., ³⁵S).

The term "stereoisomer" refers to an isomer formed as a result of at least one asymmetric center. If a compound has one or more (e.g., one, two, three or four) asymmetric centers, it can give rise to a racemic mixture, a single enantiomer, a diastereomeric mixture and an individual diastereoisomer. Certain individual molecules may also be present as geometric isomers (cis/trans). Similarly, the compound of the present invention may exist as a mixture of two or more structurally distinct forms (commonly referred to as tautomers) in rapid equilibrium. Representative examples of tautomers include keto-enol tautomers, phenol-keto tautomers, nitroso-oxime tautomers, imineenamine tautomers, etc. It should be understood that the scope of the present application encompasses all such isomers or mixtures thereof in any ratios (e.g., 60%, 65%, 70%, 75%, 80%, 85%, 90%, 95%, 96%, 97%, 98%, 99%).

Carbon-carbon bond of the compound of the present invention may be depicted herein using solid line ( 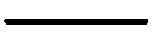 ), solid wedge ( 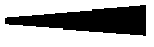 ), or dashed wedge ( 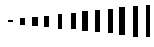 ). The use of a solid line in delineating a bond to an asymmetric carbon atom is intended to indicate that all possible stereoisomers resulted from that carbon atom are included (e.g., specific enantiomers, racemic mixture, etc.). The use of a solid or dashed wedge in delineating a bond to an asymmetric carbon atom is intended to indicate the stereoisomer as shown. When being used in a racemic mixture, solid and dashed wedges are to define relative stereochemistry rather than absolute stereochemistry. Unless otherwise indicated, the compound of the present invention is intended to be present in the form of stereoisomers (which include cis and trans isomers, optical isomers (e.g., R and S enantiomers), diastereomers, geometric isomers, rotamers, conformational isomers, atropisomers, and any mixture thereof). The compound of the present invention may exhibit more than one type of isomerism and consist of a mixture thereof (e.g., racemic mixture and pairs of diastereoisomers).

The present invention encompasses all possible crystalline forms or polymorphs of the compound of the present invention, which may be a single polymorph or a mixture of more than one polymorphs in any ratio.

It will also be understood that certain compounds of the present invention may exist in free form for use in therapy, or, where appropriate, in the form of pharmaceutically acceptable derivative thereof. In the present invention, the pharmaceutically acceptable derivative includes, but is not limited to, pharmaceutically acceptable salt, ester, solvate, N-oxide, metabolite or prodrug, when it is administered to a patient in need thereof, it can directly or indirectly provide the compound of the present invention or metabolite or residue thereof. Therefore, when a "compound of the present invention" is referred to herein, it is also intended to cover the above-mentioned various derivative forms of the compound.

The pharmaceutically acceptable salt of the compound of the present invention includes acid addition salt and base addition salt thereof.

Suitable acid addition salts are formed from acids which can form pharmaceutically acceptable salts. Examples include aspartate, benzoate, bicarbonate/carbonate, glucoheptonate, gluconate, nitrate, orotate, palmitate and other similar salts.

Suitable base addition salts are formed from bases which can form pharmaceutically acceptable salts. Examples include aluminum salts, arginine salts, choline salts, magnesium salts and other similar salts.

A review of suitable salts is referred to "Handbook of Pharmaceutical Salts: Properties, Selection, and Use" by Stahl and Wermuth (Wiley-VCH, 2002). Methods for preparing pharmaceutically acceptable salts of the compound of the present invention are known to those skilled in the art.

As used herein, the term "ester" refers to an ester derived from each of the compounds of the general Formula in the present application, including physiologically hydrolyzable ester (hydrolyze under physiological conditions to release the compound of the present invention in the form of free acid or alcohol). The compound of the present invention may also itself be ester.

The compound of the present invention may exist in the form of solvate, preferably hydrate, wherein the compound of the present invention comprises a polar solvent, such as water, methanol or ethanol in particular, as a structural element of the crystal lattice of the compound. The amount of polar solvent, especially water, may be present in a stoichiometric or non-stoichiometric ratio.

Those skilled in the art will appreciate that not all nitrogen-containing heterocyclic rings are capable of forming N-oxides since nitrogen requires available lone pair of electrons for oxidization to form oxides; those skilled in the art will recognize a nitrogen-containing heterocyclic ring capable of forming N-oxide. Those skilled in the art will also recognize that tertiary amines are capable of forming N-oxides. Synthetic methods for the preparation of N-oxides of heterocycles and tertiary amines are well known to those skilled in the art, including oxidizing heterocycles and tertiary amines with peroxyacid such as peracetic acid and m-chloroperbenzoic acid (MCPBA), hydrogen peroxide, alkyl hydroperoxide such as t-butyl hydroperoxide, sodium perborate and dioxirane such as dimethyldioxirane. These methods for preparing *N*-oxides have been extensively described and reviewed in the literature, see for example: T. L. Gilchrist, Comprehensive Organic Synthesis, vol. 7, pp 748-750; A. R. Katritzky and A. J. Boulton, Eds., Academic Press and G. W. H. Cheeseman and E. S. G. Werstiuk, Advances in Heterocyclic Chemistry, vol. 22, pp 390-392, A. R. Katritzky and A. J. Boulton, Eds., Academic Press.

Also included within the scope of the present invention is a metabolite of the compound of the present invention, i.e., a substance formed in vivo upon administration of the compound of the present invention. Such product may result, for example, from oxidation, reduction, hydrolysis, amidation, deamidation, esterification, enzymatic hydrolysis, etc., of the administered compound. Accordingly, the present invention comprises a metabolite of the compound of the present invention, including a compound produced by a method of contacting the compound of the present invention with a mammal for a time sufficient to produce a metabolite thereof.

The present invention further includes within its scope a prodrug of the compound of the present invention, which is a certain derivative of the compound of the present invention and may have little or no pharmacological activity *per se,* but can be converted to the compound of the present invention having the desired activity by, for example, hydrolytic cleavage, when administered into or on the body. Typically, such prodrug may be a functional group derivative of the compound, which can be readily converted in vivo into the compound with the desired therapeutic activity. Additional information on the use of prodrugs can be found in "Pro-drugs as Novel Delivery Systems", Volume 14, ACS Symposium Series (T. Higuchi and V. Stella). The prodrug of the present invention can be prepared, for example, by substituting an appropriate functional group present in the compound of the present invention with a certain moiety known to those skilled in the art as "pro-moiety (e.g., described in "Design of Prodrugs", H. Bundgaard (Elsevier, 1985))".

The present invention also encompasses the compound of the present invention which contains a protecting group. During any processes for preparing the compound of the present invention, it may be necessary and/or desirable to protect a sensitive or reactive group on any of the molecules involved, thereby forming a chemically protected form of the compound of the present invention. This can be accomplished by using conventional protecting groups, for example, those described in T.W. Greene & P.G.M. Wuts, Protective Groups in Organic Synthesis, John Wiley & Sons, 1991, and these references are incorporated herein by reference. Protecting groups may be removed at an appropriate subsequent stage using methods known in the art.

### Preparation method

Another aspect of the present invention provides a method for preparing the compound shown in Formula I, and the method comprises the following steps:
when Y is absent, the compounds of Formula I-TM1 and Formula I-TM2 of the present invention can be synthesized via the following synthetic route. 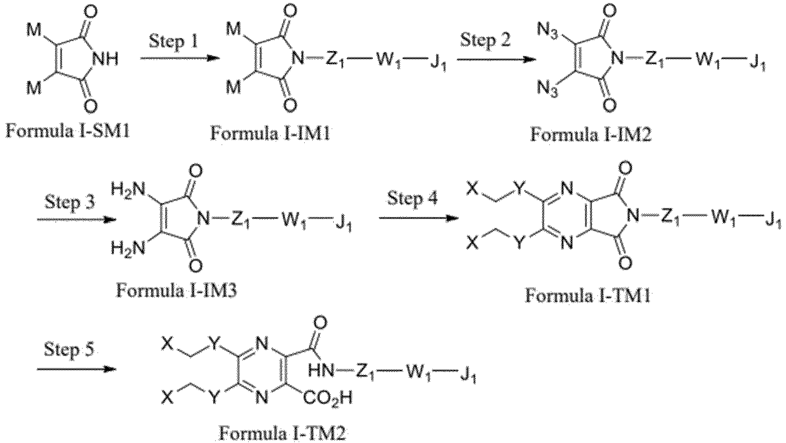
wherein:
   X, Z₁, Wi, J₁ are as defined in the general Formulas above;
   M is a leaving group that undergoes a substitution reaction, including but not limited to halogen, trifluoromethanesulfonate, p-toluenesulfonate, preferably halogen.

### Step 1

A compound of Formula I-IM1 is obtained by a substitution reaction between a compound of Formula I-SM1 and a compound of M-Z₁-W₁-J₁;
In some embodiments, the reaction is carried out under basic condition;
In some embodiments, the reaction is carried out at a suitable temperature, and the temperature is 20°C, 25°C, 50°C, 60°C, 100°C, preferably 20°C;
In some embodiments, the reaction is carried out in a suitable solvent, and the solvent includes but is not limited to acetone, tetrahydrofuran, dichloromethane, N,N-dimethylformamide, N-methylpyrrolidone, dimethyl sulfoxide, preferably acetone.

### Step 2

A compound of Formula I-IM2 is obtained by a substitution reaction between a compound of Formula I-IM1 and sodium azide;
In some embodiments, the reaction is carried out at a suitable temperature, and the temperature is 20°C, 25°C, 50°C, 60°C, 100°C, preferably 20°C;
In some embodiments, the reaction is carried out in a suitable solvent, and the solvent includes but is not limited to acetone, tetrahydrofuran, dichloromethane, N,N-dimethylformamide, N-methylpyrrolidone, dimethyl sulfoxide, preferably acetone.

### Step 3

A compound of Formula I-IM3 is obtained through a reduction reaction of the compound of Formula I-IM2;
In some embodiments, this step is carried out in the presence of a suitable reducing agent, and the reducing agent can be selected from the group consisting of palladium catalyst, platinum catalyst, rhodium catalyst, preferably palladium catalyst;
In some embodiments, this step is carried out at a suitable temperature, and the temperature is 20°C, 25°C, 50°C, 60°C, 100°C, preferably 20°C;
In some embodiments, this step is carried out in a suitable organic solvent, and the organic solvent can be selected from the group consisting of tetrahydrofuran, dichloromethane, N,N-dimethylformamide, N-methylpyrrolidone, dimethyl sulfoxide, n-heptane, n-hexane, ethyl acetate, preferably ethyl acetate.

### Step 4

A compound of Formula I-TM1 is obtained by a ring-closing reaction between a compound of Formula I-IM3 and 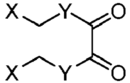 by dehydration;
In some embodiments, this step is carried out at a suitable temperature, and the temperature is 20°C, 25°C, 50°C, 60°C, 100°C, preferably 20°C;
In some embodiments, this step is carried out in a suitable organic solvent, and the organic solvent can be selected from the group consisting of acetonitrile, ethanol, tetrahydrofuran, dichloromethane, N,N-dimethylformamide, dimethyl sulfoxide, n-heptane, n-hexane, ethyl acetate, preferably acetonitrile.

### Step 5

A compound of Formula I-TM2 is obtained by the hydrolyzation of a compound of Formula I-TM1;
In some embodiments, the reaction is carried out under basic condition, and the basic condition includes but is not limited to PB buffer with a pH of 7.0, 7.4 or 8.0;
In some embodiments, the reaction is carried out at a suitable temperature, and the temperature is 20°C, 25°C, 50°C, 60°C, 100°C, preferably 20°C;
In some embodiments, the reaction is carried out in a suitable solvent, and the solvent includes but is not limited to acetonitrile, tetrahydrofuran, dichloromethane, N,N-dimethylformamide, dimethyl sulfoxide, preferably acetonitrile.

When Y is absent, the compound of Formula I-TM3 of the present invention can be synthesized via the following synthetic route. 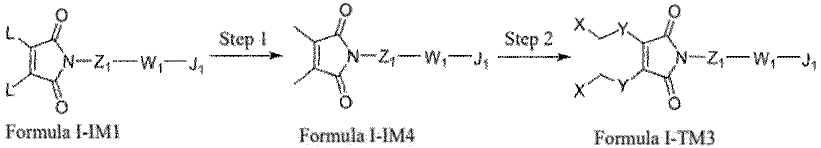 wherein:
X, Z₁, Wi, J₁ are as defined in the general formulas above;
L is a leaving group that undergoes a substitution reaction, including but not limited to halogen, trifluoromethanesulfonate, p-toluenesulfonate, preferably halogen, OTf.

### Step 1

A compound of Formula I-IM4 is obtained from a compound of Formula I-IM1 through a coupling reaction;
In some embodiments, the reagent used in the coupling reaction including but not limited to methylboronic acid, trimethylboroxine, preferably trimethylboroxine;
In some embodiments, the coupling reaction is carried out in basic condition, and the base includes but is not limited to triethylamine, DIPEA, NMM, sodium tert-butoxide, potassium acetate, sodium acetate, cesium fluoride, potassium fluoride, potassium carbonate, sodium carbonate, sodium bicarbonate, cesium carbonate, potassium phosphate, potassium dihydrogen phosphate, preferably cesium fluoride.

In some embodiments, the coupling reaction is carried out in the presence of a catalyst, which includes but is not limited to tetrakis(triphenylphosphine) palladium, palladium acetate, Pd₂(dba)₃, Pd(PPh₃)₂Cl₂, Pd(PPh₃)₂Cl₂ dichloromethane complex, Pd(dppf)Cl₂, Pd(Amphos)Cl₂, preferably tetrakis(triphenylphosphine) palladium.

In some embodiments, the coupling reaction is carried out at a temperature of 0 to 200 °C, preferably at a temperature of 50 to 150 °C.

In some embodiments, the reaction is carried out in a suitable solvent, and the solvent includes but is not limited to 1,4-dioxane, water, toluene, tetrahydrofuran, N,N-dimethylformamide, N-methylpyrrolidone, dimethyl sulfoxide and any combination thereof, preferably 1,4-dioxane.

### Step 2

A compound of Formula I-TM3 is obtained from a compound of Formula I-IM4 through a halogenation reaction;
In some embodiments, the reagent used in the halogenation reaction includes but is not limited to bromine, N-iodosuccinimide, N-bromosuccinimide, N-chlorosuccinimide, preferably N-bromo succinimide;
In some embodiments, the coupling reaction is carried out in the presence of a catalyst, and the catalyst is benzoyl peroxide.

In some embodiments, the conjugation reaction is carried out at a temperature of 0 to 200 °C, preferably at a temperature of 50 to 150 °C.

In some embodiments, the reaction is carried out in a suitable solvent, and the solvent includes but is not limited to halogenated hydrocarbons (e.g., carbon tetrachloride, dichloromethane, chloroform, 1,2-dichloroethane, etc.), methanol, ethanol, DMF, acetonitrile, ethers (e.g., ethylene glycol dimethyl ether, tetrahydrofuran, dioxane), aromatic hydrocarbons (e.g., toluene, benzene, xylene), water and any combination thereof, preferably carbon tetrachloride.

When Z₂ and W₂ are absent, the compound of Formula II-TM1 of the present invention can be synthesized via the following synthetic route. 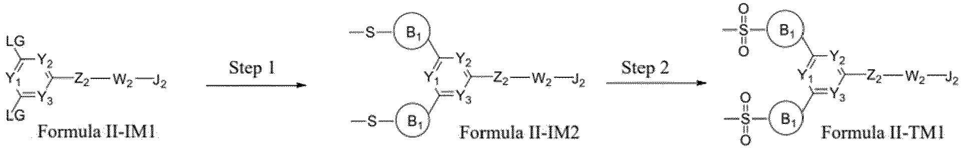 wherein:
Y₁, Y₂, Y₃, B₁, J₂ are as defined in the general formulas above;
LG is a leaving group that undergoes a coupling reaction, including but not limited to halogen, trifluoromethanesulfonate, preferably halogen.

### Step 1

A compound of Formula II-IM2 is obtained from a compound of Formula II-IM 1 through a coupling reaction;
In some embodiments, the reagent used in the coupling reaction is 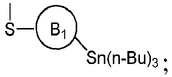
In some embodiments, the coupling reaction is carried out in the presence of a catalyst, which includes but is not limited to tetrakis(triphenylphosphine) palladium, palladium acetate, Pd₂(dba)₃, Pd(PPh₃)₂Cl₂, Pd(PPh₃)₂Cl₂ dichloromethane complex, Pd(dppf)Cl₂, Pd(Amphos)Cl₂, preferably tetrakis(triphenylphosphine) palladium.

In some embodiments, the coupling reaction is carried out at a temperature of 0 to 200 °C, preferably at a temperature of 50 to 150 °C.

In some embodiments, the reaction is carried out in a suitable solvent, and the solvent includes but is not limited to 1,4-dioxane, water, toluene, tetrahydrofuran, N,N-dimethylformamide, N-methylpyrrolidone, dimethyl sulfoxide and any combination thereof, preferably 1,4-dioxane.

### Step 2

A compound of Formula II-TM1 is obtained from a compound of Formula II-IM2 through an oxidation reaction;
In some embodiments, the conjugation reaction is carried out in the presence of an oxidizing agent, and the catalyst is m-chloroperoxybenzoic acid.

In some embodiments, the conjugation reaction is carried out at a temperature of 0 to 120 °C, preferably at a temperature of 50 to 80 °C.

In some embodiments, the reaction is carried out in a suitable solvent, and the solvent includes but is not limited to halogenated hydrocarbons (e.g., carbon tetrachloride, dichloromethane, chloroform, 1,2-dichloroethane, etc.), methanol, ethanol, DMF, acetonitrile, ethers (e.g., ethylene glycol dimethyl ether, tetrahydrofuran, dioxane), aromatic hydrocarbons (e.g., toluene, benzene, xylene), water and any combination thereof, preferably methanol.

In addition, the compounds of the present invention can also be prepared in a variety of ways known to those skilled in the art of organic synthesis. The compounds of the present invention can be synthesized using the methods described below as well as those known in the art of synthetic organic chemistry or variations thereof known to those skilled in the art. Preferred methods include but are not limited to those described above. The reactions can be conducted in a solvent or a mixed solvent appropriate to the reagents and materials employed and suitable for the conversion. Those skilled in the art of organic synthesis will appreciate that the functional groups present on the molecule should be consistent with the proposed conversion. This will sometimes require judgment to modify the order of synthetic steps or to select a particular route over another to obtain the desired compound of the present invention.

It will also be recognized that another major consideration in the design of any synthetic route in the art is the proper choice of protecting groups for protecting the reactive functional groups present in the compounds described in the present invention. Authoritative instructions describing many alternatives to those skilled in the art are provided by Greene et al. (Protective Groups in Organic Synthesis, 4th edition, Wiley-Interscience (2006)).

Unless otherwise stated, the substituents of the compounds in the above routes are as defined herein. Those skilled in the art will understand that one or more steps in the above routes can be skipped according to the structure of the desired product. Those skilled in the art can also appropriately adjust the sequence of the reaction steps as needed.

### Pharmaceutical composition

The present invention also provides a pharmaceutical composition, which comprises the bioactive conjugate described in the present invention and one or more pharmaceutically acceptable excipients.

The pharmaceutical excipients mentioned in this article refer to the excipients and additives used in the production of drugs and the formulation of prescriptions, and refer to the substances other than active ingredients, which have been reasonably evaluated in terms of safety and are included in the pharmaceutical preparation.

The pharmaceutical composition may be administered in any form as long as it achieves prevention, alleviation, stop or cure of a symptom of a human or animal patient. For example, various appropriate dosage forms can be prepared according to the routes of administration.

The present application also provides a kit product, which comprises the bioactive conjugate of the present invention, or the pharmaceutical composition, and optionally a drug instruction.

### Therapeutic method and use

Another aspect of the present application provides use of the bioactive conjugate in the manufacture of a medicament for preventing or treating a tumor disease.

Another aspect of the present application provides the bioactive conjugate, for use in preventing or treating a tumor disease.

Another aspect of the present application provides a method for preventing or treating a tumor disease, which comprises administering to a subject in need thereof an effective amount of the bioactive conjugate, or the pharmaceutical composition comprising the bioactive conjugate.

In one embodiment of the present invention, the tumor disease is a solid tumor or hematological malignancy; for example, the tumor disease is selected from the group consisting of colon cancer, gastric cancer, breast cancer, lung cancer (e.g., non-small cell lung cancer, specifically lung adenocarcinoma), lymphoma.

The term "effective amount" as used herein refers to an amount of the conjugate which, when administered, alleviates to some extent one or more symptoms of the disease being treated.

As used herein, unless otherwise stated, the term "treating" means reversing, alleviating disease or condition being treated, or the progression of one or more symptoms of such disease or condition.

An "individual" or "subject" as used herein includes a human or non-human animal. Exemplary human subject includes a human subject suffering from a disease (e.g., a disease described herein) (referred to as a patient) or normal subject. "Non-human animal" in the present invention includes all vertebrates, such as non-mammal (e.g., bird, amphibian, reptile) and mammal, such as non-human primate, livestock and/or domesticated animal (e.g., sheep, dog, cat, cow, pig, etc.).

### Specific Models for Carrying Out the invention

Examples and experimental examples are given below to describe the present invention in detail, but not to limit the scope of the present invention, and can be changed without departing from the scope of the present invention.

### 1. Preparation of anti-ROR1 antibody 19F6_Hu35V1 involved in the present invention

In the early stage, by immunizing Balb/c, C57B1/6, NZB and A/J mice and hybridoma screening, the mouse antibody 19F6 was obtained, which was humanized to obtain humanized antibody 19F6_Hu35V1 (heavy chain variable region, SEQ ID NO:1; light chain variable region, SEQ ID NO:2), and the heavy chain constant region of the above humanized antibody was human IgG1 heavy chain constant region (SEQ ID NO: 18), and the light chain constant region was human kappa light chain constant region (SEQ ID NO: 19). After synthesis and codon-optimization of the coding DNA sequences of the above humanized antibody, it was cloned into pcDNA3.4 plasmid, and the pcDNA3.4 plasmid corresponding to the heavy and light chains of the humanized antibody was simultaneously transfected into Expi293F cells. Protein A was used to purify the expressed antibody in the supernatant to obtain the corresponding antibody.

**Table of sequence information of 19F6_Hu35V1:**

| Antibody | Numbering system | SEQ ID NO: | | | | | | | | | |
|---|---|---|---|---|---|---|---|---|---|---|---|
| | | CD R H1 | CD R H2 | CD R H3 | CD R L1 | CD R L2 | CD R L3 | V H | V L | C H | C L |
| 19F6_Hu35 V1 | Chothia | 3 | 4 | 5 | 6 | 7 | 8 | 1 | 2 | 18 | 19 |
| | AbM | 9 | 10 | 5 | 6 | 7 | 8 | | | | |
| | Kabat | 11 | 12 | 5 | 6 | 7 | 8 | | | | |
| | IMGT | 13 | 14 | 15 | 16 | 17 | 8 | | | | |

### 2. Example of synthesizing drug-linker involved in the present invention.

### Abbreviations as used herein have the following meanings:

| | | | |
|---|---|---|---|
| NBS | Bromosuccinimide | DMF | N,N-Dimethylformamide |
| HOBt | 1-Hydroxybenzotriazole | DIPEA | N,N-Diisopropylethylamine |
| DIC | N,N-Diisopropylcarbodiimide | HATU | O-(7-Azobenzotriazol-1-oxy)- N,N,N',N'-tetramethyluronium hexafluorophosphate |
| EEDQ | 2-Ethoxy-1-ethoxycarbonyl-1,2-dihydroquinoline | DIAD | Diisopropyl azodicarboxylate |

The structures of the compounds described in the following Examples were identified by nuclear magnetic resonance (¹H NMR) or mass spectrometry (MS).

The nuclear magnetic resonance (¹H NMR) was determined by using a Bruker 400 MHz nuclear magnetic resonance instrument; deuterated chloroform (CDCl₃); and the internal standard substance was tetramethylsilane (TMS).

Abbreviations used in Examples in interpretation the nuclear magnetic resonance (NMR) spectra were shown below.

s: singlet, d: doublet, t: triplet, q: quartet, m: multiplet, br: broad, J: coupling constant, Hz: Hertz, CDCl₃: deuterated chloroform. The δ values were expressed in ppm.

The mass spectrum (MS) was determined by using an Agilent (ESI) mass spectrometer, model Agilent 6120B.

### Examples of chemical synthesis

### Example 1: 2-(2,3-Bis(bromomethyl)-5,7-dioxo-5,7-dihydro-6H-pyrrolo[3,4-b]pyrazin- 6-yl)acetic acid (I-1)

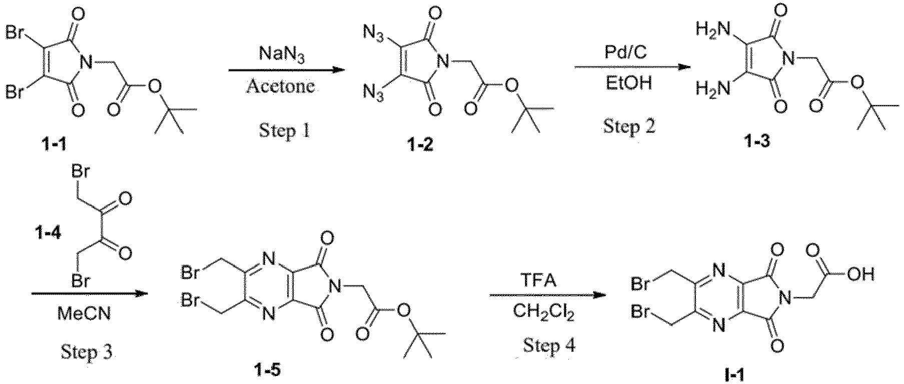

### Step 1: Synthesis of tert-butyl 2-(3,4-diazido-2,5-dioxo-2,5-dihydro-1H-pyrrol-1-yl)acetate (1-2)

Compound 1-1 (0.40 g, 1.08 mmol, referring to WO2019057964 for its synthesis) and sodium azide (141.00 mg, 2.17 mmol) were dissolved in acetone (10 mL), reacted at 25°C for 8 h, and the reaction was monitored by high performance liquid chromatography-mass spectrometry. The reaction solution was added with water and extracted with ethyl acetate, the organic phases were combined and dried over anhydrous sodium sulfate, filtered, and the filtrate was concentrated under reduced pressure to obtain 0.28 g of a crude product of the title compound, which was directly used in the next reaction without purification.

The structural identification data were as follows:
ESI-MS (m/z): 311.0[M+18]⁺.

### Step 2: Synthesis of tert-butyl 2-(3,4-diamino-2,5-dioxo-2,5-dihydro-1H-pyrrol-1-yl)acetate (1-3)

Compound **1-2** (0.15 g, 0.51 mmol) and 10% palladium on carbon (15.00 mg) were dissolved in ethanol (20 mL), subjected to replacement with hydrogen three times, reacted at 25°C for 3 h under hydrogen atmosphere, and the reaction was monitored by high performance liquid chromatography-mass spectrometry. The reaction solution was filtered with diatomite, and the filtrate was concentrated under reduced pressure to obtain 101.00 mg of a crude product of the title compound, which was directly used in the next reaction without purification.

The structural identification data were as follows:
ESI-MS (m/z): 185.9[M-56]⁺.

### Step 3: Synthesis of tert-butyl 2-(2,3-bis(bromomethyl)-5,7-dioxo-5,7-dihydro-6H-pyrrolo[3,4-b]pyrazin-6-yl)acetate (1-5)

Compound **1-3** (50.00 mg, 0.21 mmol) and compound **1-4** (54.00 mg, 0.21 mmol) were dissolved in acetonitrile (5 mL), reacted at 25°C for 3 h, and the reaction was monitored by high performance liquid chromatography-mass spectrometry. The reaction solution was directly purified by flash silica gel column (petroleum ether: ethyl acetate = 5:1) to obtain 40.00 mg of the title compound.

The structural identification data were as follows:
ESI-MS (m/z): 466.8[M+18]⁺.
¹H NMR (400 MHz, CDCl₃): δ 4.88 (s, 4H), 4.47 (s, 2H), 1.48 (s, 9H).

### Step 4: Synthesis of 2-(2,3-bis(bromomethyl)-5,7-dioxo-5,7-dihydro-6H-pyrrolo[3,4-b] pyrazin-6-yl)acetic acid (I-1)

Compound **1-5** (20.00 mg, 0.05 mmol) was dissolved in dichloromethane (5 mL) and trifluoroacetic acid (1 mL), reacted at 25°C for 5 h, and the reaction was monitored by high performance liquid chromatography-mass spectrometry. The reaction solution was directly concentrated under reduced pressure to obtain 15.00 mg of a crude product of the title compound, which was directly used in the next reaction without purification.

The structural identification data were as follows:
ESI-MS (m/z): 410.8[M+18]⁺.

### Example 2: 2-(3,4-bis(bromomethyl)-2,5-dioxo-2,5-dihydro-1H-pyrrol-1-yl)acetic acid (I-2)

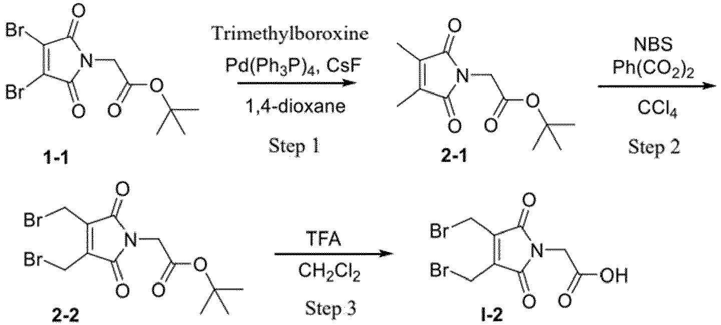

### Step 1: Synthesis of tert-butyl 2-(3,4-dimethyl-2,5-dioxo-2,5-dihydro-1H-pyrrol- 1-yl)acetate (2-1)

Under nitrogen protection, compound **1-1** (0.20 g, 0.54 mmol), trimethylboroxine (0.34 g, 2.71 mmol), cesium fluoride (0.41 g, 2.71 mmol) and tetrakis(triphenylphosphine) palladium (63.00 mg, 0.05 mmol) were dissolved in 1,4-dioxane (20 mL), reacted at 110°C for 3 h, and the reaction was monitored by high-performance liquid chromatography-mass spectrometry. The reaction solution was directly purified by flash silica gel column (petroleum ether: ethyl acetate = 5:1) to obtain 80.00 mg of the title compound.

The structural identification data were as follows:
ESI-MS (m/z): 184.0[M+H-56]⁺.

### Step 2: Synthesis of tert-butyl 2-(3,4-bis(bromomethyl)-2,5-dioxo-2,5-dihydro-1H-pyrrol- 1-yl)acetate (2-2)

Under nitrogen protection, compound **2-1** (72.00 mg, 0.30 mmol), NBS (118.00 mg, 0.66 mmol) and dibenzoyl peroxide (7.00 mg, 0.03 mmol) were dissolved in carbon tetrachloride (8 mL), reacted at 85°C for 12 h, and the reaction was monitored by high-performance liquid chromatography-mass spectrometry. The reaction solution was directly purified by flash silica gel column (petroleum ether: ethyl acetate = 10:1) to obtain 65.00 mg of the title compound.

The structural identification data were as follows:
ESI-MS (m/z): 414.8[M+18]⁺.
¹H-NMR (400 MHz, CDCl₃): δ 4.28 (s, 4H), 4.20 (s, 2H), 1.45 (s, 9H).

### Step 3: Synthesis of 2-(3,4-bis(bromomethyl)-2,5-dioxo-2,5-dihydro-1H-pyrrol-1-yl)acetic acid (I-2)

Compound **2-2** (60.00 mg, 0.15 mmol) was dissolved in dichloromethane (10 mL) and trifluoroacetic acid (2 mL), reacted at 25 °C for 5 h, and the reaction was monitored by high performance liquid chromatography-mass spectrometry. The reaction solution was directly concentrated under reduced pressure to obtain 50.00 mg of a crude product of the title compound, which was directly used in the next reaction without purification.

The structural identification data were as follows:
ESI-MS (m/z): 358.9[M+18]⁺.

### Example 3: 4-((S)-2-((S)-2-(2-(3,4-Bis(bromomethyl)-2,5-dioxo-2,5-dihydro-1H-pyrrol- 1-yl)acetamido)-3-methylbutylamido)-5-ureidopentanamido)benzyl ((S)-1-(((3R,4S,5S)-1-(((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate (drug-linker 3)

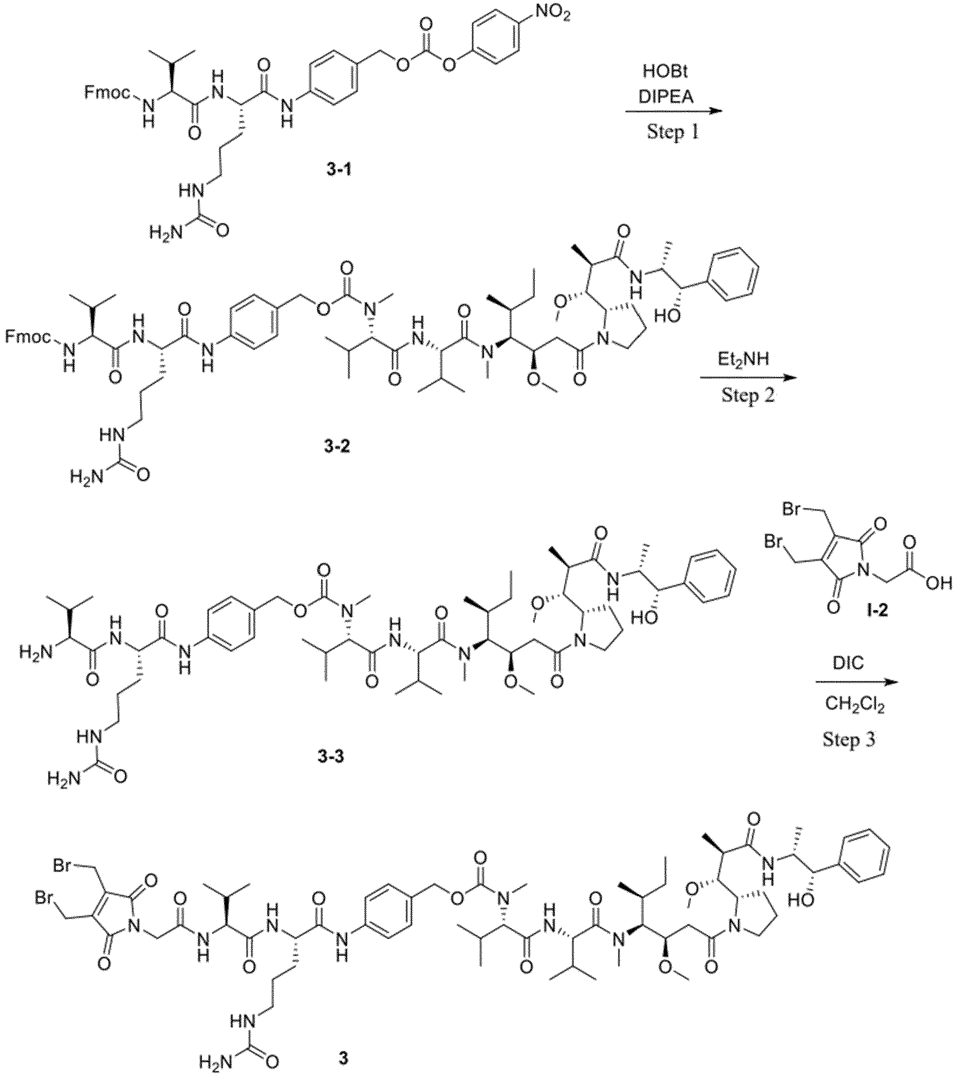

### Step 1: Synthesis of 4-((S)-2-((S)-2-((((9H-fluoren-9-yl)methoxy)carbonyl)amino)- 3-methylbutylamdo)-5-ureidopentanamido)benzyl ((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate (3-2)

At 25°C, (S)-N-((3R,4S,5S)-1-((S)-2-((1R,2R)-3-((1S,2R)-1-hydroxy-1-phenylpropan-2-yl) amino)-1-methoxy-2-methyl-3-oxopropyl)pyrrolidin-1-yl)-3-methoxy-5-methyl-1-oxyhept-4-yl)-N,3-dimethyl-2-((S)-3-methyl-2-(methylamino)butanamide (30.00 mg, 0.04 mmol) and compound **3-1** (38.50 mg, 0.05 mmol) were dissolved in DMF (3 mL), added with HOBt (8.46 mg, 0.06 mmol) and DIPEA (10.80 mg, 0.08 mmol), kept at 25°C and reacted for 2 h, and the reaction was monitored by high-performance liquid chromatography-mass spectrometry. The reaction solution was directly used in the next step without further treatment.

The structural identification data were as follows:
ESI-MS (m/z): 1345.2 [M+H]⁺.

### Step 2: Synthesis of 4-((S)-2-((S)-2-amino-3-methylbutylamido)-5-ureidopentanamido) benzyl ((S)-1-(((S))-1-(((3R,4S,5S)-1-((S)-2-((1R,2R)-3-(((1S,2R)-1-hydroxy-1-phenylpropan-2-yl) amino)-1-methoxy-2-methyl-3-oxopropyl)pyrrolidin-1-yl)-3-methoxy-5-methyl-1-oxoheptan-4-yl) (methyl)amino)-3-methyl-1-oxobutan-2-yl)amino)-3-methyl-1-oxobutan-2-yl(methyl)carbamate (3-3)

At 25°C, diethylamine (0.30 mL) was added to the reaction solution of compound **3-2,** reacted at 25°C for 2 h, and the reaction was monitored by high performance liquid chromatography-mass spectrometry. The reaction solution was concentrated under reduced pressure, the concentrate was directly purified by preparative high-performance liquid chromatography, and the fractions were lyophilized to obtain 36.30 mg of a formate salt of the title compound.
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 30 | 70 | 28 |
| 18.00 | 90 | 10 | 28 |

The structural identification data were as follows:
ESI-MS (m/z): 1123.2 [M+H]⁺.

### Step 3: Synthesis of 4-((S)-2-((S)-2-(2-(3,4-bis(bromomethyl)-2,5-dioxo-2,5-dihydro-1H-pyrrol-1-yl)acetamido)-3-methylbutylamido)-5-ureidopentanamido)benzyl ((S)-1-(((3R,4S,5S)-1- (((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate (3)

Compound **I-2** (12.75 mg, 0.04 mmol) was dissolved in dichloromethane (3 mL), added with DIC (2.36 mg, 0.02 mmol) and compound **3-3** (21.00 mg, 0.02 mmol), and reacted at 25°C for 1 h, and the reaction was monitored by high-performance liquid chromatography-mass spectrometry. The reaction solution was concentrated under reduced pressure, the concentrate was directly purified by preparative high performance liquid chromatography, and the fractions were lyophilized to obtain 14.30 mg of the title compound.
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 30 | 70 | 28 |
| 17.00 | 90 | 10 | 28 |

The structural identification data were as follows:
ESI-MS (m/z): 1445.9[M+H]⁺.

### Example 4: (S)-2-((2S,13S)-13-benzyl-22-(3,4-bis(bromomethyl)-2,5-dioxo-2,5-dihydro-1H-pyrrol-1-yl)-2-isopropyl-3-methyl-4,9,12,15,18,21-hexaoxo-6-oxa-3,8,11,14,17,20-hexaazadocosanamido)-N-((3R,4S,SS)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethylbutanamide (drug-linker 4)

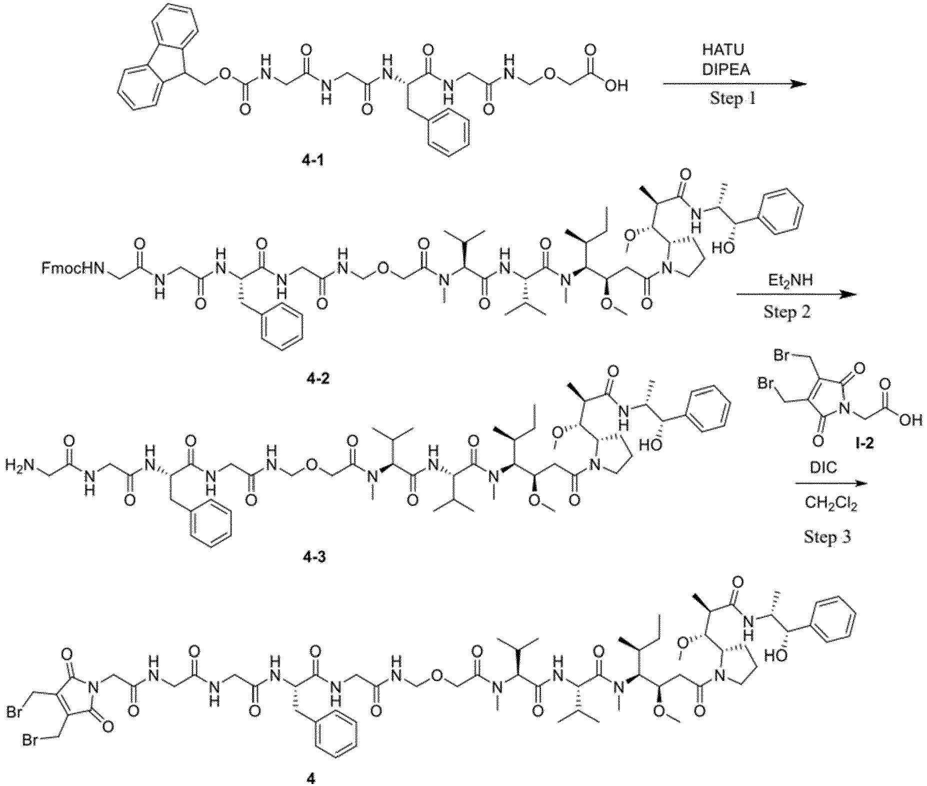

### Step 1: Synthesis of (9H-fluoren-9-yl)methyl((3R,4S,7S,10S,21S)-21-benzyl-4-((S)-sec-butyl)-3-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-7,10-diisopropyl-5,11-dimethyl-6,9,12,17,20,23,26-heptaoxo-2,14-dioxa-5,8,11,16,19,22,25-heptylheptanediamido-27)carbamate (4-2)

Compound **4-1** (50.00 mg, 0.08 mmol) and (S)-N-((3R,4S,5S)-1-((S)-2-((1R,2R)-3-(((1S,2R)-1-hydroxy-1-phenylpropan-2-yl)amino)-1-methoxy-2-methyl-3-oxypropyl)pyrrolidin-1-yl)-3-methoxy-5-methyl-1-oxoheptan-4-yl)-N,3-dimethyl-2-((S)-3-methyl-2-(methylamino)butyrylamido)butanamide (55.60 mg, 0.08 mmol) were weighed and dissolved in DMF (1 mL), then added with HATU (32.37 mg, 85.18 µmol) and DIPEA (20.02 mg, 154.88 µmol), after the addition, the reaction was carried out at room temperature for 1 h and monitored by high performance liquid chromatography-mass spectrometry. The reaction solution was directly purified by preparative high performance liquid chromatography to obtain 35.00 mg of the title compound.
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 40 | 60 | 28 |
| 2.00 | 40 | 60 | 28 |
| 18.00 | 90 | 10 | 28 |

The structural identification data were as follows:
ESI-MS (m/z): 1345.1[M+H]⁺.

### Step 2: Synthesis of (S)-2-((2S,13S)-19-amino-13-benzyl-2-isopropyl-3-methyl-4,9,12, 15,18-pentaoxo-6-oxa-3,8,11,14,17-heptaneazone)-N-((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethylbutanamide (4-3)

Compound **4-2** (20.00 mg, 0.02 mmol) was dissolved in dichloromethane (2 mL), then added with diethylamine (1 mL), after the addition, the reaction was carried out at room temperature for 1 h and monitored by high performance liquid chromatography-mass spectrometry. The reaction solution was concentrated under reduced pressure, the concentrate was directly purified by preparative high performance liquid chromatography, and the fractions were lyophilized to obtain 15.00 mg of a formate salt of the title compound.
Chromatographic column: SunFire Prep C18 OBD 19mm×150mm×5.0µm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 10 | 90 | 28 |
| 2.00 | 10 | 90 | 28 |
| 18.00 | 90 | 10 | 28 |

The structural identification data were as follows:
ESI-MS (m/z): 1123.1[M+H]⁺.

### Step 3: Synthesis of (S)-2-((2S,13S)-13-benzyl-22-(3,4-bis(bromomethyl)-2,5-dioxo- 2,5-dihydro-1H-pyrrol-1-yl)-2-isopropyl-3-methyl-4,9,12,15,18,21-hexaoxo-6-oxa-3,8,11,14,17,20-hexaazadocosanamido)-N-((3R,4S,SS)-1-((S)-2-((1R,2R)-3-(((1S,2R)-1-hydroxy-1-phenylprop-2-yl)amino)-1-methoxy-2-methyl-3-oxopropyl)pyrrolidin-1-yl)-3-methoxy-5-methyl-1-oxohept-4-yl)-N,3-dimethylbutyramide (4)

Compound **I-2** (4.00 mg, 12.0 µmol) was dissolved in dichloromethane (3 mL), added with DIC (1.12 mg, 8.00 µmol) and compound **4-3** (6.58 mg, 6.00 µmol), and reacted at 25°C for 1 h, and the reaction was monitored by high-performance liquid chromatography-mass spectrometry. The reaction solution was concentrated under reduced pressure, the concentrate was directly purified by preparative high performance liquid chromatography, and the fractions were lyophilized to obtain 4.77 mg of the title compound.
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 35 | 65 | 28 |
| 18.00 | 90 | 10 | 28 |

The structural identification data were as follows:
ESI-MS (m/z): 1445.9[M+H]⁺.

### Example 5: N-(((3R,4S,7S,10S,21S)-21-benzyl-4-((S)-sec-butyl)-3-(2-((S)-2-((1R,2R)-3-(((1S,2R)-1-hydroxy-1-phenylprop-2-yl)amino)-1-methoxy-2-methyl-3-oxopropyl)pyrrolidin-1-yl)-2-oxoethyl)-7,10-diisopropyl-5,11 -dimethyl-6,9,12,17,20,23,26-heptaoxo-2,14-dioxa-5,8,11,16,19,22,25-heptaazaheptacosan-27-yl)-1-(2-(3,4-bis(bromomethyl)-2,5-dioxo-2,5-dihydro-1H-pyrrol-1-yl)acetamido)-3,6,9,12-tetraoxapentadecan-15-amide (drug-linker 5)

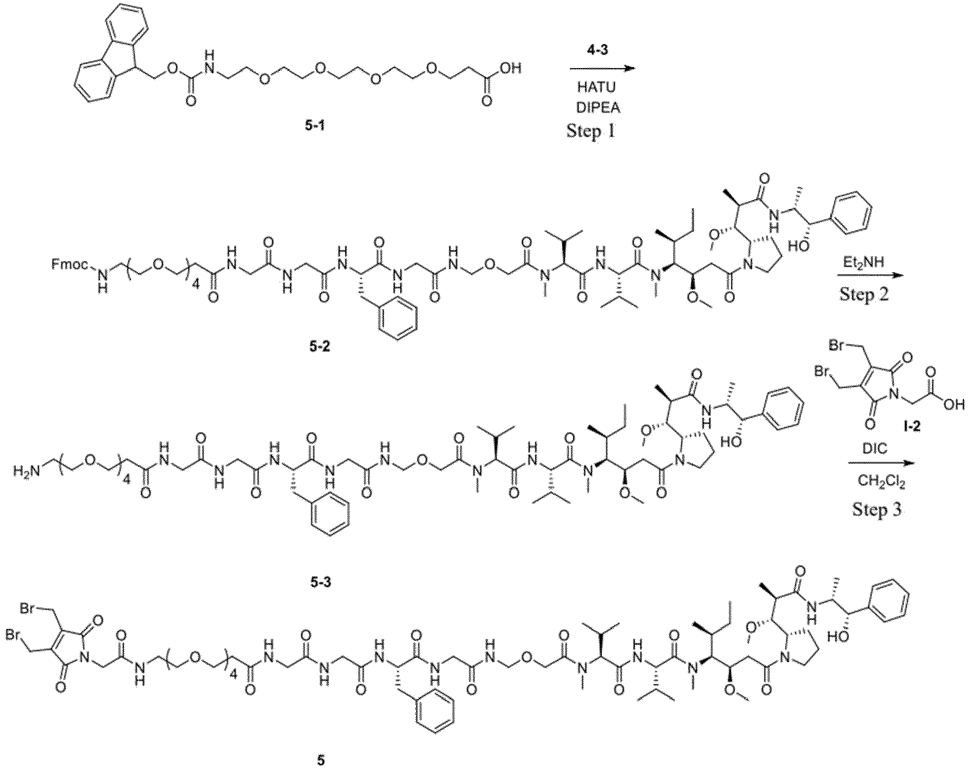

### Step 1: Synthesis of (9H-fluoren-9-yl)methyl((3R,4S,7S,10S,21S)-21-benzyl-4-((S)-sec-butyl)-3-(2-((S)-2-(((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-7,10-diisopropyl-5,11-dimethyl-6,9,12,17, 20,23,26,29-octaoxo-2,14,32,35,38,41-hexaoxa-5,8,11,16,19,22,25,28-octaazapentacosan-43-yl)carbamate (5-2)

Compounds **5-1** (5.21 mg, 11.00 µmol) and **4-3** (10.00 mg, 9.00 µmol) were weighed and dissolved in DMF (1 mL), then added with HATU (4.06 mg, 11.00 µmol) and DIPEA (2.30 mg, 18.00 µmol), after the addition, the reaction was carried out at room temperature for 1 h and monitored by high performance liquid chromatography-mass spectrometry. The reaction solution was directly purified by preparative high performance liquid chromatography to obtain 9.00 mg of the title compound.
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 20 | 80 | 28 |
| 16.00 | 90 | 10 | 28 |

The structural identification data were as follows:
ESI-MS (m/z): 1592.8[M+H]⁺.

### Step 2: Synthesis of 1-amino-N-(((3R,4S,7S,10S,21S)-21-benzyl-4-((S)-sec-butyl)-3-(2-((S)-2-((1R,2R)-3-(((S,2R)-1-hydroxy-1-phenylpropan-2-yl)amino)-1-methoxy-2-methyl-3-oxopropyl)pyrrolidin-1-yl)-2-oxoethyl)-7,10-diisopropyl-5,1 1-dimethyl-6,9,12,17,20,23,26-heptaoxo-2,14-dioxa-5,8,11,16,19,22,25-heptaazaheptacosan-27-yl)-3,6,9,12-tetraoxapentadecan-15-amide (5-3)

Compound **5-2** (10.00 mg, 6.00 µmol) was dissolved in dichloromethane (2 mL), then added with diethylamine (1 mL); after the addition, the reaction was carried out at room temperature for 1 h and monitored by high performance liquid chromatography-mass spectrometry. The reaction solution was concentrated under reduced pressure, the concentrate was directly purified by preparative high performance liquid chromatography, and the fractions were lyophilized to obtain 7.00 mg of a formate salt of the title compound.
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 10 | 90 | 28 |
| 20.00 | 90 | 10 | 28 |

The structural identification data were as follows:
ESI-MS (m/z): 1370.9[M+H]⁺.

### Step 3: Synthesis of N-(((3R,4S,7S,10S,21S)-21-benzyl-4-((S)-sec-butyl)-3-(2-((S)-2-((1R, 2R)-3-(((1S,2R)-1-hydroxy-1-phenylpropan-2-yl)amino)-1-methoxy-2-methyl-3-oxopropyl) pyrrolidin-1-yl)-2-oxoethyl)-7,10-diisopropyl-5,11-dimethyl-6,9,12,17,20,23,26-heptaoxo-2,14-dioxa-5,8,11,16,19,22,25-heptaazaheptacosan-27-yl)-1-(2-(3,4-bis(bromomethyl)-2,5-dioxo-2,5-dihydro-1H-pyrrol-1-yl)acetamido)-3,6,9,12-tetraoxopentadecanamide (5)

Compound **I-2** (4.00 mg, 12.00 µmol) was dissolved in dichloromethane (2 mL), added with DIC (1.12 mg, 8.00 µmol) and compound **5-3** (8.04 mg, 6.00 µmol), and reacted at 25°C for 1 h, and the reaction was monitored by high-performance liquid chromatography-mass spectrometry. The reaction solution was concentrated under reduced pressure, the concentrate was directly purified by preparative high performance liquid chromatography, and the fractions were lyophilized to obtain 4.68 mg of the title compound.
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 35 | 65 | 28 |
| 18.00 | 90 | 10 | 28 |

The structural identification data were as follows:
ESI-MS (m/z): 1693.7[M+H]⁺.

### Example 6: (S)-2-((2S,13S)-13-Benzyl-22-(2,3-bis(bromomethyl)-5,7-dioxo-5,7-dihydro-6H-pyrrolo[3,4-b]pyrazin-6-yl)-2-isopropyl-3-methyl-4,9,12,15,18,21-hexaoxo-6-oxa-3,8,11,14,17,20-hexaazadocosanamido)-N-(((3R,4S,SS)-1-((S)-2-((1R,2R)-3-(((1S,2R)-1-hydroxy-1-phenylpropan-2-yl)amino))-1-methoxy-2-methyl-3-oxopropyl)pyrrolidin-1-yl)-3-methoxy-5-methyl-1-oxoheptan-4-yl)-N,3-dimethylbutanamide (drug-linker 6)

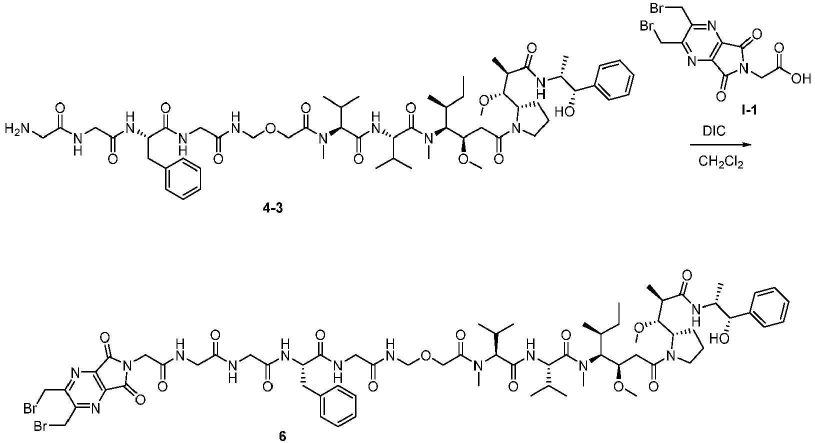

Compound **I-1** (6.03 mg, 15.00 µmol) was dissolved in dichloromethane (2 mL), added with DIC (1.45 mg, 12.00 µmol) and compound **4-3** (8.62 mg, 8.00 µmol), and reacted at 25°C for 1 h, and the reaction was monitored by high-performance liquid chromatography-mass spectrometry. The reaction solution was concentrated under reduced pressure, the concentrate was directly purified by preparative high performance liquid chromatography, and the fractions were lyophilized to obtain 4.21 mg of the title compound.
Column: SunFire Prep C18 OBD 5µm 19x150mm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 30 | 70 | 28 |
| 18.00 | 90 | 10 | 28 |

The structural identification data were as follows:
ESI-MS (m/z): 1498.5[M+H]⁺.

### Example 7: N-(((3R,4S,7S,10S,21S)-21-Benzyl-4-((S)-sec-butyl)-3-(2-((S)-2-((1R,2R)-3-((((1S,2R)-1-hydroxy-1-phenylpropan-2-yl)amino)-1-methoxy-2-methyl-3-oxopropyl)pyrrolidin-1-yl)-2-oxoethyl)-7,10-diisopropyl-5,11-dimethyl-6,9,12,17,20,23,26-heptaoxo-2,14-dioxa-5,8, 11,16,19,22,25-heptaazaheptacosan-27-yl)-1-(2-(2,3-bis(bromomethyl)-5,7-dioxo-5,7-dihydro-6H-pyrrolo[3,4-b]pyrazin-6-yl)acetamido)-3,6,9,12-tetraoxapentadecan-15-amide (drug-linker 7)

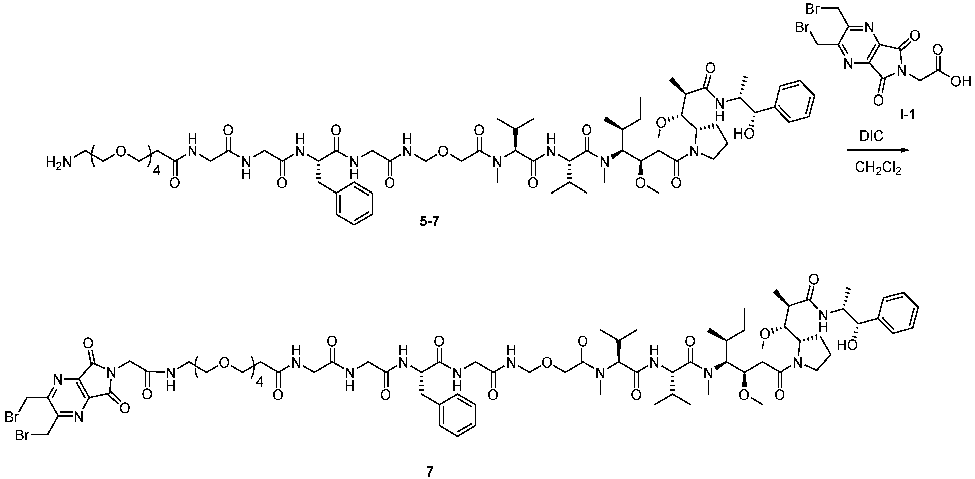

Compound **I-1** (12.06 mg, 0.03 mmol) was dissolved in dichloromethane (4 mL), added with DIC (3.00 mg, 0.02 mmol) and compound **5-3** (20.00 mg, 15.00 µmol), and reacted at 25°C for 1 h, and the reaction was monitored by high-performance liquid chromatography-mass spectrometry. The reaction solution was concentrated under reduced pressure, the concentrate was directly purified by preparative high performance liquid chromatography, and the fractions were lyophilized to obtain 12.00 mg of the title compound.
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 30 | 70 | 28 |
| 2.00 | 30 | 70 | 28 |
| 18.00 | 90 | 10 | 28 |

The structural identification data were as follows:
ESI-MS (m/z): 1745.8[M+H]⁺.

### Example 8: 4-((2S,SS)-41-(3,4-Bis(bromomethyl)-2,5-dioxo-2,5-dihydro-1H-pyrrol-1-yl)-5-isopropyl-2-methyl-4,7,11,40-tetraoxo-9,15,18,21,24,27,30,33,36-nonaoxa-3,6,12,39-tetraazapentadecanamido)phenyl ((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbonate (drug-linker 8)

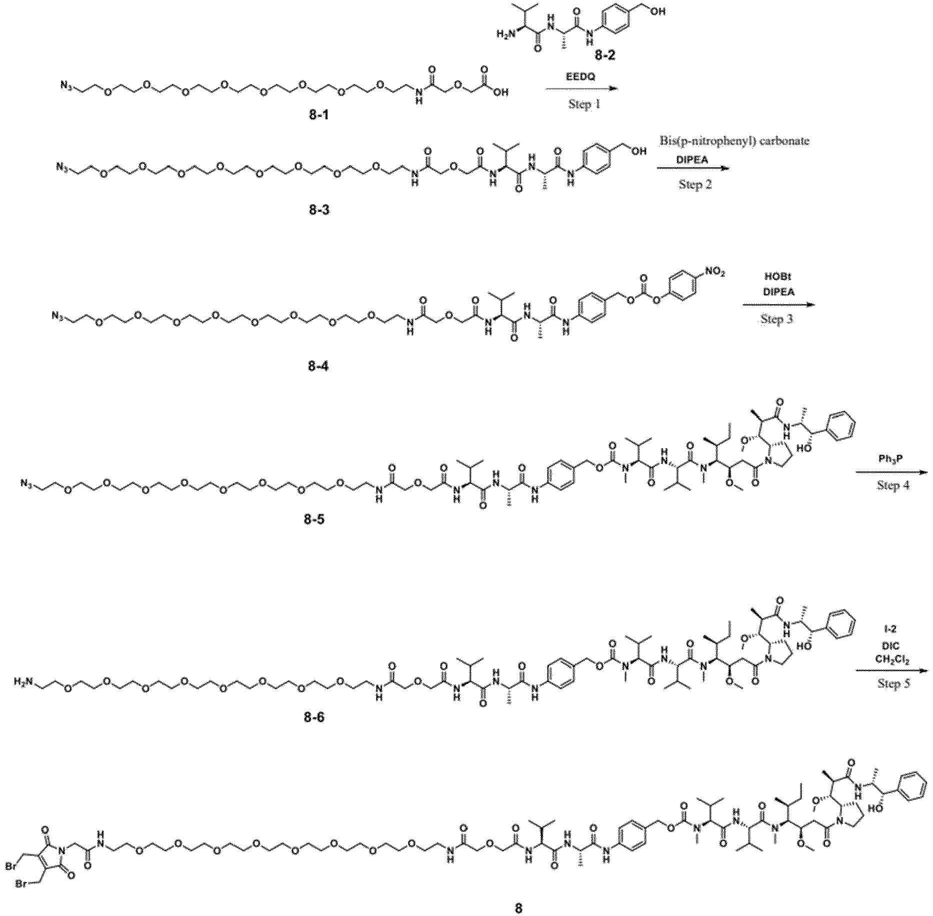

### Step 1: Synthesis of (S)-2-(32-azido-5-oxo-3,9,12,15,18,21,24,27,30-nonaoxa-6-azatetratriacontanamido)-N-((S)-1-((4-(hydroxymethyl)phenyl)amino)-1-oxopropan-2-yl)-3-methylbutanamine (8-3)

Compound **8-2** (3.60 g, 12.27 mmol) was dissolved in dichloromethane (30 mL), added with compound **8-1** (7.49 g, 13.50 mmol) and EEDQ (6.07 g, 24.54 mmol), and reacted at 25°C for 4 h. The reaction solution was concentrated under reduced pressure, and the concentrate was separated by a reversed-phase C18 column (70% acetonitrile/0.1% aqueous formic acid) to obtain the title compound **8-3** (7.80 g).

The structural identification data were as follows:
ESI-MS (m/z): 830.4[M+H]⁺.

### Step 2: Synthesis of 4-((2S,5S)-38-azido-5-isopropyl-2-methyl-4,7,11-trioxo-9,15,18,21,24, 27,30,33,36-nonaoxa-3,6,12-triazaoctatriaconatanamido)benzyl (4-nitrophenyl)carbonate (8-4)

Compound **8-3** (2.50 g, 3.01 mmol) was dissolved in dichloromethane (20 mL), added with bis(p-nitrophenyl)carbonate (3.66 g, 12.05 mmol) and DIPEA (1.56 g, 12.05 mmol), reacted at 25°C for 4 hours. The reaction solution was directly purified by silica gel column (ethyl acetate-dichloromethane:methanol=84:16) to obtain the title compound **8-4** (2.07 g).

The structural identification data were as follows:
ESI-MS (m/z): 995.4[M+H]⁺.

### Step 3: Synthesis of 4-((2S,5S)-38-azido-5-isopropyl-2-methyl-4,7,11-trioxo-9,15,18,21,24, 27,30,33,36-nonaoxa-3,6,12-triazaoctatriacontanamido)benzyl ((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbonate (8-5)

(S)-N-((3R,4S,5S)-1-((S)-2-((1R,2R)-3-((1S,2R)-1-hydroxy-1-phenylprop-2-yl)amino)-1-methoxy-2-methyl-3-oxypropyl)pyrrolidin-1-yl)-3-methoxy-5-methyl-1-oxohept-4-yl)-N,3-dimethyl-2-((S)-3-methyl-2-(methylamino)butylamide (100 mg, 139.28 µmol), compound **8-4** (180.17 mg, 181.06 µmol) and HOBt (56.46 mg, 417.84 µmol) were dissolved in DMF (3 mL), added with DIPEA (54.00 mg, 417.84 µmol), and reacted at 25°C for 6 hours. The reaction solution was concentrated under reduced pressure, and the concentrate was separated by a reversed-phase C18 column (55% acetonitrile/0.1% aqueous formic acid) to obtain the title compound **8-5** (60.00 mg).

The structural identification data were as follows:
ESI-MS (m/z): 1573.9[M+H]⁺.

### Step 4: Synthesis of 4-((2S,5S)-38-amino-5-isopropyl-2-methyl-4,7,11-trioxo-9,15,18,21, 24,27,30,33,36-nonaoxa-3,6,12-triazaoctatriacontanamido)benzyl ((S)-1-(((S)-1-(((3R,4S,5S)- 1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbonate (8-6)

Compound **8-5** (0.60 g, 381.22 µmol) was dissolved in THF (10 mL), added with triphenylphosphine (232.02 mg, 762.44 µmol), then added with water (10 mL), and reacted at 50°C for 6 hours. The reaction solution was added with water and extracted with dichloromethane, dried over anhydrous sodium sulfate, filtered, and the filtrate was concentrated under reduced pressure, and purified by a silica gel column (dichloromethane:methanol=84:16) to obtain the title compound **8-6** (50.00 mg).

The structural identification data were as follows:
ESI-MS (m/z): 1548.9[M+H]⁺.

### Step 5: Synthesis of 4-((2S,5S)-41-(3,4-bis(bromomethyl)-2,5-dioxo-2,5-dihydro-1H-pyrrol-1-yl)-5-isopropyl-2-methyl-4,7,11,40-tetraoxo-9,15,18,21,24,27,30,33,36-nonaoxa-3,6,12,39-tetraazapentadecanamido)phenyl ((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbonate (8)

Compound **I-2** (10.00 mg, 0.03 mmol) was dissolved in dichloromethane (4 mL), added with DIC (3.70 mg, 0.03 mmol) and compound **8-6** (23.37 mg, 15.00 µmol), and reacted at 25°C for 1 h, and the reaction was monitored by high-performance liquid chromatography-mass spectrometry. The reaction solution was concentrated under reduced pressure, the concentrate was directly purified by preparative high performance liquid chromatography, and the fractions were freezedried to obtain the title compound **8** (12.88 mg).
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 35 | 65 | 28 |
| 2.00 | 35 | 65 | 28 |
| 18.00 | 95 | 5 | 28 |

The structural identification data were as follows:
ESI-MS (m/z): 1871.8[M+H]⁺.

### Example 9: 2-(2-(2-(2-(2,3-Bis(bromomethyl)-5,7-dioxo-5,7-dihydro-6H-pyrrolo[3,4-b] pyrazin-6-yl)ethoxy)ethoxy)acetic acid (I-3)

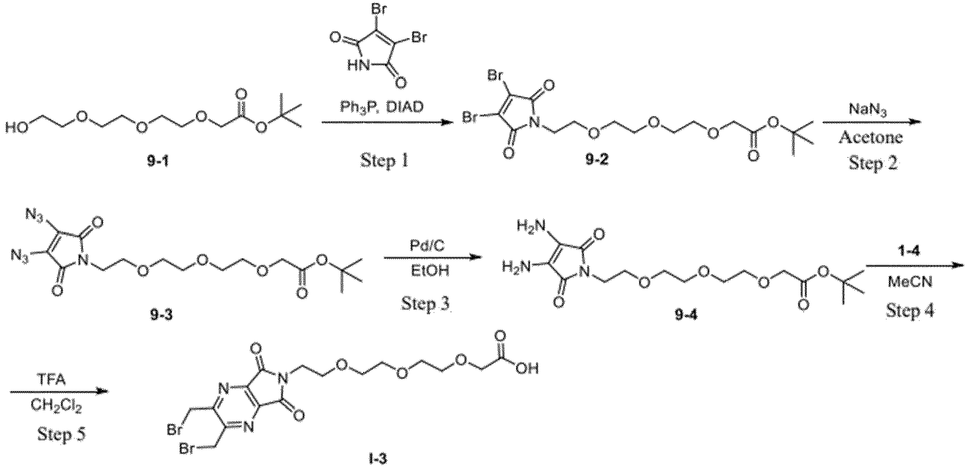

### Step 1: Synthesis of tert-butyl 2-(2-(2-(2-(3,4-dibromo-2,5-dioxo-2,5-dihydro-1H-pyrrol- 1-yl)ethoxy)ethoxy)acetate (9-2)

At 0°C, compound **9-1** (523.00 mg, 1.98 mmol) and triphenylphosphine (518.97 mg, 1.98 mmol) were dissolved in tetrahydrofuran (8 mL), added with DIAD (400.11 mg, 1.98 mmol, 389.59 µL), then stirred for 5 minutes, added with 3,4-dibromopyrrol-2,5-dione (504.30 mg, 1.98 mmol), and reacted at 0°C for 3 hours. The reaction solution was added with saturated ammonium chloride aqueous solution and extracted with ethyl acetate, dried over anhydrous sodium sulfate, filtered, and the filtrate was concentrated under reduced pressure. The concentrate was separated by silica gel column chromatography (petroleum ether: ethyl acetate = 1:1) to obtain the title compound **9-2** (0.90 g).

The structural identification data were as follows:
ESI-MS (m/z): 519.0[M+18]⁺.

### Step 2: Synthesis of tert-butyl 2-(2-(2-(2-(3,4-diazido-2,5-dioxo-2,5-dihydro-1H-pyrrol- 1-yl)ethoxy)ethoxy)acetate (9-3)

Compound **9-2** (0.20 g, 399.07 µmol) was dissolved in acetone (8 mL), added with sodium azide (51.89 mg, 798.14 µmol), and reacted at 25°C for 8 hours. The reaction solution was added with water and extracted with ethyl acetate, dried over anhydrous sodium sulfate, filtered, and the filtrate was concentrated under reduced pressure to obtain a crude product of the title compound **9-3** (160.00 mg).

The structural identification data were as follows:
ESI-MS (m/z): 443.1[M+18]⁺.

### Step 3: Synthesis of tert-butyl 2-(2-(2-(2-(3,4-diamino-2,5-dioxo-2,5-dihydro-1H-pyrrol- 1-yl)ethoxy)ethoxy)acetate (9-4)

Compound **9-3** (170.00 mg, 399.63 µmol) and 10% palladium on carbon (85.00 mg) were dissolved in ethanol (34 mL), and reacted at 25°C for 8 hours under hydrogen atmosphere. The reaction solution was filtered, and the filtrate was dried under reduced pressure to obtain a crude product of the title compound **9-4** (140.00 mg).

The structural identification data were as follows:
ESI-MS (m/z): 391.3[M+18]⁺.

### Step 4: Synthesis of 2-(2-(2-(2-(2,3-bis(bromomethyl)-5,7-dioxo-5,7-dihydro-6H-pyrrolo[3,4-b]pyrazin-6-yl)ethoxy)ethoxy)acetic acid (I-3)

Compound **9-4** (70.00 mg, 187.47 µmol) and compound **1-4** (45.72 mg, 187.47 µmol) were dissolved in acetonitrile (5 mL), and reacted at 25°C for 2 hours. The reaction solvent was dried under reduced pressure, and the concentrate was separated by silica gel column chromatography (petroleum ether: ethyl acetate = 1:1) to obtain the title compound **I-3** (26.00 mg).

The structural identification data were as follows:
ESI-MS (m/z): 391.3[M+18]⁺.

### Example 10: 4-((2S,5S)-17-(2,3-Bis(bromomethyl)-5,7-dioxo-5,7-dihydro-6H-pyrrolo[3,4-b] pyrazin-6-yl)-5-isopropyl-4,7-dioxo-2-(3-ureidopropyl)-9,12,15-trioxa-3,6-diazaheptadecanamido)benzyl ((S)-1-(((S)-1-(((3R,4S,SS)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbonate (drug-linker 10)

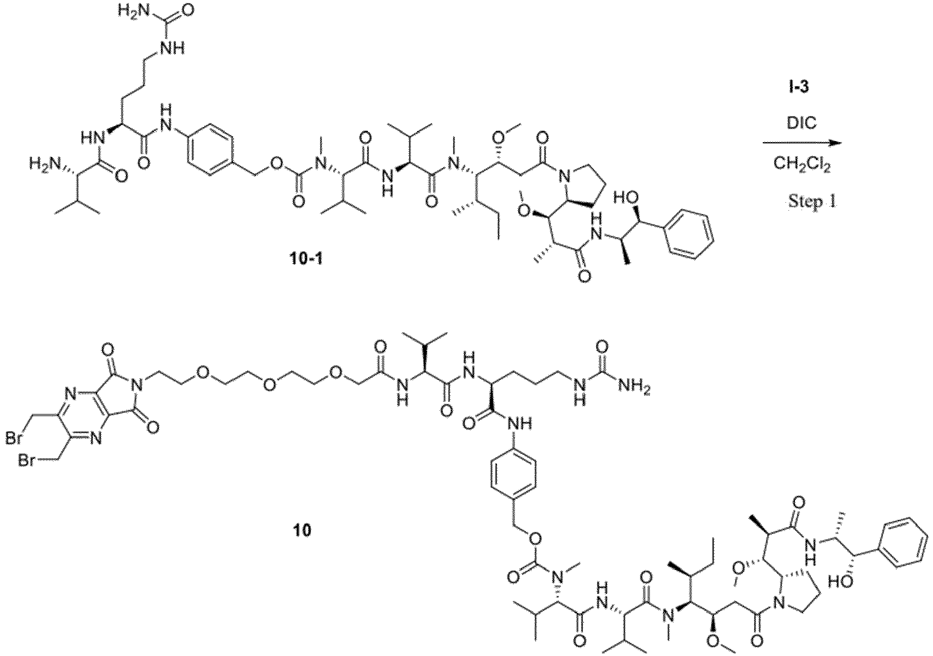

Compound **I-3** (13.47 mg, 25.65 µmol) was dissolved in dichloromethane (4 mL), added with DIC (2.43 mg, 19.24 µmol, 2.98 µL), stirred for 20 min, then added with the formate of compound **10-1** (15.00 mg, 12.83 µmol), and reacted at 20°C for 2 hours. The solvent was removed under reduced pressure, the concentrate was directly purified by preparative high-performance liquid chromatography, and the fractions were freeze-dried to obtain the title compound **10** (9.00 mg).
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 35 | 65 | 28 |
| 2.00 | 35 | 65 | 28 |
| 16.00 | 90 | 10 | 28 |

The structural identification data were as follows:
ESI-MS (m/z): 1630.4[M+H]⁺.

### Example 11: 3,5-Bis(2-(methylsulfonyl)pyrimidin-4-yl)benzoic acid (11-19)

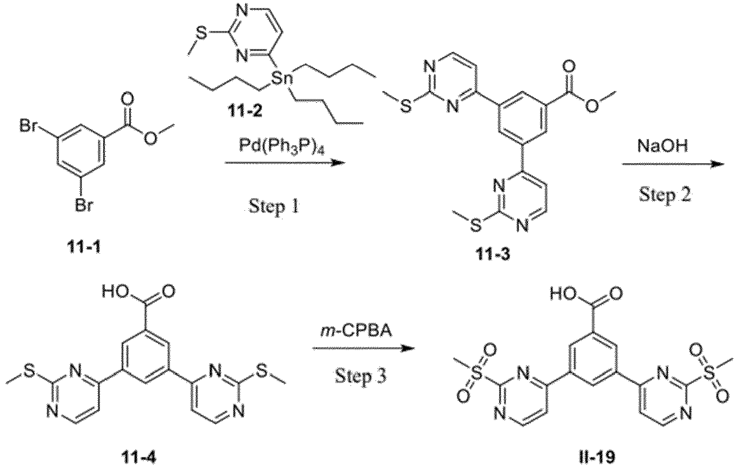

### Step 1: Synthesis of methyl 3,5-bis(2-(methylthio)pyrimidin-4-yl)benzoate (11-3)

Methyl 3,5-dibromobenzoate (1.00 g, 3.40 mmol), 4-tributylstannyl-2-thiomethylpyrimidine (3.11 g, 7.48 mmol) and tetrakis(triphenylphosphine)palladium (393.00 mg, 0.034 mmol) were dissolved in 1,4-dioxane (10 mL), subjected to replacement with nitrogen, and reacted under microwaves at 110°C for 6 hours. The reaction solution was concentrated under reduced pressure, and the concentrate was purified by silica gel column (petroleum ether: ethyl acetate = 5:1) to obtain compound **11-3** (398.00 mg).

The structural identification data were as follows:
ESI-MS (m/z): 385.0[M+H]⁺.

### Step 2: Synthesis of 3,5-bis(2-(methylthio)pyrimidin-4-yl)benzoic acid (11-4)

Compound **11-3** (398.00 mg, 1.04 mmol) was dissolved in methanol (5 mL), tetrahydrofuran (5 mL) and water (1 mL), added with sodium hydroxide (166.00 mg, 4.14 mmol), and reacted under stirring for 1 hour. The reaction solution was added dropwise with 3N aqueous hydrochloric acid solution for neutralization, concentrated under reduced pressure, then added with water and stirred, filtered, the solid was washed with water and dried in vacuum to obtain the title compound **11-4** (0.38 g).

### Step 3: Synthesis of 3,5-bis(2-(methylsulfonyl)pyrimidin-4-yl)benzoic acid (II-19)

Compound **11-4** (0.38 g, 1.03 mmol) was dissolved in methanol (20 mL), added with m-chloroperoxybenzoic acid (1.25 g, 80%, 6.15 mmol) under stirring, heated to 60°C and reacted for 4 hours. The solvent was blow-dried with nitrogen, and the solid was dissolved in dichloromethane and directly purified on a silica gel column (dichloromethane:methanol=10:1) to obtain the title compound **II-19** (0.25 g).

The structural identification data were as follows:
ESI-MS (m/z): 452.0[M+18]⁺.

### Example 12: 4-((31S,34S)-1-(3,5-Bis(2-(methylsulfonyl)pyrimidin-4-yl)phenyl)-31-isopropyl-1,29,32-trioxo-34-(3-ureidopropyl))-5,8,11,14,17,20,23,26-octaoxa-2,30,33-triazatetrapentacontan-35-amido)benzyl ((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3- methyl-1-oxobutan-2-yl)(methyl)carbonate (drug-linker 12)

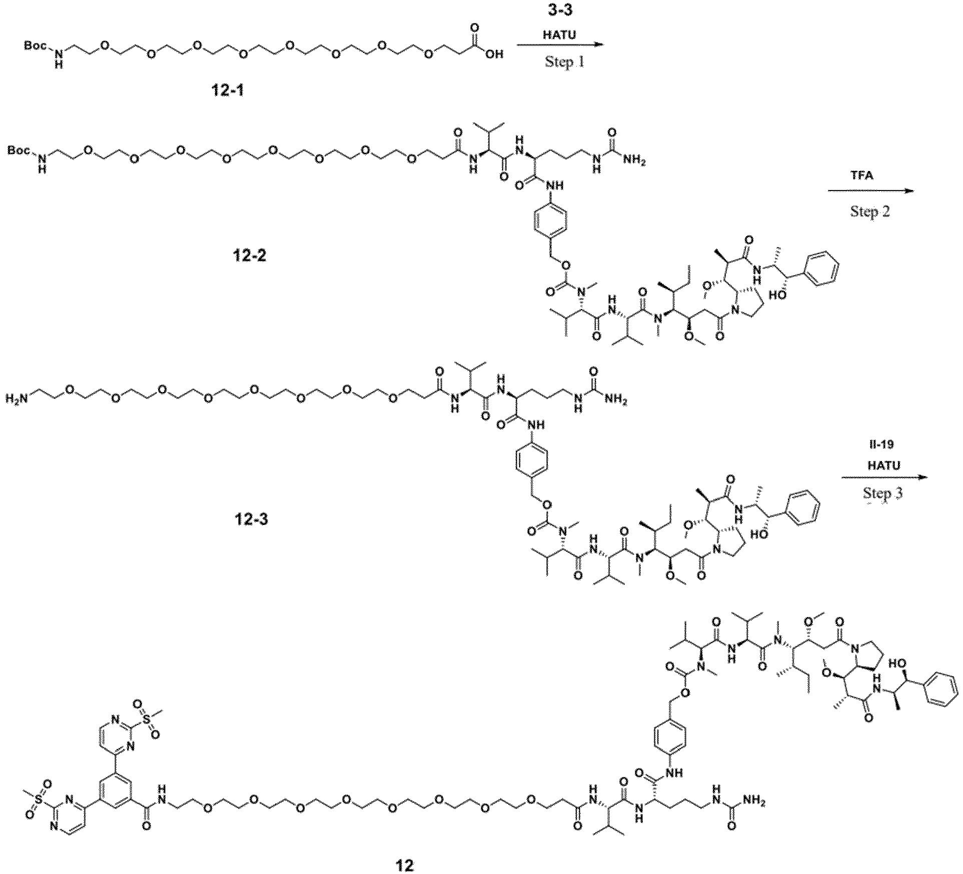

### Step 1: Synthesis of 4-((34S,37S)-34-isopropyl-2,2-dimethyl-4,32,35-trioxo-37-(3-ureidopropyl)-3,8,11,14,17,20,23,26,29-nonaoxa-5,33,36-triazaoctadecan-38-amido)benzyl ((S)-1-(((S)-1-(((3R,4S,5S)-1-((S)-)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl) (methyl)amino)-3-methyl-1-oxobutan-2-yl)amino)-3-methyl-1-oxobutan-2-yl)(methyl)carbamate (12-2)

The formate salt of compound **3-3** (200.00 mg, 171.02 µmol), HATU (91.04 mg, 239.43 µmol) and compound **12-1** (120.42 mg, 222.33 µmol) were dissolved in DMF (6 mL), and then added with DIPEA (66.31 mg, 513.06 µmol); after the addition, the reaction was carried out at room temperature for 0.5 hours. The solvent was dried under reduced pressure, the concentrate was directly purified by preparative high performance liquid chromatography, and the fractions were lyophilized to obtain the title compound **12-2** (210.00 mg).
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 30 | 70 | 28 |
| 2.00 | 30 | 70 | 28 |
| 18.00 | 90 | 10 | 28 |

The structural identification data were as follows:
ESI-MS (m/z): 824.0[(M+H)/2] +.

### Step 2: Synthesis of 4-((29S,32S)-1-amino-29-isopropyl-27,30-dioxo-32-(3-ureidopropyl)-3,6,9,12,15,18,21,24-octaoxa-28,31-diazatetratriacontan-33-amido)benzyl ((S)-1-(((S)-1-(((3R, 4S,5S)-1-((S)-2-((1R,2R)-3-(((1S,2R)-1-hydroxy-1-phenylpropan-2-yl)amino)-1-methoxy-2-methyl-3-oxypropyl)pyrrolidin-1-yl)-3-methoxy-5-methyl-1-oxobut-4-yl)(methyl)amino)-3-methyl-1-oxobutan-2-yl)amino)-3-methyl-1-oxobutan-2-yl)(methyl)carbonate (12-3)

Compound **12-2** (140.00 mg, 85.00 µmol) was dissolved in trifluoroacetic acid (0.5 mL) and dichloromethane (5 mL), and reacted at 0°C for 2 hours. The solvent was dried under reduced pressure to obtain 73.00 mg of a crude formate of the title compound **12-3.**

The structural identification data were as follows:
ESI-MS (m/z): 774.1[(M+H)/2] +.

### Step 3: Synthesis of 4-((31S,34S)-1-(3,5-bis(2-(methylsulfonyl)pyrimidin-4-yl)phenyl)-31-isopropyl-1,29,32-trioxo-34-(3-ureidopropyl))-5,8,11,14,17,20,23,26-octaoxa-2,30,33-triazatetrapentacontan-35-amido)benzyl ((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3- methyl-1-oxobutan-2-yl)(methyl)carbonate (12)

Compound **II-19** (5.45 mg, 12.56 µmol), HATU (14.32 mg, 37.67 µmol) and the formate of compound **12-3** (20.00 mg, 12.56 µmol) were dissolved in DMF (2 mL), then added with DIPEA (8.11 mg, 62.78 µmol, 11.18 µL), after the addition, the reaction was carried out at room temperature for 2 hours. The solvent was dried under reduced pressure. The concentrate was directly purified by preparative high-performance liquid chromatography, and the fractions were lyophilized to obtain the title compound **12** (6.50 mg).
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 40 | 60 | 28 |
| 2.00 | 40 | 60 | 28 |
| 18.00 | 80 | 20 | 28 |

The structural identification data were as follows:
ESI-MS (m/z): 982.5[(M+H)/2] ⁺.

### Example 13: N-((S)-10-Benzyl-1-((1S,9S)-9-ethyl-5-fluoro-9-hydroxy-4-methyl-10,13-dioxo-1,2,3,9,10,12,13,15-octahydrobenzo[de]pyrano[3',4':6,7]indolizino[1,2-b]quinolin-1-yl)amino)-1,6,9,12,15-pentaoxo-3-oxa-5,8,11,14-tetraazahexadecan-16-yl)-1-(2-(2,3-bis(bromomethyl)-5,7-dioxo-5H-pyrrolo[3,4-b]pyrazin-6(7H)-yl)acetamido)-3,6,9,12-tetraoxapentadecanamide (drug-linker 13)

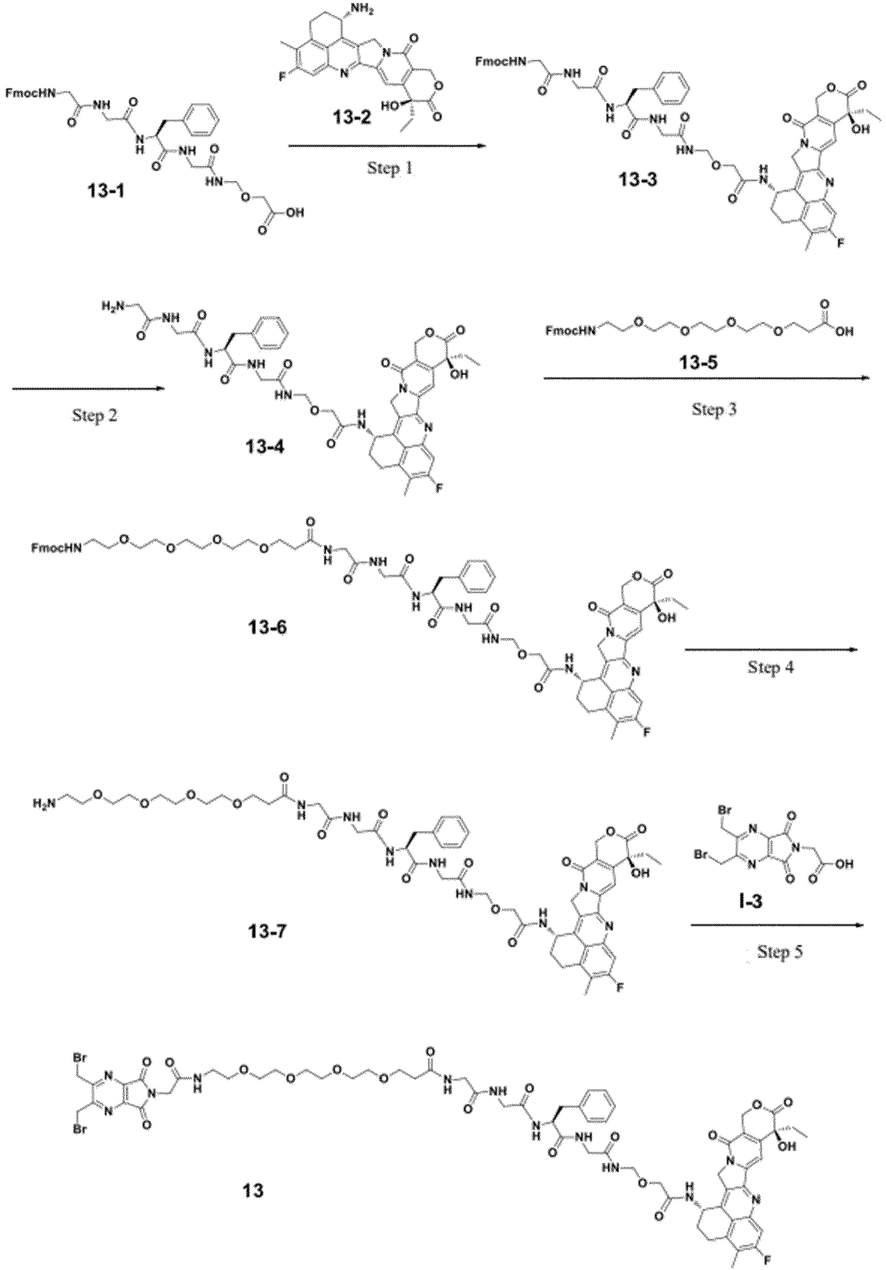

### Step 1: Synthesis of (9H-fluoren-9-yl)methyl ((S)-10-benzyl-1-((1S,9S)-9-ethyl-5-fluoro- 9-hydroxy-4-methyl-10,13-dioxo-1,2,3,9,10,12,13,15-octahydrobenzo [de]pyrano [3',4':6,7]indolizino [1,2-b]quinolin-1-yl)amino)-1,6,9,12,15-pentaoxo-3-oxa-5,8,11,14-tetraazahexadecan-16-yl)) carbamate (13-3)

Compound **13-1** (1.00 g, 1.55 mmol) was dissolved in DMF (5 mL), added sequentially with HATU (647.40 mg, 1.70 mmol), Exatecan mesylate of compound **13-2** (1.00 g, 1.55 mmol) and DIPEA (400.34 mg, 3.10 mmol), reacted at 25°C for 2 hours. The solvent was removed under reduced pressure, and the concentrate was directly prepared by high performance liquid chromatography, and freeze-dried to obtain the title compound **13-3** (1.05 g).
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 30 | 70 | 28 |
| 18.00 | 90 | 10 | 28 |

The structural identification data were as follows:
ESI-MS (m/z): 1063.4[M+H]⁺.

### Step 2: Synthesis of (S)-2-(2-aminoacetamido)acetamido)-N-(2-((2-(((1S,9S)-9-ethyl- 5-fluoro-9-hydroxy-4-methyl-10,13-dioxo-1,2,3,9,10,12,13,15-octahydrobenzo[de]pyrano[3',4':6, 7]indolizino[1,2-b]quinolin-1-yl)amino)-2-oxo-ethoxy)methyl)-2-oxo-3-phenylpropanamide (13-4)

Compound **13-3** (1.05 g, 987.69 µmol) was dissolved in dichloromethane (100 mL), added with diethylamine (20 mL), and reacted at 25°C for 1 hour. The solvent was removed under reduced pressure, the concentrate was directly purified by high performance liquid chromatography, and freeze-dried to obtain a formate of the title compound **13-4** (285.00 mg).
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 30 | 70 | 28 |
| 18.00 | 90 | 10 | 28 |

The structural identification data were as follows:
ESI-MS (m/z): 841.2[M+H]⁺.

### Step 3: Synthesis of (9H-fluoren-9-yl)methyl ((S)-10-benzyl-1-((1S,9S)-9-ethyl-5-fluoro-9-hydroxy-4-methyl-10,13-dioxo-1,2,3,9,10,12,15-octahydrobenzo [de]pyrano [3',4':6,7]indolizino [1,2-b]quinolin-1-yl)amino)-1,6,9,9,15,18-hexaoxo-3,21,24,27,30-pentaoxa-5,8,11,14,17-pentaazatricarboxylate (13-6)

The formate of compound **13-4** (83.00 mg, 98.71 µmol) and HATU (45.04 mg, 118.45 µmol), compound **13-5** (57.75 mg, 118.45 µmol) were dissolved in DMF (2 mL), then added with DIPEA (25.51 mg, 197.42 µmol, 35.14 µL), and reacted at room temperature for 0.5 hours after the addition. The solvent was removed under reduced pressure, the concentrate was directly purified by high performance liquid chromatography, and freeze-dried to obtain the title compound **13-6** (30.00 mg).
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 30 | 70 | 28 |
| 16.00 | 90 | 10 | 28 |

The structural identification data were as follows:
ESI-MS (m/z): 1310.5[M+H]⁺.

### Step 4: Synthesis of 1-amino-N-((S)-10-benzyl-1-((1S,9S)-9-ethyl-5-fluoro-9-hydroxy- 4-methyl-10,13-dioxo-1,2,3,9,10,12,13,15-octahydrobenzo[de]pyrano[3',4':6,7]indolizino[1,2-b] quinolin-1-yl)amino-1,6,9,12,15-pentaoxo-3-oxa-5,8,11,14-tetraazahexadecan-16-yl)-3,6,9,12-tetraoxa-15-decanamide (13-7)

Compound **13-6** (30.00 mg, 22.89 µmol) was dissolved in diethylamine (1 mL) and DMF (2 mL), and reacted at room temperature for 1 hour. The solvent was removed under reduced pressure, and the concentrate was directly purified by high performance liquid chromatography, and freeze-dried to obtain a formate of the title compound **13-7** (16.00 mg).
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 5 | 95 | 28 |
| 18.00 | 90 | 10 | 28 |

The structural identification data were as follows:
ESI-MS (m/z): 1088.4[M+H]⁺.

### Step 5: Synthesis of N-((S)-10-benzyl-1-((1S,9S)-9-ethyl-5-fluoro-9-hydroxy-4-methyl-10, 13-dioxo-1,2,3,9,10,12,13,15-octahydrobenzo[de]pyrano[3',4':6,7]indolizino[1,2-b]quinolin-1-yl)amino)-1,6,9,12,15-pentoxo-3-oxa-5,8,11,14-tetraazahexadecan-16-yl)-1-(2-(2,3-bis(bromomethyl)-5,7-dioxo-5H-pyrrolo[3,4-b]pyrazin-6(7H)-yl)acetamido)-3,6,9,12-tetraoxapentadecanamide (13)

The formate of compound **13-7** (16.00 mg, 14.70 µmol), compound **I-3** (11.56 mg, 29.41 µmol) and DIC (2.60 mg, 20.59 µmol, 3.19 µL) were dissolved in dichloromethane (4 mL), and reacted at 20°C for 1 hour. The solvent was removed under reduced pressure, the concentrate was directly purified by high performance liquid chromatography, and freeze-dried to obtain the title compound **13** (5.33 mg).
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 30 | 70 | 28 |
| 3.00 | 30 | 70 | 28 |
| 18.00 | 95 | 5 | 28 |

The structural identification data were as follows:
ESI-MS (m/z): 1463.3[M+H]⁺.

### Example 14: (S)-2-(17-(2,3-Bis(bromomethyl)-5,7-dioxo-5H-pyrrolo[3,4-b]pyrazin-6(7H)-yl)-4,7-dioxo-9,12,15-trioxa-3,6-diazaheptanamido)-N-(2-((2-((1S,9S)-9-ethyl-5-fluoro-9-hydroxy-4-methyl-10,13-dioxo)-1,2,3,9,10,12,13,15-octahydrobenzo[de]pyrano[3',4':6,7]indolizino[1,2-b]quinolin-1-yl)amino)-2-oxoethoxy(methyl)amino)-2-oxoethyl)-3-phenylpropanamide (drug-linker 14)

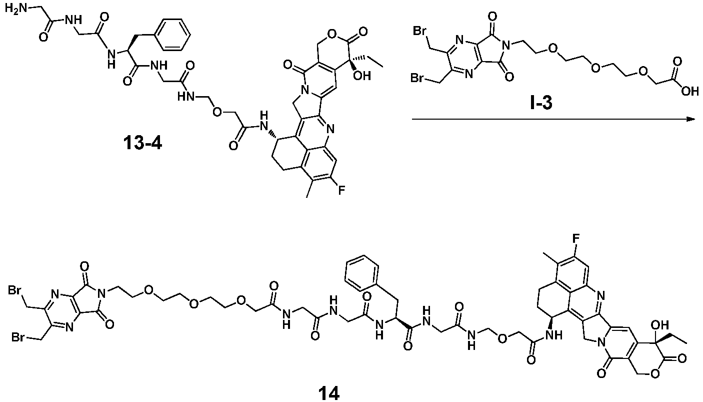

Compound **I-3** (40.26 mg, 76.67 µmol) was dissolved in dichloromethane (5 mL), added with DIC (9.68 mg, 76.67 µmol), stirred for 20 minutes, then added with the formate of compound **13-4** (34.00 mg, 38.34 µmol), and reacted at 20°C for 2 hours. The solvent was removed under reduced pressure, the concentrate was directly purified by high performance liquid chromatography, and freeze-dried to obtain the title compound **14** (5.33 mg).
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 30 | 70 | 28 |
| 2.00 | 30 | 70 | 28 |
| 18.00 | 90 | 10 | 28 |

The structural identification data were as follows:
ESI-MS (m/z): 1348.3[M+H]⁺.

### Example 15: 1-(3,5-Bis(2-(methylsulfonyl)pyrimidin-4-yl)phenyl)-1-oxo-5,8,11-trioxa- 2-azatridecan-13-oleic acid (15)

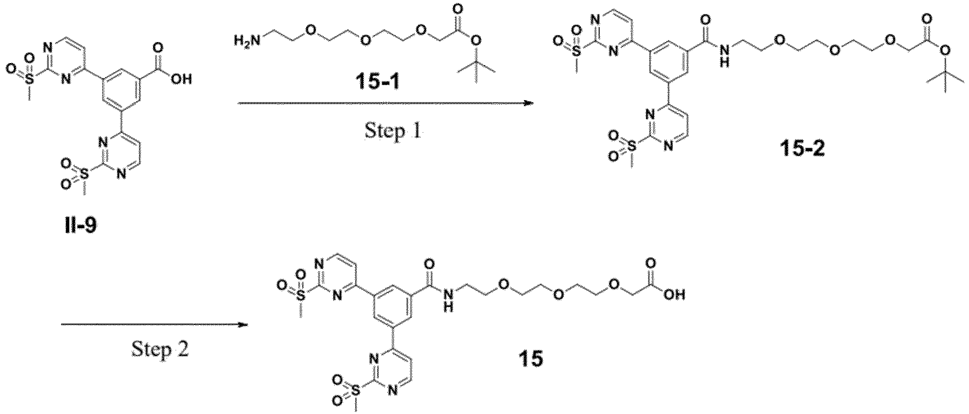

### Step 1: Synthesis of tert-butyl 1-(3,5-bis(2-(methylsulfonyl)pyrimidin-4-yl)phenyl)- 1-oxo-5,8,11-trioxa-2-azatridecan-13-oleate (15-2)

Compound **II-9** (50.00 mg, 115.09 µmol) was dissolved in DMF (2 mL), added with HATU (65.60 mg, 172.53 µmol), stirred, then added with compound **15-1** (36.37 mg, 138.11 µmol) and DIPEA (44.62 mg, 345.27 µmol, 61.46 µL), and reacted at 25°C for 2 hours. The solvent was removed under reduced pressure, and the concentrate was directly purified by high performance liquid chromatography, and freeze-dried to obtain the title compound **15-2** (25.00 mg).
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 30 | 70 | 28 |
| 2.00 | 30 | 70 | 28 |
| 18.00 | 90 | 10 | 28 |

The structural identification data were as follows:
ESI-MS (m/z): 697.2[M+18]⁺.

### Step 2: Synthesis of 1-(3,5-bis(2-(methylsulfonyl)pyrimidin-4-yl)phenyl)-1-oxo-5,8,11-trioxa-2-azatridecan-13-oleic acid (15)

Compound **15-2** (20.00 mg, 29.42 µmol) was dissolved in trifluoroacetic acid (1 mL) and dichloromethane (5 mL), and reacted at 25°C for 2 hours. The solvent was removed under reduced pressure, and a concentrate was obtained as crude product of the title compound **15** (15.00 mg), which was directly used in the next step without purification.

The structural identification data were as follows:
ESI-MS (m/z): 641.1[M+18]⁺.

### Example 16: N-((S)-10-Benzyl-1-((1R,9R)-9-ethyl-5-fluoro-9-hydroxy-4-methyl-10,13-dioxo-l,2,3,9,10,12,13,15-octahydrobenzo[de]pyrano[3',4':6,7]indolizino[1,2-b]quinolin-1-yl)amino)-1,6,9,12,15,18,46-heptaoxo-3,21,24,27,30,33,36,39,42,48,51,54-dodecaoxa-5,8,11,14,17,45 -hexaazahexapentan-56-yl)-3,5-bis(2-(methylsulfonyl)pyrimidin-4-yl)benzamide (drug-linker 16)

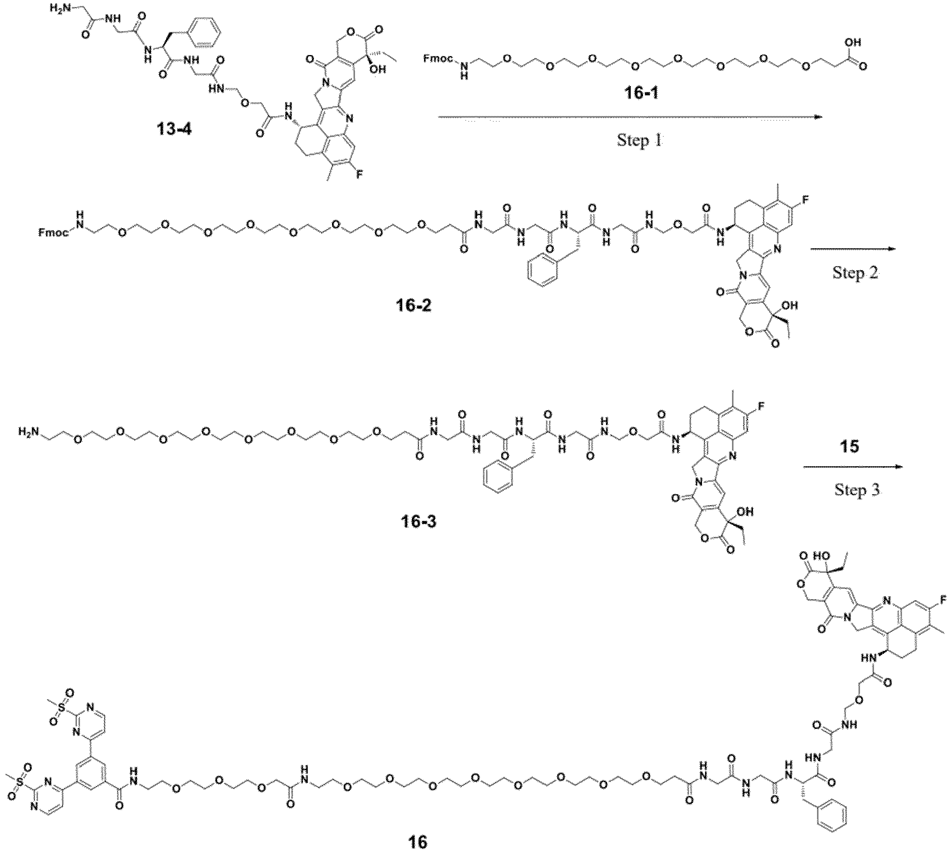

### Step 1: Synthesis of (9H-fluoren-9-yl)methyl ((S)-10-benzyl-1-((1S,9S)-9-ethyl-5-fluoro- 9-hydroxy-4-methyl-10,13-dioxo-1,2,3,9,10,12,13,15-octahydrobenzo [de]pyrano [3',4':6,7]indolizino [1,2-b]quinolin-1-yl)amino)-1,6,9,12,15,18-hexaoxo-3,21,24,27,30,33,36,39,42-nonaoxa-5, 8,11,14,17-pentaazatetradecan-44-yl)carbamate (16-2)

The mesylate of compound **13-4** (132.00 mg, 148.84 µmol) was dissolved in DMF (5 mL), added with HATU (90.55 mg, 238.14 µmol), compound **16-1** (148.19 mg, 223.26 µmol) and DIPEA (96.18 mg, 744.19 µmol, 132.48 µL), and reacted at 25°C for 2 hours. The solvent was removed under reduced pressure, the concentrate was directly purified by high performance liquid chromatography, and freeze-dried to obtain the title compound **16-2** (110.00 mg).
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 20 | 80 | 28 |
| 3.00 | 20 | 80 | 28 |
| 17.00 | 90 | 10 | 28 |

The structural identification data were as follows:
ESI-MS (m/z): 1486.5[M+1]⁺.

### Step 2: Synthesis of 1-amino-N-((S)-10-benzyl-1-((1S,9S)-9-ethyl-5-fluoro-9-hydroxy-4-methyl-10,13-dioxo-1,2,3,9,10,12,13,15-octahydrobenzo[de]pyrano[3',4':6,7]indolizino[1,2-b] quinolin-1-yl)amino-1,6,9,12,15-pentaoxo-3-oxa-5, 8,11,14-tetraazahexadecan-16-yl)-3,6,9,12,15,18,21,2-octaoxaheptan-27-amide (16-3)

Compound **16-2** (110.00 mg, 74.00 µmol, FR) was dissolved in diethylamine (1 mL) and dichloromethane (5 mL), and reacted at 25°C for 2 hours. The solvent was removed under reduced pressure, the concentrate was directly purified by high performance liquid chromatography, and freeze-dried to obtain the formate of the title compound **16-3** (40.00 mg).
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 10 | 90 | 28 |
| 2.00 | 10 | 90 | 28 |
| 18.00 | 90 | 10 | 28 |

The structural identification data were as follows:
ESI-MS (m/z): 1264.6[M+1]⁺.

### Step 3: Synthesis of N-((S)-10-benzyl-1-((1R,9R)-9-ethyl-5-fluoro-9-hydroxy-4-methyl-10, 13-dioxo-1,2,3,9,10,12,13,15-octahydrobenzo[de]pyrano[3',4':6,7]indolizino[1,2-b]quinolin-1-yl)amino)-1,6,9,12,15,18,46-heptaoxo-3,21,24,27,30,33,36,39,42,48,51,54-dodecaoxa-5, 8,11,14,17,45-hexaazahexapentan-56-yl)-3,5-bis(2-(methylsulfonyl)pyrimidin-4-yl)benzamide (16)

Compound **15** (7.00 mg, 11.22 µmol) was dissolved in DMF (3 mL), added with HATU (7.76 mg, 20.41 µmol), the formate of compound **16-3** (13.37 mg, 10.20 µmol) and DIPEA (3.96 mg, 30.61 µmol, 5.45 µL), and reacted at 25 °C for 2 hours. The solvent was removed under reduced pressure, and the concentrate was directly purified by high performance liquid chromatography, and freeze-dried to obtain the title compound **16** (7.00 mg).
Column: SunFire Prep C18 OBD 5µm 19x150mm
Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 15 | 85 | 28 |
| 2.00 | 15 | 85 | 28 |
| 18.00 | 80 | 20 | 28 |

The structural identification data were as follows:
ESI-MS (m/z): 1869.7[M+1]⁺.

### 3. Preparation of bioactive conjugate

### Conjugation Method A:

0.5 mL of antibody (anti-ROR1 antibody 19F6_Hu35V1, 3 to 20 mg/mL, referred to as 19F6 in Table 1) was diluted with 0.1 M edetate disodium solution (pH 7.60), and then adjusted with 1M Na₂HPO₄ to pH 7.60, added with 10 mM TCEP (tris(2-carboxyethyl)phosphine) solution (pH 7.60) and mixed well, and allowed to stand at room temperature for 2 h. The above solution system was added with the drug-linker (10 mM) dissolved in DMSO in an amount 5-10 times that of the antibody, mixed well, and allowed to stand at room temperature for 20 h, then NAP-5 gel column (Cytiva) was used to replace the buffer solution with 10 mM histidine buffer solution with a pH of 6.0, thereby obtaining an ADC product as shown in Table 1.

### Conjugation Method B:

0.5 mL of antibody (anti-ROR1 antibody 19F6_Hu35V1, 3 to 20 mg/mL, referred to as 19F6 in Table 1) was diluted with 0.1M edetate disodium solution (pH 7.60), and then adjusted with 1M Na₂HPO₄ solution to pH 7.60, added with 10 mM TCEP (tris(2-carboxyethyl)phosphine) solution (pH 7.60) and mixed well, and allowed to stand at room temperature for 2 h. NAP-5 gel column (Cytiva) was used to remove TCEP, and the resulting solution system was added with the drug-linker (10 mM) dissolved in DMSO in an amount 5-10 times that of the antibody, mixed well, and allowed to stand at room temperature for 20 h; then NAP-5 gel column (Cytiva) was used to replace the buffer with a 10 mM histidine buffer solution with a pH of 6.0, thereby obtaining an ADC product as shown in Table 1.

**Table 1: Preparation methods, serial numbers and DARs of ADCs**

| Substrate of the conjugation reaction | Conjugatio n method | Name of ADC | Molar ratio of drug-linker to antibody | Average DAR (n₁) | DAR4 proportio n |
|---|---|---|---|---|---|
| 19F6+drug-linker 7 | Method B | 19F6-ADC III-5-A | 6.0 | 3.87 | 56.7% |
| 19F6+drug-linker 10 | Method B | 19F6-ADC III-6-A | 7.0 | 4.42 | 53.2% |
| 19F6+drug-linker 12 | Method A | 19F6-ADC IV-1-A | 6.5 | 3.74 | 79.1% |
| 19F6+drug-linker 12 | Method A | 19F6-ADC IV-1-B | 8.0 | 3.75 | 80.0% |
| 19F6+drug-linker 12 | Method B | 19F6-ADC IV-1-C | 6.5 | 3.82 | 88.5% |
| 19F6+drug-linker 12 | Method B | 19F6-ADC IV-1-D | 8.0 | 3.87 | 91.4% |
| 19F6+drug-linker 16 | Method A | 19F6-ADC IV-7-A | 10.0 | 3.98 | 57.6% |

### Determination of drug to antibody ratio (DAR) of bioactive conjugate

SEC-MS was used to determine the molecular weight of ADC samples, and calculate the drug to antibody ratio (DAR).

### Chromatography conditions:

Chromatographic column: ACQUITY UPLC Protein BEH SEC Column, BTQR-18-016;
Sample chamber temperature: 8°C; column temperature: column temperature was not controlled; UV: 280 nm;
Mobile phase: 20mM ammonium acetate, flow rate: 0.1ml/min, 20 min, sample size: 50 ug;

### Mass-spectrometry conditions:

Mass-spectrometer model: AB Sciex Triple TOF 5600+;
GS1 55; GS2 55; CUR 30; TEM 450; ISVF 5500; DP 75; CE 5;
m/z 900-7000; Time bins to sum 100.

### (1) Molecular weight measurement of 19F6-ADC III-5-A by SEC-MS, and calculation of drug to antibody ratio

The SEC-MS molecular weight analysis of the 19F6-ADC III-5-A obtained after conjugation was shown in Table 2, and its DAR was 3.87.

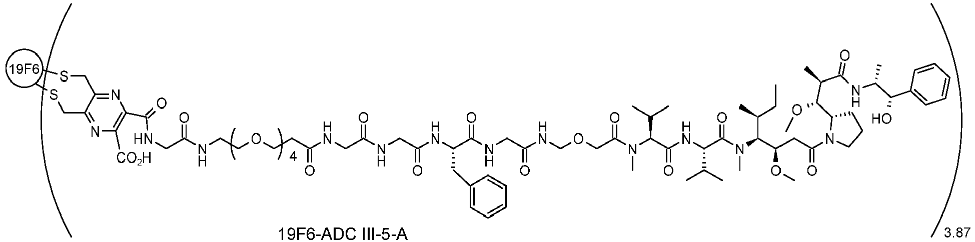

**Table 2: Measured molecular weight and DAR calculation of 19F6-ADC III-5-A**

| Load | MW_{Exp.} | Maximum signal intensity | Ratio | DAR |
|---|---|---|---|---|
| Naked antibody | 145050.00 | 0.00 | 0.000 | 3.87 |
| DAR2 | N/A | 0.00 | 0.000 | |
| DAR3 | 149863.68 | 18.50 | 0.282 | |
| DAR4 | 151472.75 | 37.25 | 0.567 | |
| DAR5 | 153078.63 | 9.92 | 0.151 | |

### (2) Molecular weight measurement of 19F6-ADC III-6-A by SEC-MS, and calculation of drug to antibody ratio

The SEC-MS molecular weight analysis of the 19F6-ADC III-6-A obtained after conjugation was shown in Table 3, and its DAR was 4.42.

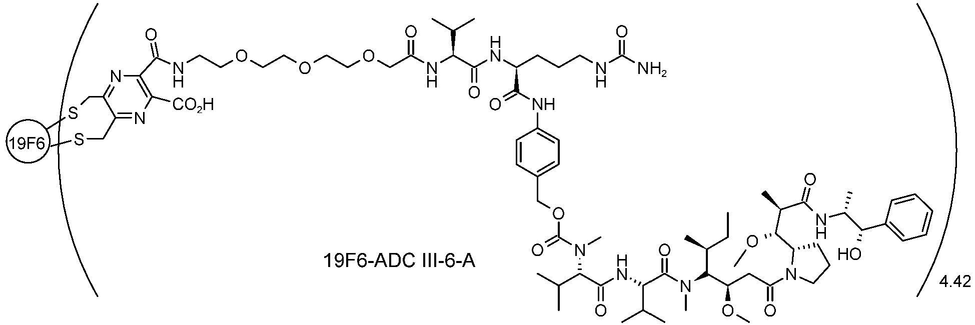

**Table 3: Measured molecular weight and DAR calculation of 19F6-ADC III-6-A**

| Load | MW_{Exp.} | Maximum signal intensity | Ratio | DAR |
|---|---|---|---|---|
| Naked antibody | 145050.00 | 0.00 | 0.000 | 4.42 |
| DAR2 | N/A | 0.00 | 0.000 | |
| DAR3 | 149510.90 | 4.85 | 0.052 | |
| DAR4 | 151001.13 | 49.64 | 0.532 | |
| DAR5 | 152483.55 | 33.24 | 0.356 | |
| DAR6 | 153972.11 | 5.54 | 0.059 | |

### (3) Molecular weight measurement of 19F6-ADC IV-1-A by SEC-MS, and calculation of drug to antibody ratio

The SEC-MS molecular weight analysis of the 19F6-ADC IV-1-A obtained after conjugation was shown in Table 4, and its DAR was 3.74.

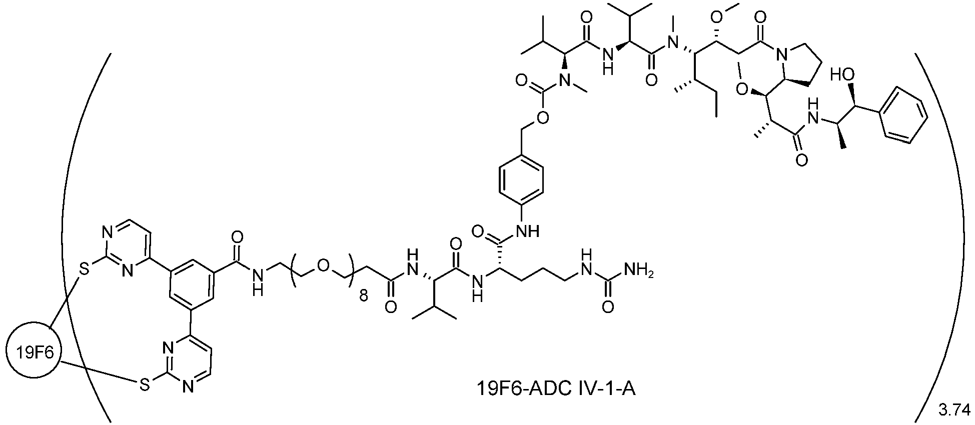

**Table 4: Measured molecular weight and DAR calculation of 19F6-ADC IV-1-A**

| Load | MW_{Exp.} | Maximum signal intensity | Ratio | DAR |
|---|---|---|---|---|
| Naked antibody | 145054.77 | 0.00 | 0.000 | 3.74 |
| DAR1 | 146863.97 | 2.09 | 0.017 | |
| DAR2 | 148669.83 | 1.75 | 0.014 | |
| DAR3 | 150468.31 | 21.98 | 0.178 | |
| DAR4 | 152274.07 | 97.91 | 0.791 | |

### (4) Molecular weight measurement of 19F6-ADC IV-1-B by SEC-MS, and calculation of drug to antibody ratio

The SEC-MS molecular weight analysis of the 19F6-ADC IV-1-B obtained after conjugation was shown in Table 5, and its DAR was 3.75.

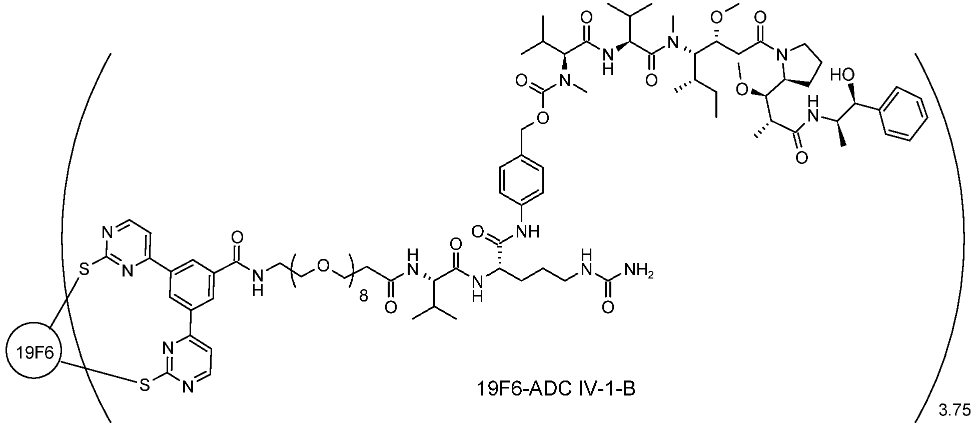

**Table 5: Measured molecular weight and DAR calculation of 19F6-ADC IV-1-B**

| Load | MW_{Exp.} | Maximum signal intensity | Ratio | DAR |
|---|---|---|---|---|
| Naked antibody | 145054.77 | 0.00 | 0.000 | 3.75 |
| DAR1 | 146861.63 | 1.96 | 0.015 | |
| DAR2 | 148662.46 | 2.54 | 0.020 | |
| DAR3 | 150467.51 | 21.19 | 0.165 | |
| DAR4 | 152273.42 | 102.47 | 0.800 | |

### (5) Molecular weight measurement of 19F6-ADC IV-1-C by SEC-MS, and calculation of drug to antibody ratio

The SEC-MS molecular weight analysis of the 19F6-ADC IV-1-C obtained after conjugation was shown in Table 6, and its DAR was 3.82.

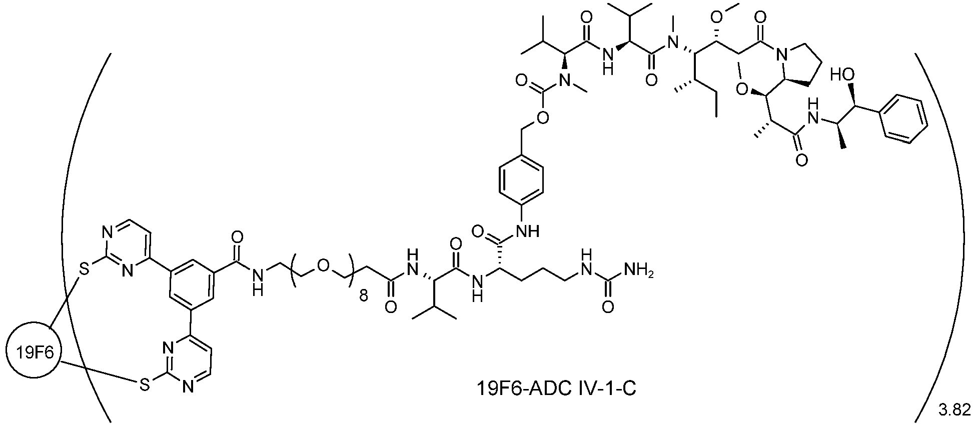

**Table 6: Measured molecular weight and DAR calculation of 19F6-ADC IV-1-C**

| Load | MW_{Exp.} | Maximum signal intensity | Ratio | DAR |
|---|---|---|---|---|
| Naked antibody | 145054.77 | 0.00 | 0.000 | 3.82 |
| DAR1 | 146862.39 | 1.68 | 0.025 | |
| DAR2 | 148667.57 | 0.96 | 0.014 | |
| DAR3 | 150470.67 | 5.12 | 0.076 | |
| DAR4 | 152273.43 | 60.04 | 0.885 | |

### (6) Molecular weight measurement of 19F6-ADC IV-1-D by SEC-MS, and calculation of drug to antibody ratio

The SEC-MS molecular weight analysis of the 19F6-ADC IV-1-D obtained after conjugation was shown in Table 7, and its DAR was 3.87.

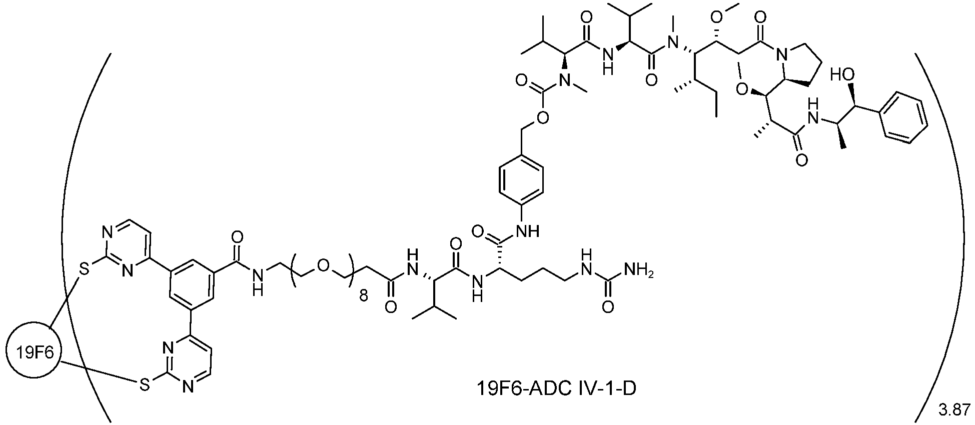

**Table 7: Measured molecular weight and DAR calculation of 19F6-ADC IV-1-D**

| Load | MW_{Exp.} | Maximum signal intensity | Ratio | DAR |
|---|---|---|---|---|
| Naked antibody | 145054.77 | 0.00 | 0.000 | 3.87 |
| DAR1 | 146855.00 | 1.79 | 0.017 | |
| DAR2 | 148660.10 | 1.00 | 0.009 | |
| DAR3 | 150470.36 | 6.44 | 0.060 | |
| DAR4 | 152273.33 | 98.45 | 0.914 | |

### (7) Molecular weight measurement of 19F6-ADC IV-7-A by SEC-MS, and calculation of drug to antibody ratio

The SEC-MS molecular weight analysis of the 19F6-ADC IV-7-A obtained after conjugation was shown in Table 8, and its DAR was 3.98.

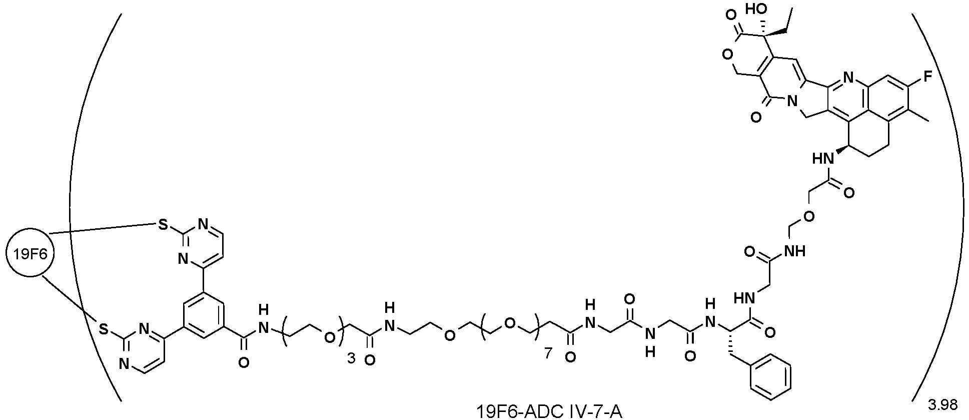

**Table 8: Measured molecular weight and DAR calculation of 19F6-ADC IV-7-A**

| Load | MW_{Exp.} | Maximum signal intensity | Ratio | DAR |
|---|---|---|---|---|
| Naked antibody | 145054.69 | 1.75 | 0.013 | 3.98 |
| DAR1 | 146760.24 | 2.02 | 0.016 | |
| DAR2 | 148460.32 | 5.75 | 0.044 | |
| DAR3 | 150178.32 | 11.66 | 0.090 | |
| DAR4 | 151894.99 | 74.75 | 0.576 | |
| DAR5 | 153762.19 | 33.88 | 0.261 | |

### 4. In vitro inhibition test of bioactive conjugate against cell viability

Inhibitory effect of ADC compounds on cell proliferation
(1) Cell plating: Firstly, tumor cells N87 were cultured in the corresponding medium, the cells were digested with trypsin, resuspended, and then counted. The cells were adjusted to an appropriate concentration and coated on a plate. The source of tumor cells was shown in Table 9.

**Table 9: Source of tumor cells**

| Cell Name | Tumor type | Source |
|---|---|---|
| NCI-N87 | Human gastric cancer cells | ATCC |

Co-incubation of the compound of the present invention and tumor cells: After the cells were attached to the wall, the medium was removed, and the diluted bioactive molecule (the compound of the present invention) was added to the wells of the above plate, and incubated for 96 hours.

In vitro cell viability detection: After the incubation, 50 µL of Cell Counting-Lite^{™} 2.0 reagent (Vazyme/Novazyme) was added to each well, shaken and mixed well in the dark, reacted for 10 minutes, and then detected by a microplate reader (manufacturer: BMG, model : PHERAStar-FS) to obtain readings. Cell Counting-Lite^{™} of medium without cells was used to obtain the background RLU, and Cell Counting-Lite^{™} of medium containing cells was used to obtain the vehicle RLU. Cell inhibition rate = 1 - (sample RLU - background RLU) / (vehicle RLU - background RLU) × 100%. According to the four-parameter model fitting curve, the half inhibitory concentration (IC₅₀) of the compound was calculated.

### (2) Data results: The detection results were shown in Table 10.

**Table 10: Killing results of ADC conjugates on NCI-N87 cell line**

| ADC No. | IC₅₀(µg/mL) |
|---|---|
| | Cell name: NCI-N87 (96 hours) |
| 19F6-ADC III-6-A | 8.48 |
| 19F6-ADC IV-1-D | 2.57 |

It was shown that the ADCs formed by the new conjugation method could kill tumor cells, and the new conjugation method could be effectively applied to ADC molecules.

Various modifications of the present invention, in addition to those described herein, will be apparent to those skilled in the art from the foregoing description. Such modifications are also intended to fall within the scope of the appended claims. Each reference cited in the present application, including all patents, patent applications, journal articles, books, and any other publications, is hereby incorporated by reference in its entirety.

## Claims

1. A compound or a pharmaceutically acceptable salt, ester, stereoisomer, tautomer, polymorph, solvate, N-oxide or isotopically labeled compound thereof, wherein the compound has the structure of Formula I: 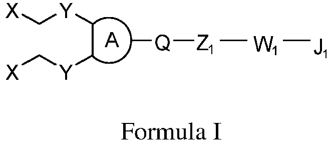 wherein:
X is a leaving group such as Cl, Br, I, OMs, OTs, OTf or 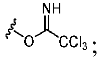
Y is absent or a carbonyl;
ring A is selected from the group consisting of substituted or unsubstituted C₆₋₁₀ aromatic ring, 5- to 12-membered heteroaromatic ring or 5- to 12-membered heterocyclic ring;
Q is absent or -C(O)-NH-;
Z₁ is absent or selected from the group consisting of -CH₂- or C₂₋₆ alkynylene;
Wi is absent or one or more selected from the group consisting of C₁₋₁₀ alkylene, - (CH₂CH₂O)ₚ-, and -(OCH₂CH₂)ₚ-;
J₁ is selected from the group consisting of -COOH, -NH₂, 3- to 10-membered nitrogen-containing heterocyclic group, sulfonylurea group or hydroxyl;
p is an integer of 1 to 10.

2. The compound of Formula I according to claim 1, which has a structure selected from the followings: 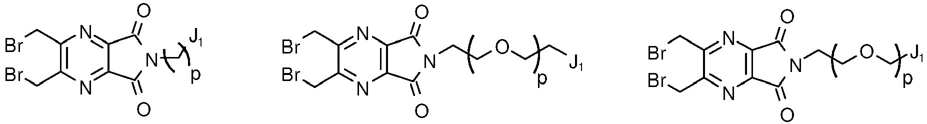 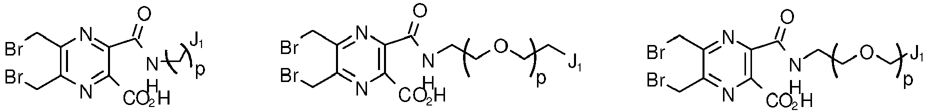 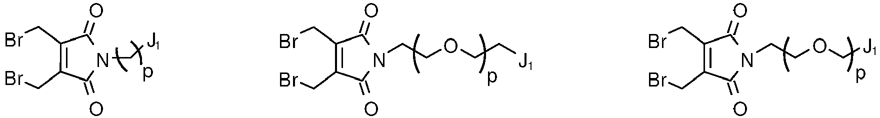
wherein p is an integer from 1 to 10, and J₁ is -COOH or -NH₂;
preferably, the compound of Formula I has a structure selected from:

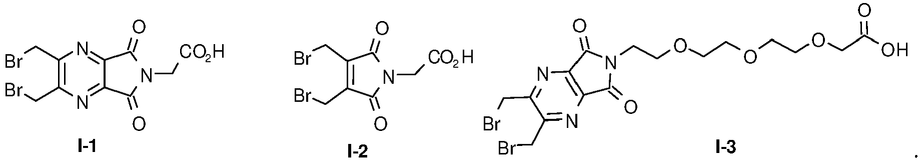

3. A compound or a pharmaceutically acceptable salt, ester, stereoisomer, tautomer, polymorph, solvate, N-oxide or isotopically labeled compound thereof, wherein the compound has the structure of Formula II: 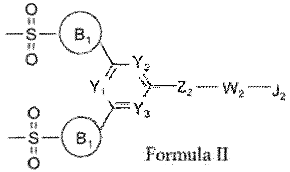
wherein, B₁ in each occurrence is independently selected from the group consisting of single bond or 5- to 12-membered nitrogen-containing heteroaromatic ring;
Y₁, Y₂ and Y₃ in each occurrence are each independently selected from the group consisting of CH and N;
Z₂ is absent or selected from the group consisting of -NH-, -CH₂-, carbonyl, -C(=O)NH-, - NHC(=O)- or C₂₋₆ alkynylene;
W₂ is absent or one or more selected from the group consisting of C₁₋₁₀ alkylene, - (CH₂CH₂O)ₚ- or -(OCH₂CH₂)ₚ-;
J₂ is selected from the group consisting of -COOH, -NH₂, 3- to 10-membered nitrogen-containing heterocyclic group, sulfonylurea group or hydroxyl;
p is an integer of 1 to 10.

4. The compound of Formula II according to claim 3, which has a structure selected from the followings: 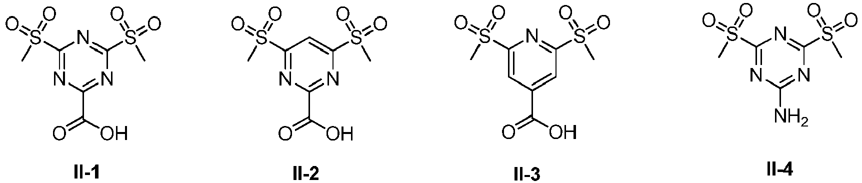 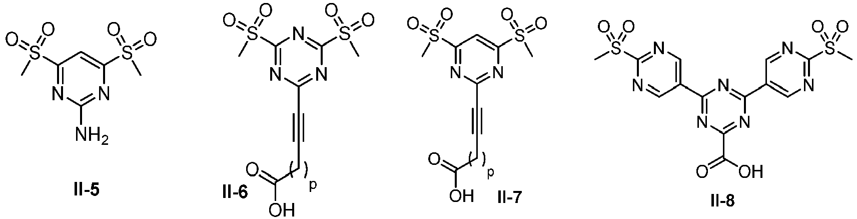 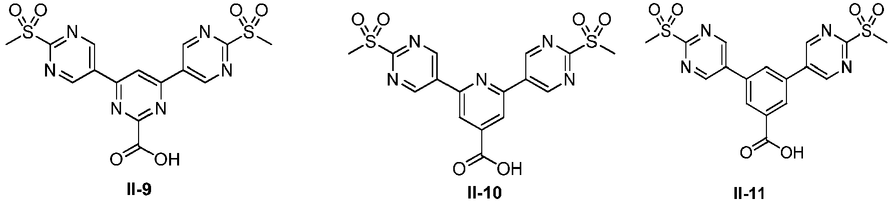 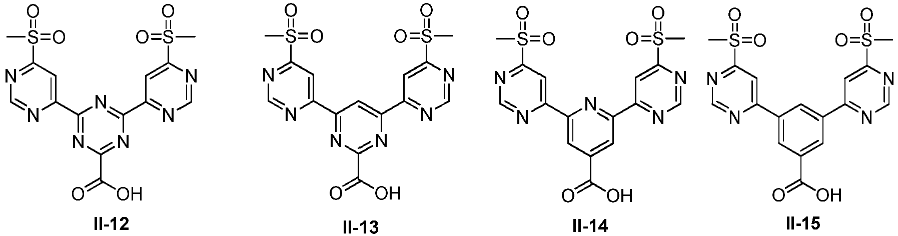 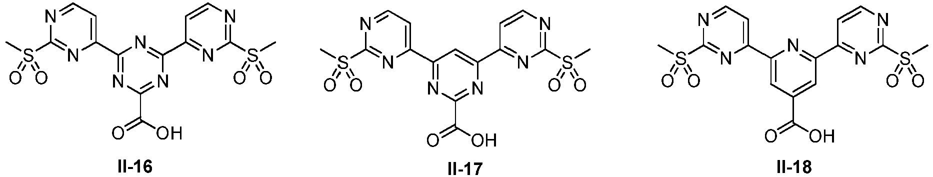 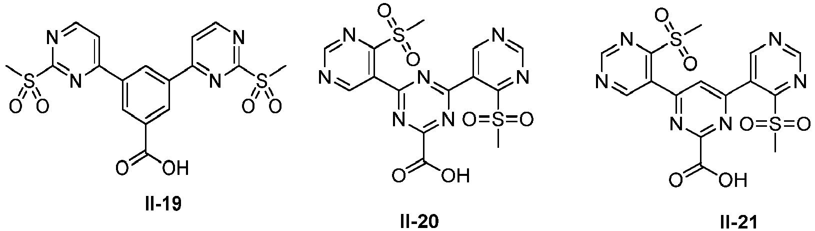 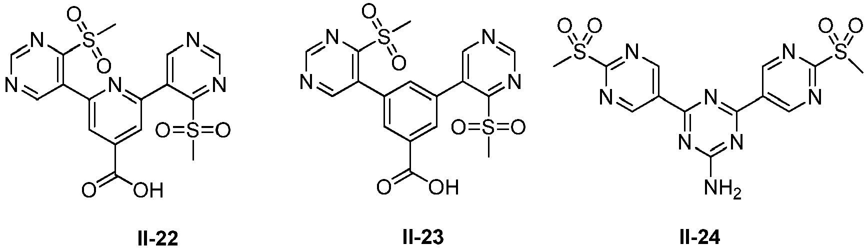 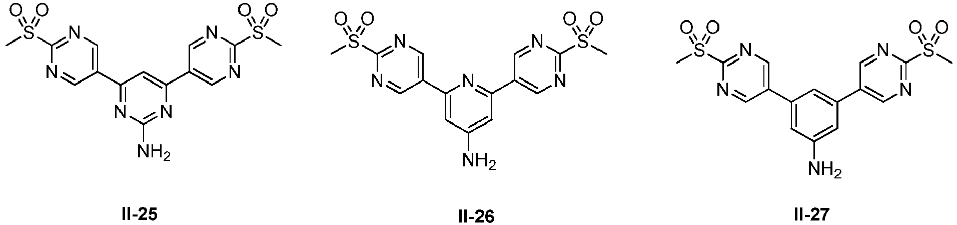 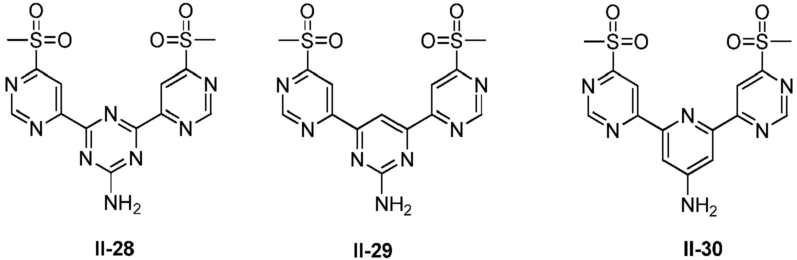 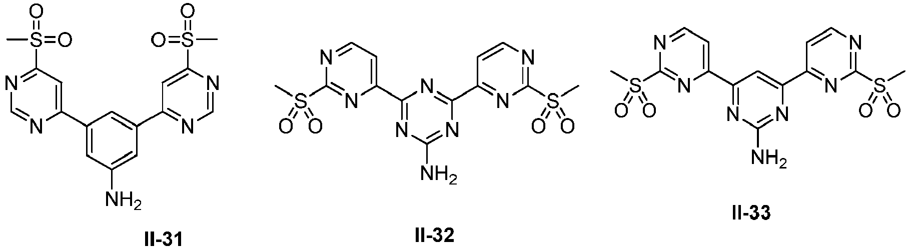 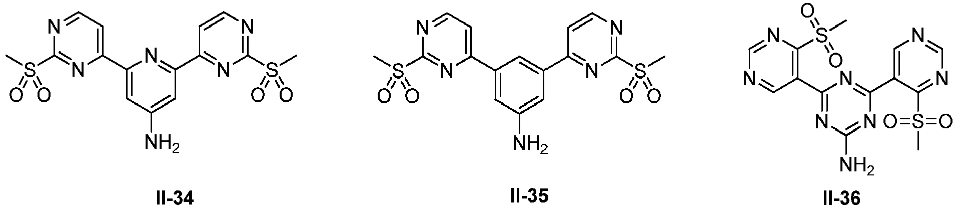 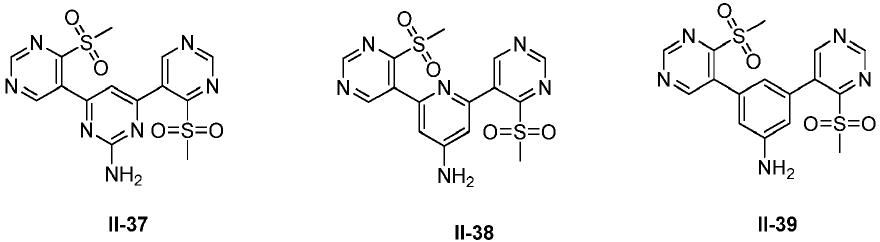 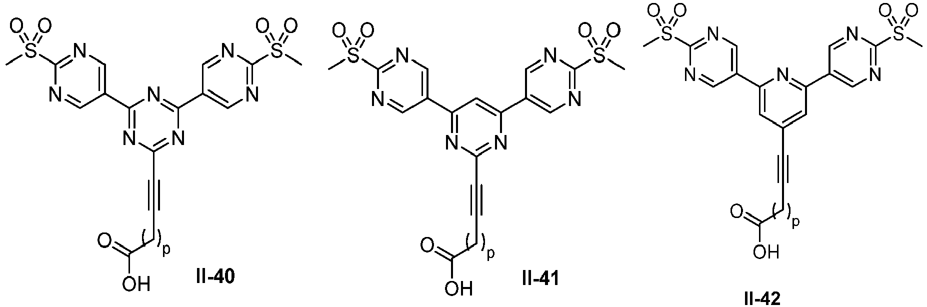 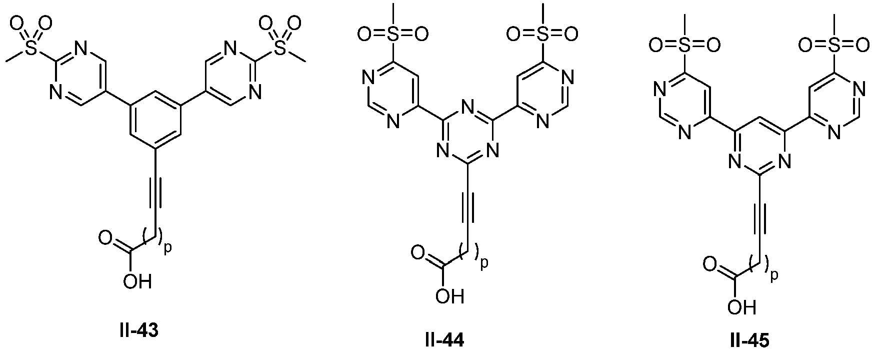 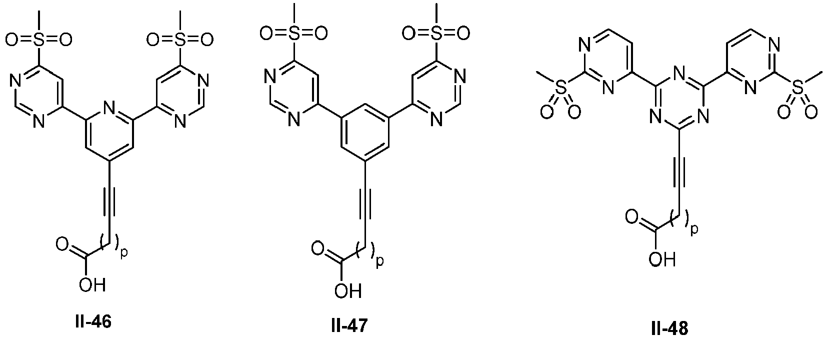 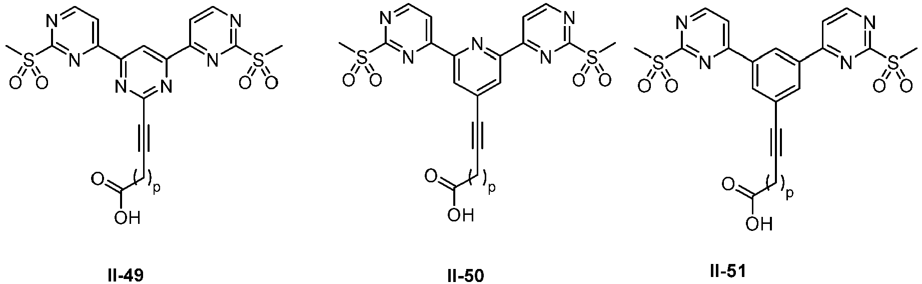 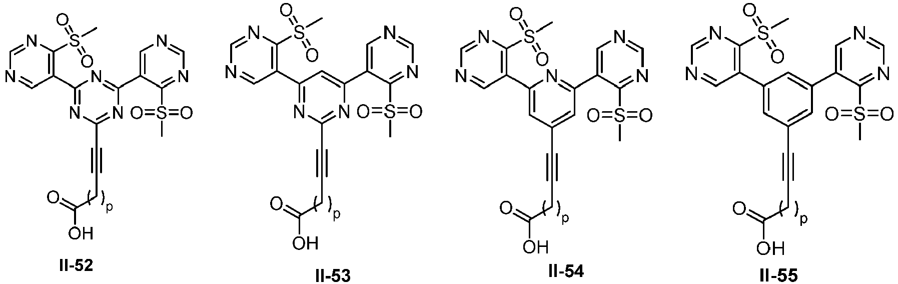 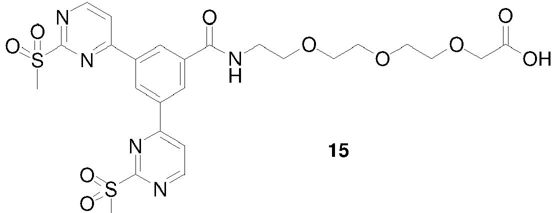
p is an integer from 1 to 10;
preferably, the compound of Formula II has the following structure:

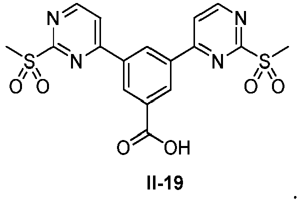

5. A compound or a pharmaceutically acceptable salt, ester, stereoisomer, tautomer, polymorph, solvate, N-oxide or isotopically labeled compound thereof, wherein the compound has the structure of Formula V: 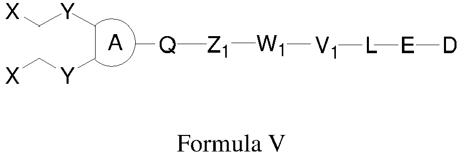 wherein:
E is selected from the group consisting of single bond, -NH-CH₂-, and the following structures: 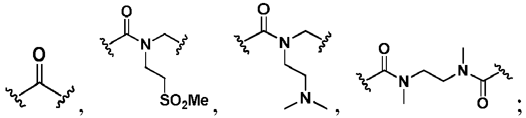
D is a fragment of a bioactive molecule (e.g., a cytotoxic drug);
X, Y, A, Q, Z₁, Wi are as defined in claim 1 or 2;
V₁ is a group formed from J₁ of the compound of Formula I according to claim 1 or 2 when it is connected to L; preferably, V₁ is selected from the group consisting of -CO-, -N(Ri)-, -O-, 3-to 10-membered nitrogen-containing heterocyclic group or sulfonylurea group, wherein R₁ is H, C₁₋₆ alkyl or C₂₋₆ alkoxyalkyl; further preferably, V₁ is selected from the group consisting of - C(O)- and -N(Ri)-, wherein R₁ is H, C₁₋₆ alkyl or C₂₋₆ alkoxyalkyl;
L is a linker connecting V₁ and E.

6. The compound of Formula V according to claim 5, which has the structure selected from the followings:

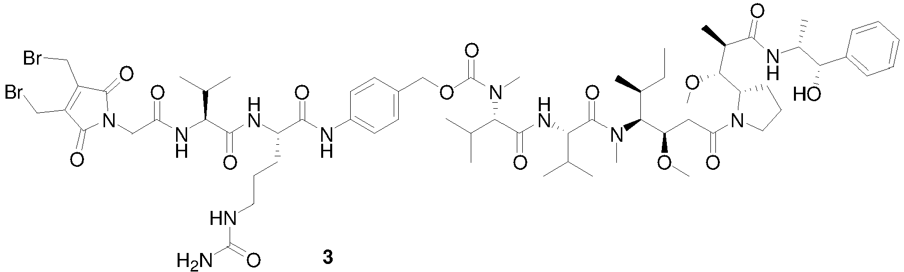

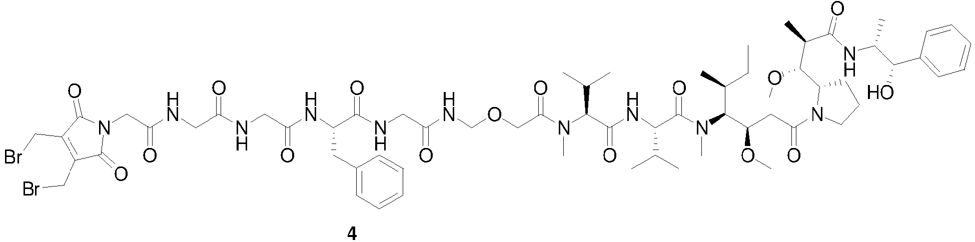

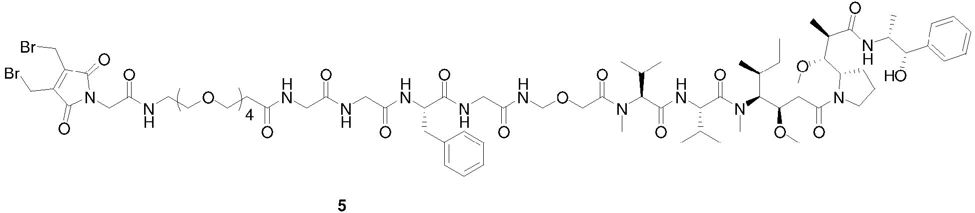

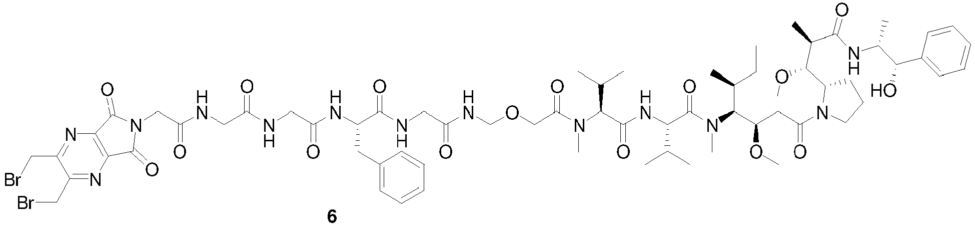

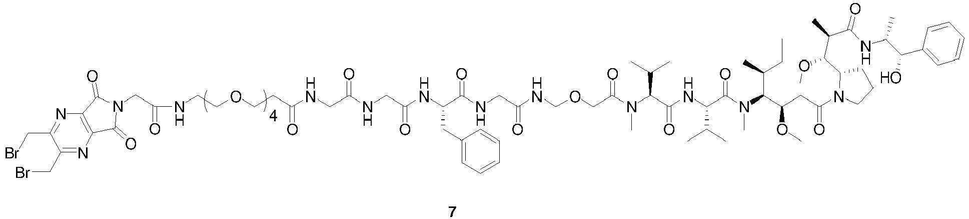

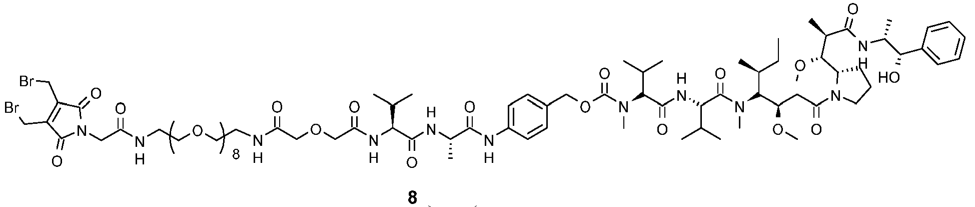

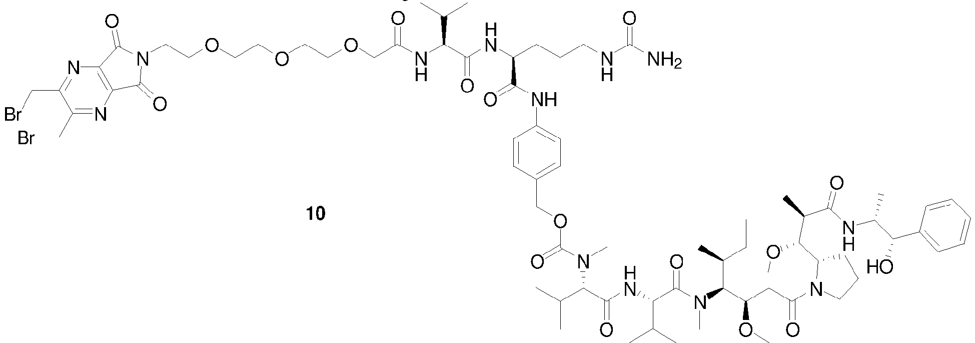

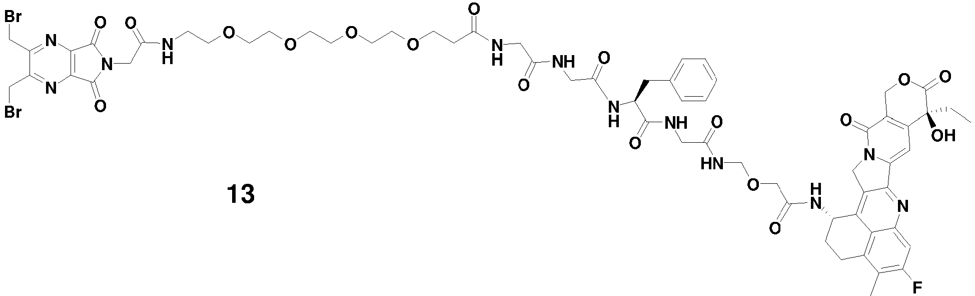

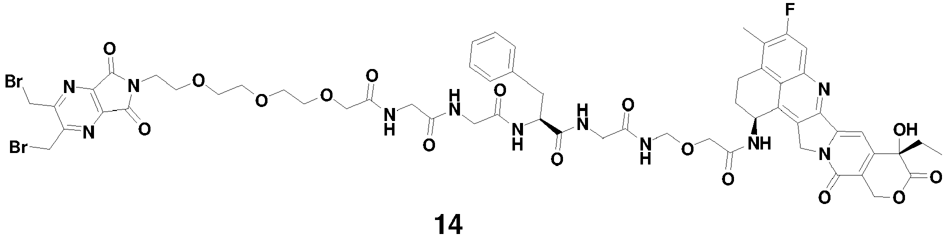

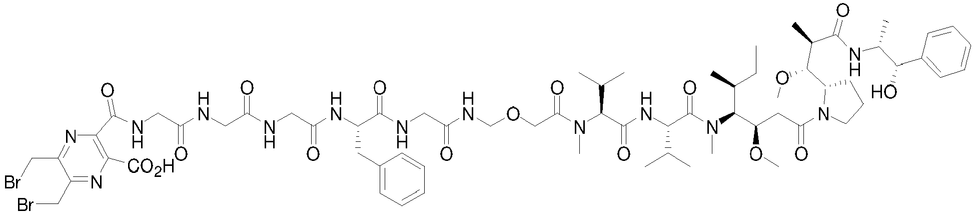

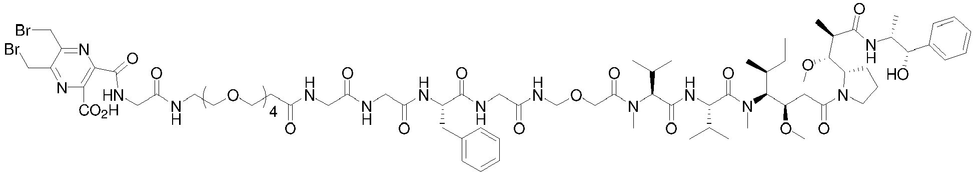

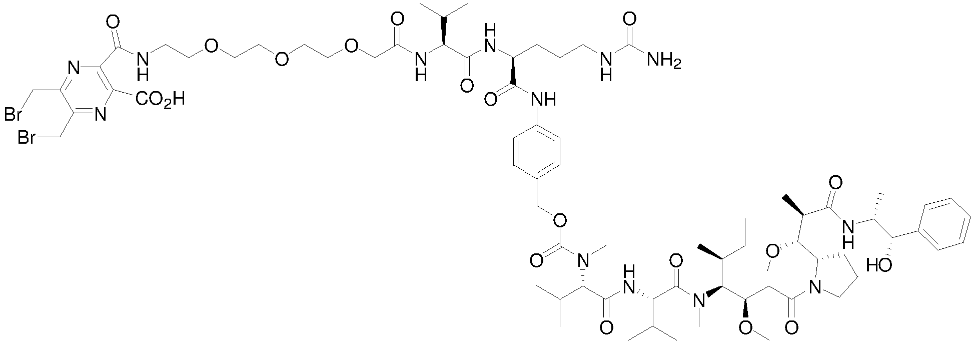

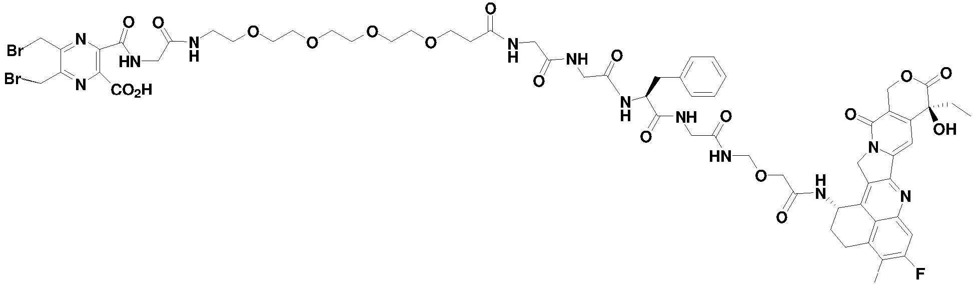

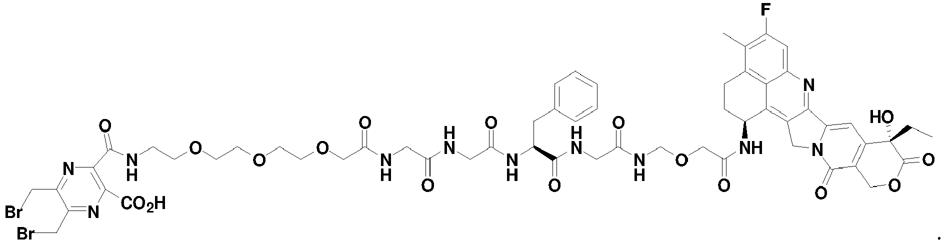

7. A compound or a pharmaceutically acceptable salt, ester, stereoisomer, tautomer, polymorph, solvate, *N*-oxide or isotopically labeled compound thereof, wherein the compound has the structure of Formula VI: 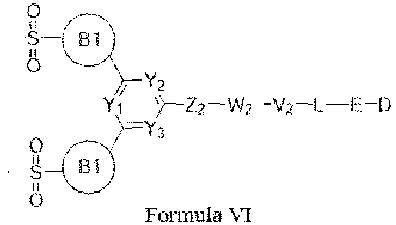
B₁, Y₁, Y₂, Y₃, Z₂ and W₂ are as defined in claim 3 or 4;
L is a linker connecting V₂ and E;
V₂ is a group formed from J₂ as defined in claim 3 or 4 when it is connected to L; preferably, V₂ is selected from the group consisting of -CO-, -N(R₂)-, -O-, 3- to 10-membered nitrogen-containing heterocyclic group or sulfonylurea group, wherein R₂ is H, C₁₋₆ alkyl or C₂₋₆ alkoxyalkyl; further preferably, V₂ is selected from the group consisting of -C(O)- and -N(R₂)-, wherein R₂ is H, C₁₋₆ alkyl or C₂₋₆ alkoxyalkyl;
E is selected from the group consisting of single bond, -NH-CH₂-, and the following structures: 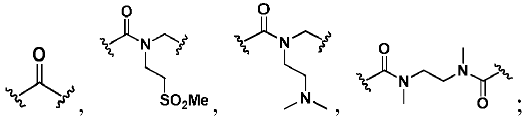
D is a fragment of a bioactive molecule (e.g., a cytotoxic drug).

8. The compound according to claim 7, which has a structure selected from:

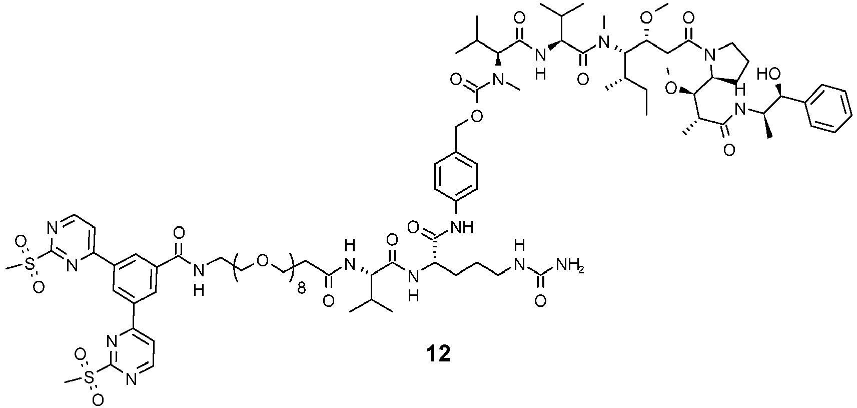

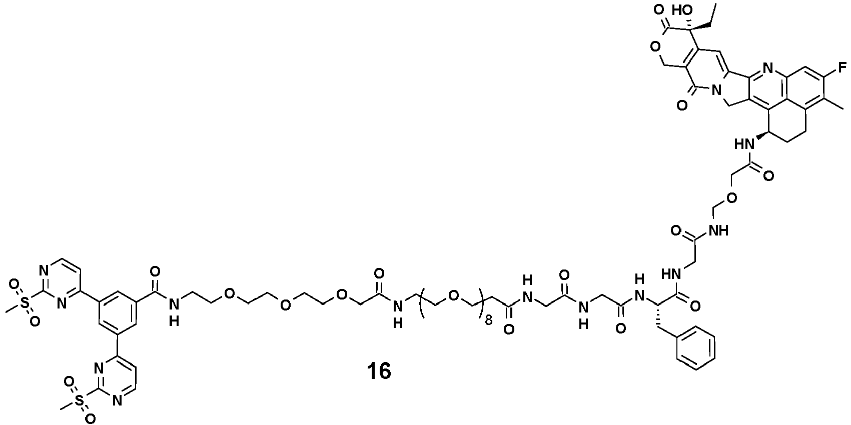

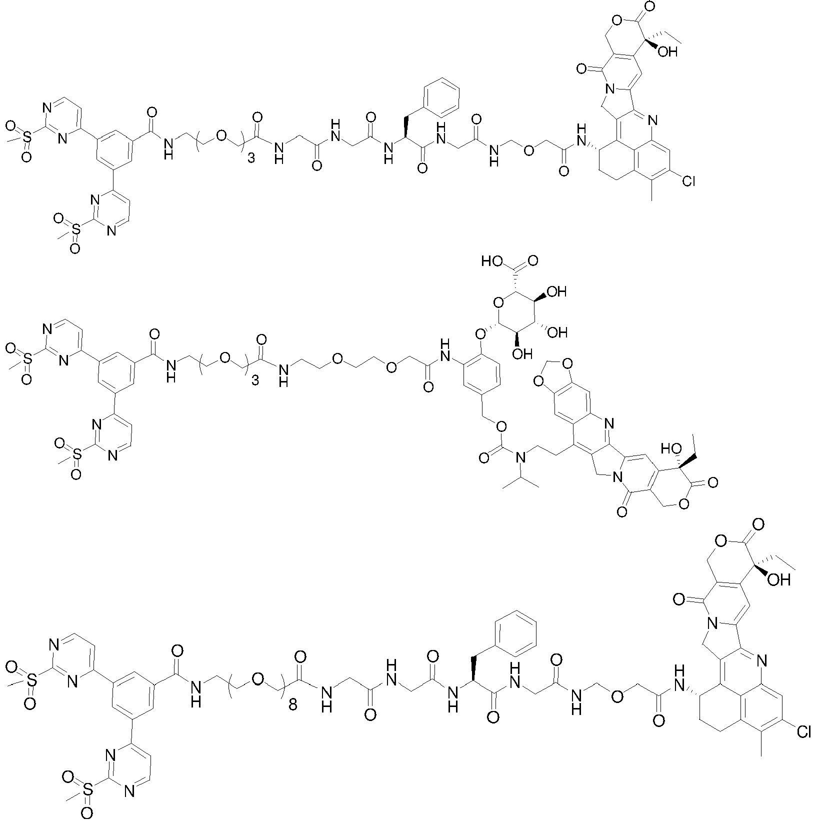

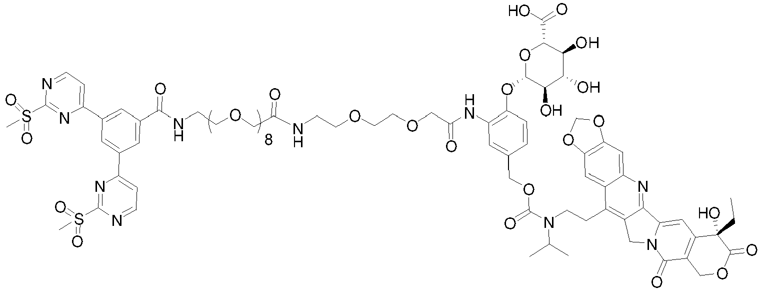

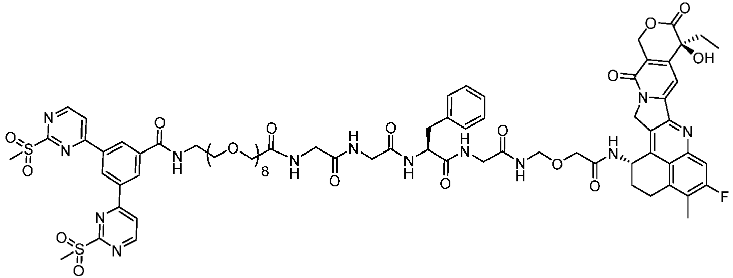

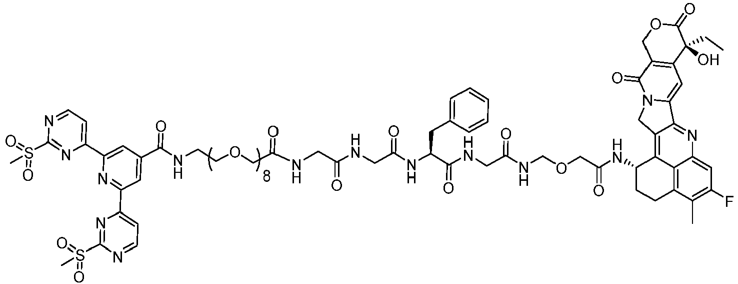

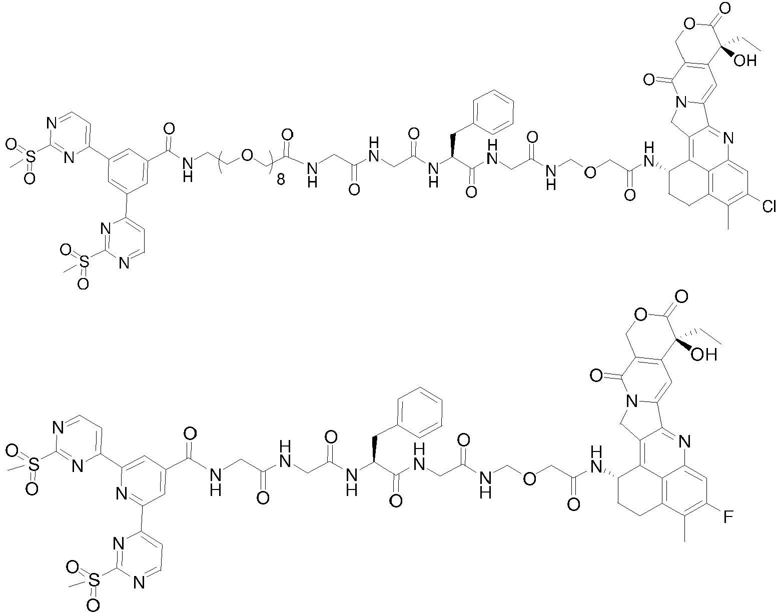

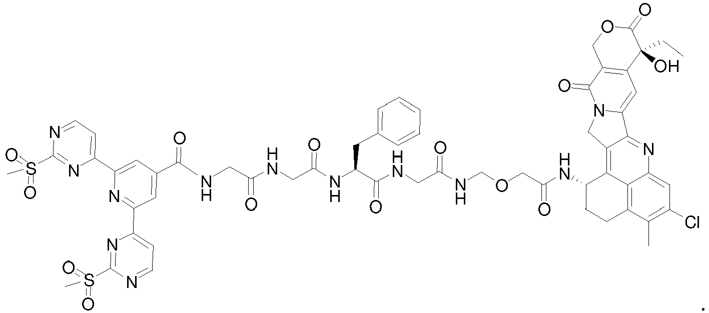

9. A bioactive conjugate, which has a structure as shown in Formula VII: 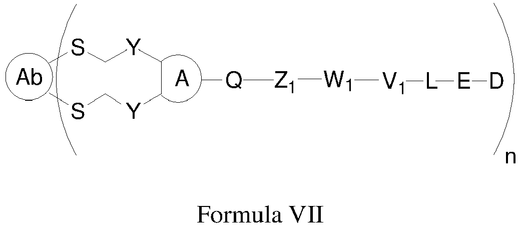
wherein, Ab is a targeting moiety (e.g., small molecule ligand, protein (e.g., antibody), polypeptide, non-protein reagent (e.g., saccharide, RNA or DNA)); n is an integer or decimal of 1 to 10;
V₁ is selected from the group consisting of -C(O)- and -N(Ri)-, wherein R₁ is H, C₁₋₆ alkyl or C₂₋₆ alkoxyalkyl;
L is a linker connecting V₁ and E;
E is a structural fragment connecting L and D;
D is a fragment of a bioactive molecule (e.g., a cytotoxic drug);
when the targeting moiety is an antibody, 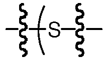 in the conjugate represents a specific way that a sulfhydryl group in the antibody connects to the rest of the conjugate;
all the other groups are as defined in any one of claims 1 to 2.

10. A bioactive conjugate, which has a structure as shown in Formula VIII: 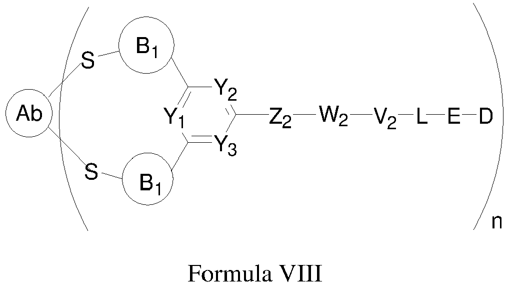
wherein, Ab is a targeting moiety (e.g., small molecule ligand, protein (e.g., antibody), polypeptide, non-protein reagent (e.g., saccharide, RNA or DNA)); n is an integer or decimal of 1 to 10;
V₂ is selected from the group consisting of -C(O)- and -N(R₂)-, wherein R₂ is H, C₁₋₆ alkyl or C₂₋₆ alkoxyalkyl;
L is a linker connecting V₂ and E;
E is a structural fragment connecting L and D;
D is a fragment of a bioactive molecule (e.g., a cytotoxic drug);
when the targeting moiety is an antibody, 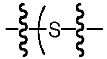 in the conjugate represents a specific way that a sulfhydryl group in the antibody connects to the rest of the conjugate;
all the other groups are as defined in any one of claims 3 to 4.

11. The bioactive conjugate according to claim 9, which has a structure as follows, wherein Ab is selected from the group consisting of the anti-Her2 antibody trastuzumab or the anti-Trop2 antibody sacituzumab or the anti-ROR1 antibody 19F6_Hu35V1 (19F6 for short), n₁ is 1 to 8, preferably 3 to 5:

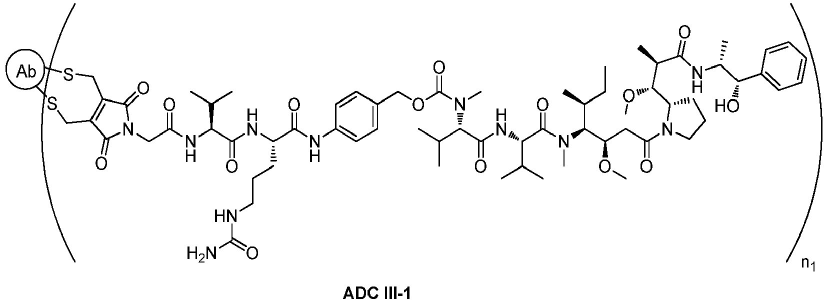

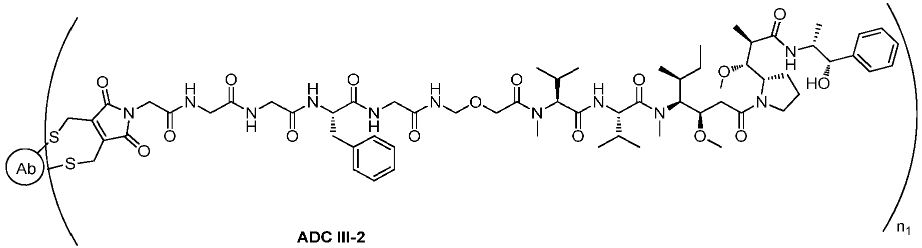

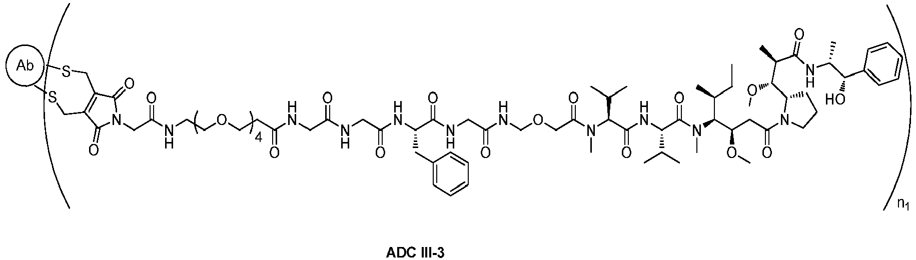

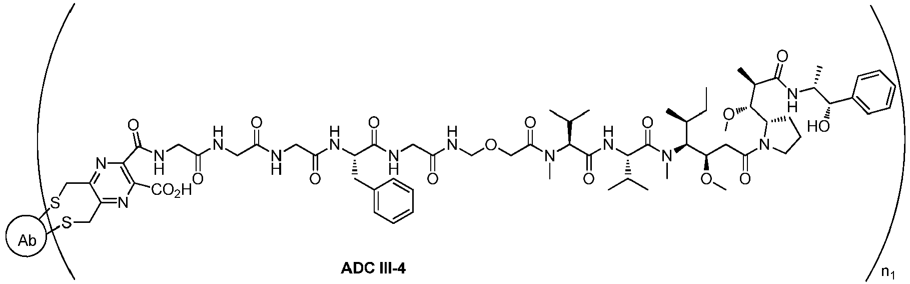

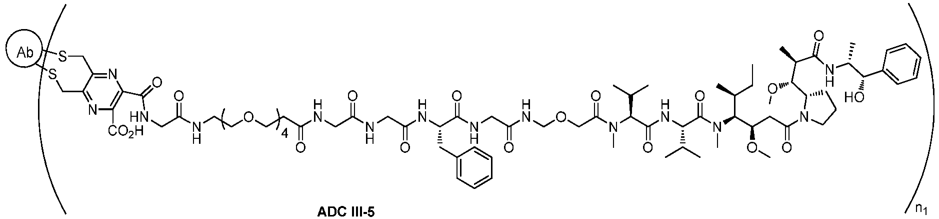

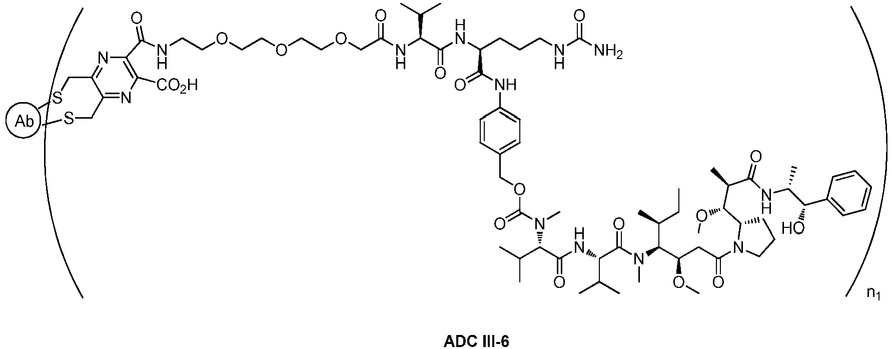

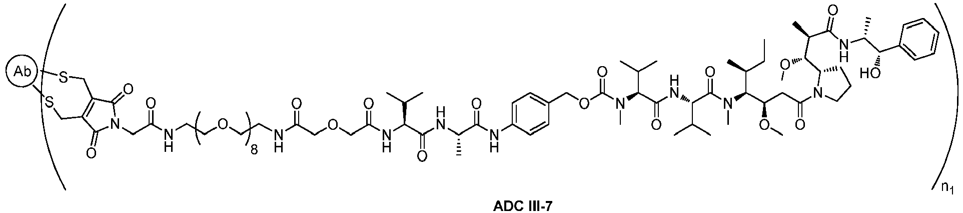

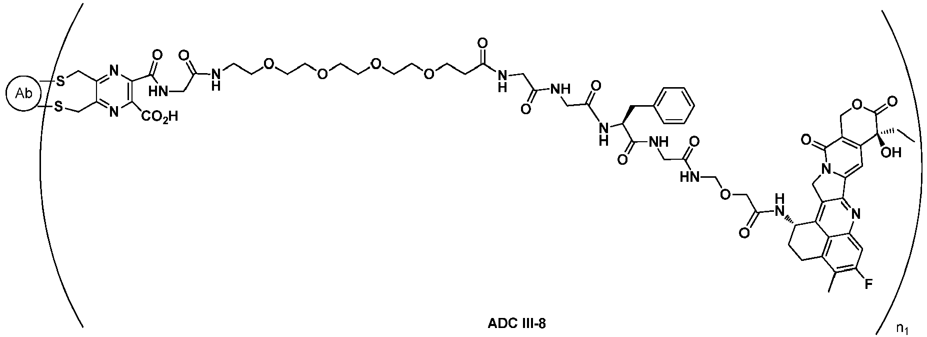

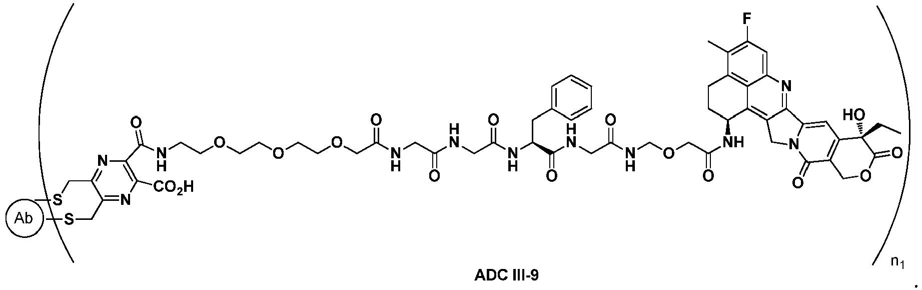

12. The bioactive conjugate according to claim 10, which has a structure as follows, wherein Ab is selected from the group consisting of the anti-HeR2 antibody trastuzumab or the anti-Trop2 antibody sacituzumab or the anti-ROR1 antibody 19F6_Hu35V1 (19F6 for short), n₁ is 1 to 8, preferably 3 to 5:

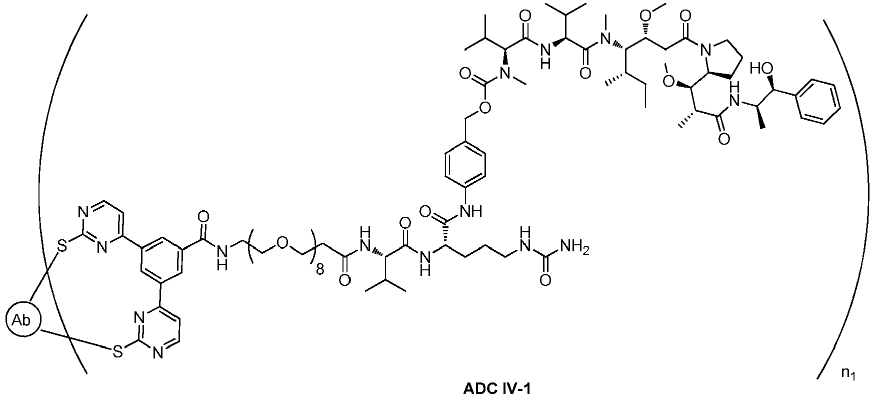

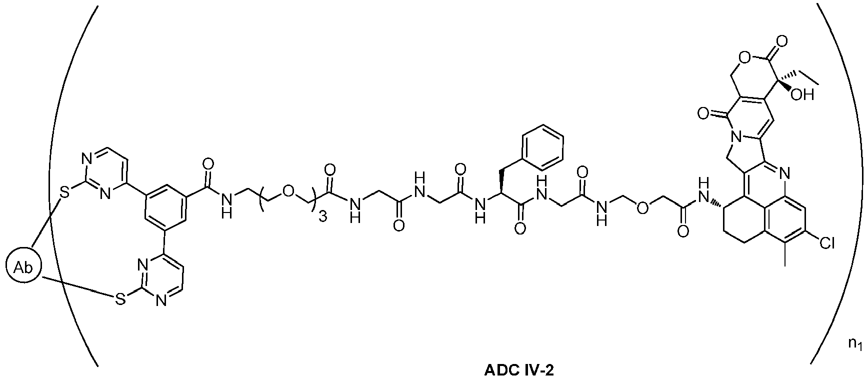

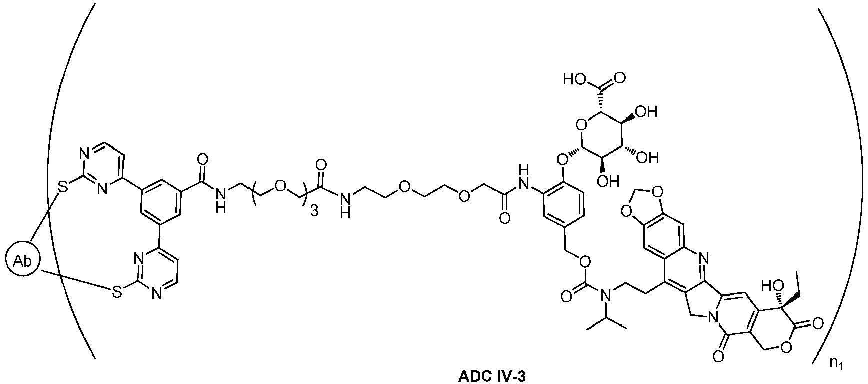

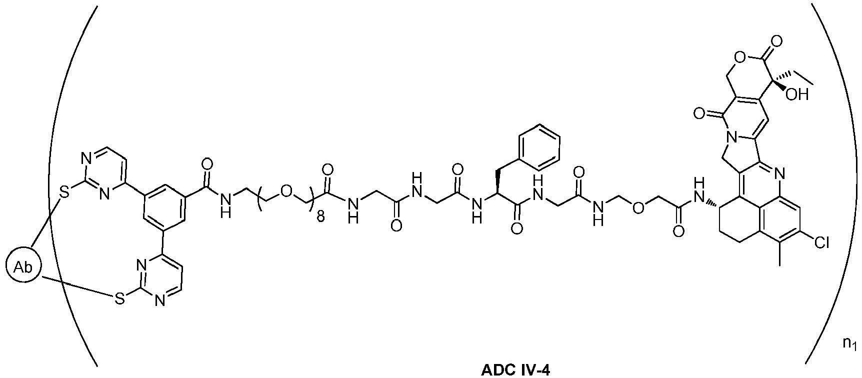

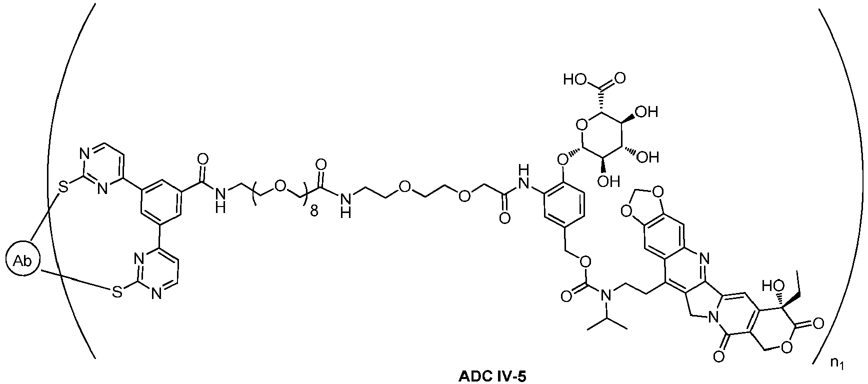

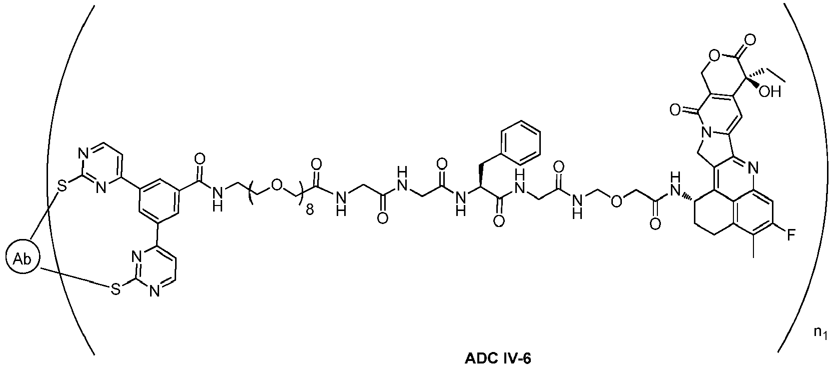

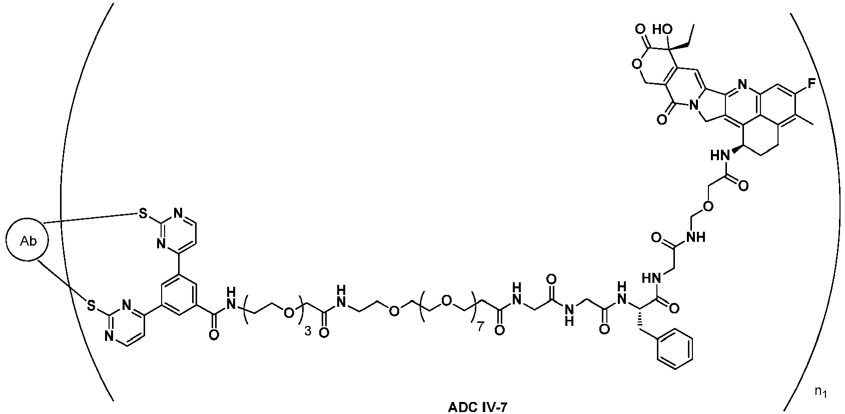

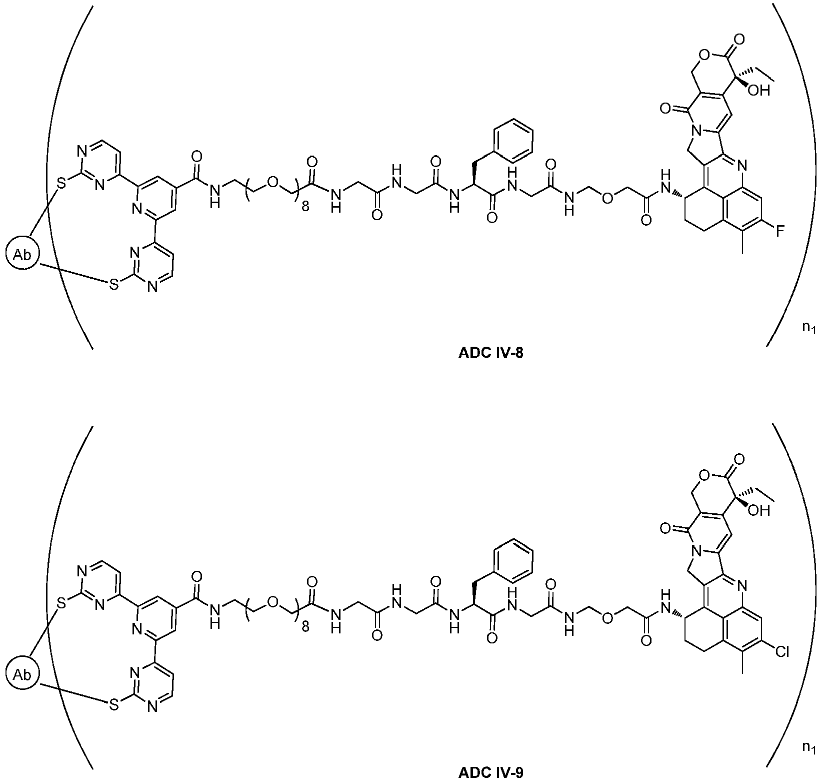

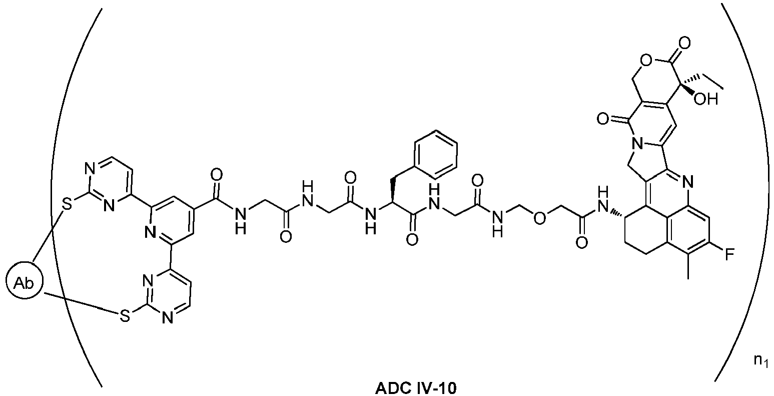

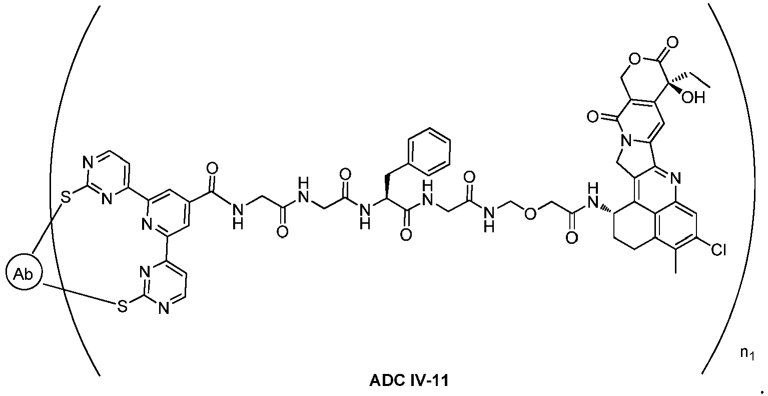

13. A pharmaceutical composition, which comprises the compound or the pharmaceutically acceptable salt, ester, stereoisomer, tautomer, polymorph, solvent compound, N-oxide or isotopically labeled compound thereof according to any one of claims 5 to 8, or the bioactive conjugate according to any one of claims 9 to 12, and one or more pharmaceutically acceptable carriers.

14. A kit product, which comprises the compound or the pharmaceutically acceptable salt, ester, stereoisomer, tautomer, polymorph, solvent compound, N-oxide or isotopically labeled compound thereof according to any one of claims 5 to 8, or the bioactive conjugate according to any one of claims 9 to 12, or the pharmaceutical composition according to claim 13, and optionally instructions.

15. Use of the compound or the pharmaceutically acceptable salt, ester, stereoisomer, tautomer, polymorph, solvent compound, *N*-oxide or isotopically labeled compound thereof according to any one of claims 5 to 8, or the bioactive conjugate according to any one of claims 9 to 12 in the manufacture of a medicament for preventing or treating a tumor disease.

16. The compound or the pharmaceutically acceptable salt, ester, stereoisomer, tautomer, polymorph, solvent compound, *N*-oxide or isotopically labeled compound thereof according to any one of claims 5 to 8, or the bioactive conjugate according to any one of claims 9 to 12, for use in preventing or treating a tumor disease.

17. A method for preventing or treating a tumor disease, comprising administering to a subject in need thereof an effective amount of the compound or the pharmaceutically acceptable salt, ester, stereoisomer, tautomer, polymorph, solvent compound, *N*-oxide or isotopically labeled compound thereof according to any one of claims 5 to 8, or the bioactive conjugate according to any one of claims 9 to 12.

18. A method for synthesizing a compound, **characterized in that** the method comprises the following steps: 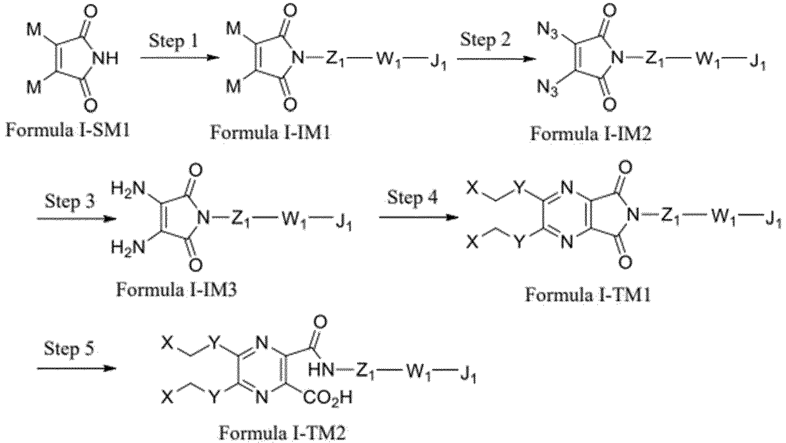 wherein:
X, Z₁, W₁, J₁ are as defined in claim 1; Y is absent;
M is a leaving group that undergoes a substitution reaction, including but not limited to a halogen, trifluoromethylsulfonyl, p-toluenesulfonyl, preferably a halogen;
alternatively, comprises the following steps: 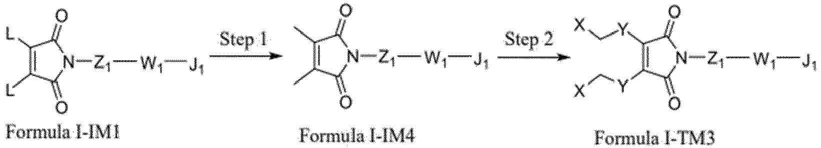
wherein:
X, Z₁, W₁, J₁ are as defined in the general formulas above;
L is a leaving group that undergoes a substitution reaction, including but not limited to a halogen, trifluoromethylsulfonyl, p-toluenesulfonyl, preferably a halogen or OTf;
alternatively, comprises the following steps: 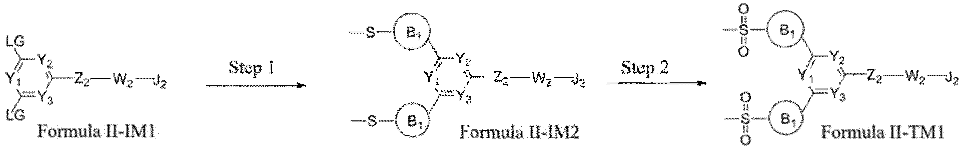
wherein:
Y₁, Y₂, Y₃, B₁, J₂ are as defined in the general formulas above;
LG is a leaving group that undergoes a conjugation reaction, including but not limited to a halogen, trifluoromethylsulfonyl, preferably a halogen.
